(19) Europäisches Patentamt
European Patent Office
Office européen des brevets

(11) EP 2 064 348 B1

(12) **EUROPEAN PATENT SPECIFICATION**

(45) Date of publication and mention of the grant of the patent:
**08.02.2012 Bulletin 2012/06**

(21) Application number: **07838435.1**

(22) Date of filing: **18.09.2007**

(51) Int Cl.:
***C12Q 1/68*** *(2006.01)* ***G01N 33/53*** *(2006.01)*

(86) International application number:
**PCT/US2007/020223**

(87) International publication number:
**WO 2008/036273 (27.03.2008 Gazette 2008/13)**

(54) **RECEPTOR FAMILY PROFILING**

PROFILIERUNG EINER REZEPTORFAMILIE

PROFILAGE DE FAMILLES DE RÉCEPTEURS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **18.09.2006 US 845330 P**
**28.09.2006 US 847859 P**
**07.03.2007 US 905364 P**
**14.03.2007 US 918023 P**

(43) Date of publication of application:
**03.06.2009 Bulletin 2009/23**

(73) Proprietor: **Ensemble Therapeutics Corporation**
**Cambridge MA 02139 (US)**

(72) Inventors:
- **LANDSMAN, Tanya**
**Brookline, MA 02446 (US)**
- **LIVINGSTON, David, J.**
**Barrington, RI 02806 (US)**
- **MARTINELLI, Richard, Allen**
**Belmont, MA 02478 (US)**
- **HUANG, Yumei**
**North Billerica, MA 01862 (US)**
- **SEIGAL, Benjamin, Adam**
**Watertown, MA 02472 (US)**
- **YAN, Dingxue**
**South Grafton, MA 01560 (US)**
- **COULL, James, M.**
**Westford, MA 01886 (US)**
- **STERN, Andrew, M.**
**Boston, MA 02116 (US)**

(74) Representative: **White, Martin Paul**
**Patents Designs & Brands Ltd.**
**211 The Colonnades**
**Liverpool**
**L3 4AB (GB)**

(56) References cited:
**WO-A-03/076943**
**WO-A-2005/074417**
**WO-A-2006/128138**
**US-A1- 2004 229 294**

- **LAUX I ET AL: "Epidermal growth factor receptor dimerization status determines skin toxicity to HER-kinase targeted therapies" BRITISH JOURNAL OF CANCER, vol. 94, no. 1, January 2006 (2006-01), pages 85-92, XP002477032 ISSN: 0007-0920**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 064 348 B1

## Description

### FIELD OF THE INVENTION

The present invention relates generally to assay technologies and their use in biodetection and diagnostics. More particularly, the invention relates to compositions and methods of nucleic acid templated chemistry in biodetection of receptors (and their families) and proteins (and their families), and the use of same in diagnostics.

### BACKGROUND

Laux et al have published an article in the British Journal of Cancer (2006), 94, 85-92 entitled "Epidermal growth factor receptor dimerization status determines skin toxicity to HER-kinase targeted therapies". Here it is disclosed that skin toxicity, a common drug-related adverse event observed in cancer patients treated with epidermal growth factor receptor (EGFR)-directed therapies, is rarely seen with therapies targeting HER2. This study is said to report the significance of EGFR and HER2 dimerization status in skin with regard to these dermatologic side effects. The differential effect of HER-directed therapies on ligand driven activation status of EGFR, HER2 and MAPK in normal human epidermal keratinocytes is discussed, as is a proximity-based dimerization assay (*eTag*™).

WO 2005/074417 discloses multiple antigen detection (MAD) assays and reagents for performing such assays. MAD assay embodiments are said to be useful for identifying proximal antigens in a sample (e.g., antigens existing in a complex), and are performed by contacting a sample with two or more hybrids, where each hybrid comprises a nucleic acid and a binding partner that specifically binds to an antigen. In these embodiments, the hybrid nucleic acids hybridize to one another when the hybrids bind to proximal antigens, and the hybridization product then is detected, e.g. by a polymerase chain reaction (PCR) process or a nucleic acid binding agent comprising a detectable label. Also disclosed are kits for conducting MAD applications.

WO 2003/076943 discloses a chemical compound comprising a chemical moiety (p) capable of performing a binding interaction with a target molecule (e.g. a biological target) and further comprising an oligonucleotide (b) or functional analogue thereof. In a first embodiment, the chemical compound is characterized in that the oligonucleotide (b) or functional analogue comprises at least one self-assembly sequence (b1) capable of performing a combination reaction with at least one self-assembly sequence (b1') of a complementary oligonucleotide or functional analogue bound to another chemical compound comprising a chemical moiety (q). In a second embodiment, the chemical compound which comprises a coding sequence (b1) coding for the identification of the chemical moiety (p) is characterized in that the chemical compound further comprises at least one self-assembly moiety (m) capable of performing a combination reaction with at least one self-assembly moiety (m') of a similar chemical compound comprising a chemical moiety (q). Corresponding libraries of chemical compounds are discussed, as well as methods of biopanning of target molecules and of identifying such targets.

DNA-Programmed Chemistry (DPC) technology is a method that uses oligonucleotides attached to reactive chemical groups or building blocks whereby the oligonucleotides specifically direct chemical reactions via oligonucleotide hybridization. The use of DNA in bringing together reactants controls reaction stoichiometry, lowers energies of activation, and segregates reactants, to ensure highly specific reactions under physiologic conditions. One application of DPC is protein detection. See, *e.g.*, Gartner, et al., 2004, Science, vol. 305, pp. 1601-1605; Li, et al., 2004, Angew. Chem. Int. Ed Vol. 43, pp.4848-4870; U.S. Patent No. 7,070,928; U.S. Patent No. 7,223,545; WO06128138A2 by Coull et al. As DPC provides the potential for low-noise detection and/or specific detection of biomarkers, methods and compositions useful for efficient and accurate detection and analysis of biomarker profiles are desirable and can improve diagnostic and potential treatment options.

For example, ErbB family of receptor tyrosine kinases are involved in an array of combinatorial interactions involving homo- and heterodimers. **ErbB** overexpression is associated with cancers of the breast, colon, prostate gland, lung, ovaries and brain. Although ErbB-directed therapeutics are effective against such ErbB-overexpressing cancers, their efficacy is limited and variable for reasons that are not well understood. For example, although Trastuzumab (Herceptin™) is effective against ErbB2-overexpressing metastatic breast cancers, which can be diagnosed using an immunohistochemical (IHC) test (HercepTest™) for ErbB2, Trastuzumab is nevertheless effective in only a minority of such cases. A principle reason for the efficacy limit is that the current diagnostic tests used to select appropriate patients are unable to distinguish active receptor dimers. This remains a significant technical challenge with current technologies.

### SUMMARY OF THE INVENTION

The present invention is based, in part, upon the discovery that biomarker profiling, e.g., ErbB receptor family dimerization profiles, can be achieved by DPC-based detection methods. More particularly, immunohistochemical (IHC)-based ErbB dimer assays for all known ErbB family dimers, can be developed using DNA-Programmed Chemistry

for highly specific detection of protein complexes. These assays hold the promise of enabling drug response and cancer prognosis predictions in individual patients based on complete ErbB family profiling of cancer tissue samples. These assays, using formalin-fixed, paraffin-embedded tissue samples, will improve diagnosis, treatment, and prognosis of all cancers in which ErbB proteins are dysregulated.

Methods and probes for use in the present invention are set out in the accompanying claims.

In certain embodiments, the family of receptors is the ErbB receptor family and, optionally, the ErbB receptor dimers are selected from the group consisting essentially of (e.g. greater than 80%, greater than 90%, greater than 95%, greater than 97% greater than 99.5%, and/or greater than 99.9%) of homodimers of ErbB1, ErbB2, ErbB3, and ErbB4. In certain embodiments, the ErbB receptor dimers are selected from the group consisting essentially of (e.g. greater than 80%, greater than 90%, greater than 95%, greater than 97% greater than 99.5%, and/or greater than 99.9%) of hetero-dimers of ErbB1, ErbB2, ErbB3, and ErbB4.

## DEFINITIONS

The term, "DNA programmed chemistry" or "DPC", as used herein, refers to nucleic acid-templated chemistry, for example, nucleic acid sequence specific control of chemical reactants to yield specific products accomplished by (1) providing one or more templates, which have associated reactive group(s); (2) contacting one or more transfer groups (reagents) having an anti-codon (e.g., complementary sequence with one or more templates) and reactive group(s) under conditions to allow for hybridization to the templates and (3) reaction of the reactive groups to yield products. For example, in a one-step nucleic acid-templated reaction, hybridization of a "template" and a "complementary" oligonucleotide bring together reactive groups followed by a chemical reaction that results in the desired product. Structures of the reactants and products need not be related to those of the nucleic acids comprising the template and transfer group oligonucleotides. See, *e.g.*, U.S. Patent Nos. 7,070,928 B1 and 7,223,545 and European Patent No. 1,423,400 B1 by Liu et al.; U.S. Patent Publication No. 2004/0180412 (USSN 10/643,752; Aug. 19, 2003) by Liu et al., by Liu *et al.*; Gartner, et al., 2004, Science, vol. 305, pp. 1601-1605; Doyon, et al., 2003, JACS, vol. 125, pp. 12372-12373. See, also, "Turn Over Probes and Use Thereof" by Coull et al., PCT WO07/008276A2, filed on May 3, 2006.

The terms, "nucleic acid", "oligonucleotide" (sometimes simply referred to as "oligo") or "polynucleotide," as used herein, refer to a polymer of nucleotides. The polymer may include, without limitation, natural nucleosides (i.e., adenosine, thymidine, guanosine, cytidine, uridine, deoxyadenosine, deoxythymidine, deoxyguanosine, and deoxycytidine), nucleoside analogs (e.g., 2-aminoadenosine, 2-thiothymidine, inosine, pyrrolo-pyrimidine, 3-methyl adenosine, 5-methylcytidine, C5-bromouridine, C5-fluorouridine, C5-iodouridine, C5-propynyl-uridine, C5-propynyl-cytidine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (e.g., methylated bases), intercalated bases, modified sugars (e.g., 2'-fluororibose, ribose, 2'-deoxyribose, arabinose, and hexose), or modified phosphate groups (e.g., phosphorothioates and 5'-N-phosphoramidite linkages). Nucleic acids and oligonucleotides may also include other polymers of bases having a modified backbone, such as a locked nucleic acid (LNA), a peptide nucleic acid (PNA), a threose nucleic acid (TNA), among others.

Throughout the description, where compositions are described as having, including, or comprising specific components, or where processes are described as having, including, or comprising specific process steps, it is contemplated that compositions of the present invention also consist essentially of, or consist of, the recited components, and that the processes of the present invention also consist essentially of, or consist of, the recited processing steps. Further, it should be understood that the order of steps or order for performing certain actions are immaterial so long as the invention remains operable. Moreover, two or more steps or actions may be conducted simultaneously.

## BRIEF DESCRIPTION OF THE FIGURES

The invention may be further understood from the following figures in which:

**FIG. 1** is a schematic representation of a method for the detection of a biological target under one embodiment of the present invention, illustrating a zip-coded format.

**FIG. 2** is a schematic representation of a method for the detection of a biological target under one embodiment of the present invention, illustrating a nonzip-coded format.

**FIG. 3** is a schematic representation of a method for the detection of receptor dimerization under one embodiment of the present invention, illustrating a nonzip-coded format.

**FIG. 4** is a schematic representation of a method for the detection of receptor dimerization under one embodiment of the present invention, illustrating a zip-coded format.

**FIG. 5** illustrates the general chemical structures of polymethine, cyanine and hemicyanine dyes.

**FIG. 6** is a schematic representation of DPC-based hemicyanine formation.

**FIG. 7** illustrates the general chemical structures of hemicyanine dyes useful for multiplex and their aldehyde

and quaternary salt precursors.

**FIG. 8** illustrates the chemical structures of a four-plex hemicyanine_DNA dye system and their spectroscopic properties.

**FIG. 9** is a schematic representation of a method for the detection of a biological target under one embodiment of the present invention.

**FIG. 10** is a schematic representation of a method for the detection of a biological target under one embodiment of the present invention.

**FIG. 11** shows examples of hybridization as affected by concentration, temperature, and the presence or absence of a single base pair mismatch.

**FIG. 12** shows exemplary oligonucleotides used in certain melting curve experiments.

**FIG. 13** is a schematic representation of a method for the detection of a biological target under one embodiment of the present invention.

**FIG. 14** is a schematic representation of a method for the detection of platelet derived growth factor (PDGF) under one embodiment of the present invention.

**FIG. 15** shows exemplary embodiment of a splinted, zip-coded detection system with aptamers as target binding moieties.

**FIG. 16** shows exemplary embodiment of a splinted, zip-coded detection system with antibodies as target binding moieties.

**FIG. 17** shows exemplary embodiment of a splinted, zip-coded detection system with antibodies as target binding moieties.

**FIG. 18** shows one embodiment of generation of two fluorescent hemicyanine dyes via DPC.

**FIG. 19** shows certain results from a DPC assay for EGFR homodimers utilizing indolinium condensation.

**FIG. 20** shows certain results from a DAZR-based DPC assay for ErbB2 dimers on N87 cells.

**FIG. 21** shows certain results from a DPC assay (indolinium condensation) for ErbB2 dimers on A431 cells.

**FIG. 22** is shows an example of fluorescence signal generation and biological target detection via triphenylphosphine (TPP) and azidocoumarin (AzC) reporter chemistry.

**FIG. 23** shows an example of fluorescence signal generation and biological target detection via TPP and AzC reporter chemistry.

**FIG. 24** shows certain examples of melt curves illustrating the effect of oligonucleotide concentration on $T_m$.

**FIG. 25** shows certain examples with DNA hybridization melting curves of biotinylated oligonucleotides with and without avidin.

**FIG. 26** shows certain examples of $T_m$ changes of complementary biotinylated oligos upon binding to avidin.

**FIG. 27** shows certain examples of the effect of salt and magnesium concentrations upon $T_m$ of oligonucleotides +/- biotin.

**FIG. 28** shows certain examples of the melting temperature behavior of biotinylated oligonucleotides at different ratios of oligonucleotides to avidin.

**FIG. 29** shows certain examples of melting curves of 5' and 3' (-) biotin-strand oligos duplexed with biotin-5' (+) strand oligo in the absence and presence of avidin.

**FIG. 30** shows certain examples of melting curves of AT-rich biotinylated oligo dimers with and without avidin.

**FIG. 31** is a schematic representation of a method for the detection of a biological target under one embodiment of the present invention.

**FIG. 32** shows examples of experimental results on detection of a biological target under one embodiment of the present invention.

**FIG. 33A and FIG. 33B** show examples of experimental results (the effect of formamide in the reaction mixture) on detection of a biological target under one embodiment of the present invention.

**FIG. 34A and FIG. 34B** show examples of experimental results (the effect of formamide in the reaction mixture) on detection of a biological target under one embodiment of the present invention.

**FIG. 35** shows examples of experimental results (the effect of formamide in the reaction mixture) on detection of a biological target under one embodiment of the present invention.

**FIG. 36** shows examples of experimental results (time course of reaction mixtures) on detection of a biological target under one embodiment of the present invention.

**FIG. 37** shows examples of experimental results (time course of reaction mixtures) on detection of a biological target under one embodiment of the present invention.

**FIG. 38** shows examples of experimental results (probe ratios) on detection of a biological target under one embodiment of the present invention.

**FIG. 39** shows an example of detection of PDGF by a zip-coded detection system.

**FIG. 40** shows experiments on ratios of aptamers and reporters.

**FIG. 41** illustrates an embodiment of a "one-piece" detection system for the detection of PDGF.

## DETAILED DESCRIPTION OF THE INVENTION

In its simplest sense, the invention provides methods and compositions for biomarker family measurement and profiling using DNA-programmed probe chemistry.

## PROFILING OF ErbB DIMERS

For example, the invention provides methods and compositions for the measurement and analysis of the dimerization profiles of the ErbB family of receptor tyrosine kinases.

The family of ErbB receptors includes ErbB I (also known as human epidermal growth factor (EGF) receptor 1 (HER1) or EGF receptor (EGFR)), ErbB2 (also known as HER2 or Neu), ErbB3, and ErbB4. They consist of an extracellular ligand-binding domain, a single transmembrane domain, an uninterrupted tyrosine kinase domain, and a cytoplasmic tail, and are involved in an array of combinatorial interactions involving homo- and heterodimers. See, e.g., Citri, et al., Nat Rev Mol Cell. Biol, 2006. 7(7), 505-16; Muthuswamy, et al. Mol Cell Biol, 1999. 19(10), 6845-57; Hynes, et al. Nat Rev Cancer, 2005, 5(5) 341-54**.**

They have been implicated in the development of many human cancers and have been intensely pursued as therapeutic targets. See, Hynes, et al. Nat Rev Cancer, 2005, 5(5) 341-54; Baselga, J. Science, 2006 312(5777), 1175-8**.** ErbB-directed therapeutics have demonstrated clinical efficacy; however, anti-tumor effects are often not as strong as predicted from pre-clinical studies. For example, only 35% of patients with ErbB2 overexpressing metastatic breast cancer respond effectively to Trastuzamab (anti-ErbB2, Herceptin™) therapy. Vogel, et al., J Clin Oncol, 2002, 20(3) 719-26. Moreover, trastuzamab is somewhat, but not always, effective in treating breast cancers that show elevated levels of both ErbB1 and ErbB2. Indeed, ErbB homo- and heterodimers differ in their signaling, response to ligands, and tumorigenic properties. Citri, et al., Nat Rev Mol Cell Biol, 2006. 7(7), 505-16; Muthuswamy, et al. Mol Cell Biol, 1999. 19(10), 6845-57*;* Hynes, et al. Nat Rev Cancer, 2005, 5(5) 341-54**.** It is therefore highly likely that variation in the levels as well as dimerization status of ErbB family members in a tumor cell contributes to variations in drug efficacy between patients. For example, co-expression of ErbB2/ErbB 1 and ErbB2/ErbB3 may predict responsiveness to Herceptin™ treatment. The efficacy of Herceptin therapies is influenced by the expression of other ErbB receptors, their ligands and the activation of downstream signalling proteins. Smith, et al., Br J Cancer, 2004, 91(6), 1190-4; Wehrman, et al., Proc Natl Acad Sci U S A, 2006, 103(50), 19063-8.

Furthermore, anti-cancer therapy includes antibodies that disrupt ErbB1/ErbB2 dimers (e.g. Omintarg™), but treatment responsiveness cannot be predicted, because no suitable assay is available to detect dimer status from tissue samples. In addition, the presence of ErbB2 extracellular domain (ECD)-truncated forms, as a result of alternative initiation of translation within the *ErbB2* mRNA, may promote resistance to Trastuzumab. Anido, et al., Embo J, 2006, 25(13), 3234-44**.** Hence detection of full-length vs. truncated receptor may help to predict responsiveness to Trastuzumab.

Homo- and heterodimerization of ErbB receptors are measured using probe sets. Each set is specifically directed at a particularly dimer. As illustrated in **FIG. 1,** which include a "zip-coded" reporter format, each probe set includes a pair of reactive groups (reactive precursors R1 and R2) separately linked to a corresponding reporter oligonucleotide (reporter DNA). The pair of oligonucleotides are complementary in sequence. The reactive precursors can only react when brought into reactive proximity through a hybridization event between the complementary oligonucleotides, which results from the binding of the associated binding moieties (e.g., ErbB antibodies) to the corresponding ErbB monomer. For example, the reactive precursors may be non-fluorescent precursor pairs (e.g. diazidorhodamine (DAZR) and bisdiphenylphosphine) that generate fluorescent products (e.g. rhodamine) when the precursor pairs are in close proximity, i.e. upon binding of the associated antibodies to their target protein hetero- or homodimer *in situ.* This method can be used to detect homodimers, heterodimers, antibodies, and nucleic acids.

Illustrated in **FIG. 2** is another format which employs a non-zip-coded reporter design. The binding moieties are directly linked to the respective reactive precursors through corresponding complementary reporter oligonucleotides.

**FIG. 3** illustrates a multiplexed IHC test for multiple ErbB family receptors. Multiple pairs of probes, each pair being directed at a particular ErbB homo- or hetero dimer and with a distinct DPC product (e.g., a distinct fluorescent signal from each pair as shown), can provide simultaneous detection and profiling of multiple ErbB receptor dimers. Both zip-coded, as illustrated in **FIG. 4,** and non-zip-coded probe pairs can be employed in a multiplex test. As to reporter chemistry, polymethine dyes may be used in a multiplexed detection system.

Polymethine dye is characterized by a chain of methine (-CH=) groups with an electron donor and an electron acceptor at opposite ends of their polyene chain **(FIG. 5),** Zollinger, Color Chemistry: Syntheses, Properties, and Applications of Organic Dyes and Pigments, 3nd Edn., Verlag Helvetica Chimica Acta, Postfach, Switzerland, 2003**).** Typical A and D terminals for polymethine dyes (as shown in **FIG. 5)** include thiazoles, pyrroles, pyrrolines, indoles, 1, 3, 3-trimethylindolines, tetrazoles, pyrimidine, pyridines, quinolines, and higher fused N-heterocycles or any substituted benzyl rings. If the terminals are both N-atom containing heterocycles, the compound is named cyanine. If only one N-atom is part of the ring system, the compound is named hemicyanine. By changing the number of the vinyl group in the

polyene chain, the fluorescence emission wavelength of the polymethine dye can be tuned from near-UV to near-IR. The terminal group may also provide mean for finer tuning.

**FIG. 6** gives an example of DPC hemicyanine formation through end of helix architecture. Upon annealing, the two hemicyanine precursors were placed in reactive proximity at the end of helix and a hemicyanine linked to both DNA was formed after condensation.

**FIG. 7** shows the general chemical structures of hemicyanine dyes useful for multiplex and their aldehyde and quaternary salt precursors. An example of four-plex hemicyanine_DNA dyes derived from the general structure and their maximum UV absorption and fluorescence's emission wavelength has been described in **FIG. 8.**

Advantages of DPC-based dimer profiling Include: (1) *High sensitivity.* Since DNA hybridization increases effective molarity of the reactants (from nM concentration to mM effective concentration, $10^6$ fold), assays can be performed at very low probe concentration. (2) *Low non-specific background.* Many conventional assays rely on an increase or decrease in fluorescence of a fluorophore-labeled probe upon binding of the probe to a target, or upon liberation (cleavage) of a fluorophore, a quencher, or a protecting group from the target-bound probe by a chemical or enzymatic event. The associated fluorescence backgrounds are usually high. In DPC-based assays, non-specific background can be kept very low ("zero") when non-fluorescent precursors are used. For example, in the hemicyanine based DPC system, the probes become fluorescent only after binding to the probes' target protein hetero- or homodimer and upon reaction to create a fluorescent signal molecule. **(3)** *Improved specificity and affinity*. The assay format of the present invention requires two probes and dual recognition of the target. The analyte recognition involves two independent binding events (protein recognition and nucleic acid hybridization). Thus, the dimerization induced nucleic acid hybridization and DPC greatly increases its detection specificity. For example, only a probe pair with completely matched oligonucleotide sequences can produce a fluorescent hemicyanine product. **(4)** *Detection in functional context.* DPC-based assay format allows detection of receptors and dimerization in their functional context. It is minimally invasive to the biological system. It is also very simple and amenable to point-of-care or point-of-sale format. (5) *Can be customized to a multiplex platform as described herein.*

## DPC-BASED PROTEIN DETECTION

Methods and compositions of biodetection using nucleic acid-templated chemistry based probes are described in WO06128138A2 by Coull et al.

**FIG. 9** and **FIG. 10** illustrate one embodiment of the invention for the detection of a protein target.

**FIG. 9** shows an embodiment of detection of a protein target by DPC-based probes. Two probes contain target binding moieties, complementary oligonucleotides, and chemically reactive species X and Y, respectively. Upon hybridization, X and Y react to create a signal generating (e.g., fluorescent) compound, which may or may not covalently link both probes. The reaction product of X and Y may also be released as an unbound, soluble compound into the solution. The protein target may be attached to a solid-phase such as the surface of a bead, glass slide (microarray), etc., or be in solution. The target binding moieties may be aptamers, antibodies, antibody fragments (i.e., Fab), receptor proteins, or small molecules, for example.

More particularly illustrated in **FIG. 10** is an example of the dual-probe approach with two probes, each carrying a "prefluorophore" precursor (R1 and R2) and containing a binding moiety for a target and an oligonucleotide sequence that is designed to anneal to each other. In this embodiment, the detection is performed under conditions such that the prefluorophore oligos will not anneal to each other in the absence of a target. These conditions are generally selected such that the ambient temperature is higher than the $T_m$ of the oligonucleotide pairs in the absence of the target (so that the oligo pairs will not anneal in the absence of the intended target analyte). In the presence of the intended target, however, the localized high concentration of the oligos then shifts the $T_m$ of their double stranded complex upwards so that hybridization occurs, which is followed by a signal-generating nucleic acid-templated reaction (a reaction between R1 and R2). The signal-generating nucleic acid-templated reaction is accelerated both due to the localized higher concentration of the prefluorophores, but may also be facilitated by the proximity and orientation of the prefluorophore groups towards one another. This configuration of signal generation has the potential to enable creation of kits for the detection of various biomolecules, cells, surfaces and for the design *of in situ* assays. The signal generation does not require enzymes and the homogeneous format requires no sample manipulation.

In **FIG. 10,** two oligonucleotides are shown, each of which is linked through an optional spacer arm to a separate binder, as shown in this case is an antibody but may be other binders such as aptamers or small molecules. Each antibody recognizes a separate epitope on a common target analyte such as a protein. Spacer arms can be added to one or both oligonucleotides between the oligo and the binder. In certain cases, this spacer arm may be required to meet proximity requirements to achieve a desired reactivity. Spacer arms in principle can be any suitable groups, for example, linear or branched aliphatic carbon chains C3 to C5, C10, C15, C20, C25, C30, C35, C40, or C100 groups, a DNA sequence of 1 to 10, 15, 20, 30, 50 or 100 bases long, or polyethylene glycol oligomers of the appropriate length.

The prefluorophores may reside in an "end of helix" configuration **(FIG. 10 top),** one attached to the 5' end of

an oligo and other to the 3' end. (Other configurations can be applied, including placing the two prefluorophores within the sequence or having one oligo hybridize to a partial hairpin structure (e.g., 100 Angstroms long), for example.) In the first example, one oligonucleotide is attached 5' to a spacer arm and a target binder, and the other 3' to a spacer arm and separate target binder. Spacer arms, which can consist of non-complementary DNA sequences, or synthetic spacer arms such as oligomers of ethylene glycol, can be added to meet proximity requirements. Such spacer arms can be very flexible, which has the advantage of overcoming any steric hindrance to binding that might occur with a rigid spacer. A suitably long spacer arm design can permit both oligonucleotides to be linked 5' to their binders (FIG. 10 bottom), or both linked 3', as long as the oligonucleotides can anneal in the antiparallel configuration and allow the reactive groups to react with each other. An optimal spacer arm length may be designed for each target. Spacer arms which are excessively long should be avoided as they may reduce specificity in the system or a reduced increased $T_m$ effect.

The proximity effect afforded by tethering the pair of oligonucleotides may affect the kinetics of annealing of two complementary oligonucleotide sequences compared to the two oligonucleotides free in solution. More importantly, a localized high concentration shifts the melting curve upwards compared to the free complex, i.e. increase the $T_m$ of the complex. In a bulk solution, it is known that $T_m$ has dependence upon total oligonucleotide concentration as illustrated in the equation below. Wetmur, Criti. Rev. in Biochem. And Mol. Biol., 1991, 26, 227-259.

$$T_m = (1000 * \Delta H) / (A + \Delta S + R \ln(C_t/4) - 273.15 + 16.6 \log Na^+)$$

where $\Delta H$ and $\Delta S$ are the enthalpy and entropy for helix formation, R is the molar gas constant, $C_t$ is the total concentration of oligomers, and $Na^+$ is the molar concentration of sodium ion in the solution.

**FIG. 11** shows the slope of $T_m$ vs. concentration within the range of short oligonucleotides in 0.1 M salt has a dependence of about +7° C per 10-fold increase in concentration of oligonucleotides (sequences in **FIG. 12)** based on the above equation. So, for example, a 1000-fold increase in local concentration would be expected to raise $T_m$ by about +21° C.

Reaction products of **R1** and **R2** may be released from the hybridization complex as a result of the chemical transformation. Thus, the fluorophore or chromophore may be separated from the hybridization complex and analyzed independently, or the fluorophore or chromophore and the annealed oligonucleotides may be removed once detected so that additional rounds of interrogation of the sample can be conducted. The reaction between R1 and R2 may or may not covalently link the two probes once the product(s) is formed.

**FIG. 13** illustrates another embodiment of the invention, which employs a "zip-coded" splint architecture for nucleic acid template-based biodetection. In this embodiment, instead of the target binding moieties being directly linked (optionally via spacer groups) to the complementary oligonucleotides that hybridize and set up nucleic acid templated reactions, the target binding moieties is linked to a "zip code" oligonucleotide sequence. Each of the corresponding reporter oligonucleotide has a complementary, "anti-zip code" sequence (in addition to a "reporter" sequence that set up nucleic acid-templated reaction). The nucleic acid-templated chemical reactions are set up by the hybridization of the reporter oligos, which are linked to reactive groups that react and generate detectable signals. It is important that each oligonucleotide sequence of the probes is complementary only to its intended hybridization partner and not complementary to other oligonucleotides in the detection system.

This zip-coded architecture supports creating a single reporter-oligonucleotide conjugate which would assemble with different downstream reporter oligonucleotides through an anti-zip code sequence. Libraries of different reporters linked to a unique anti-zip code may be tested simply by mixing each one with stoicheometric amounts of the binder-zip code oligonucleotide conjugate with its complementary zip code.

**FIG. 14** is an illustration of a zip-coded splinted architecture approach where the target binding moieties are two aptamers. In this example for detection of platelet derived growth factor (PDGF) with illustrative oligo sequences and reporter chemistry (e.g., triphenylphosphine, TPP, and **7**-azidocoumarin, AzC), the TPP reporter oligonucleotide self-assembles to the PDGF aptamer oligonucleotide through hybridization of zip code sequence (NNN.....) to the complementary anti zip code sequence (N'N'N'......) on the TPP reporter oligonucleotide. The reporter oligonucleotide terminates with an exemplary 10-base reporter sequence and a 5'-TPP group. A separate pair of oligonucleotides, with different zip codes and anti-zip codes (complementary to each other pairwise), also self-assembles to provide the AzC reporter sequence and a 3'-AzC group. The AzC oligonucleotides are complementary and antiparallel to the TPP oligonucleotides so the TPP and AzC groups terminate end-to-end when the TPP and AzC oligonucleotides anneal to each other.

**FIG. 15** illustrates in more detail the zip-coded splinted architecture approach for detection of PDGF with illustrative oligo sequences and reporter chemistry (TPP and AzC). The TPP pair includes, first, a PDGF-aptamer on the 5'-end, a C18 polyethylene-glycol based spacer, and an 18-mer zip code sequence. The TPP reporter sequence includes a complementary anti-zip code sequence on its 3' terminus, a C18 PEG spacer, and a ten base pair reporter

sequence terminating in a 5' TPP group. The AzC pair of oligonucleotides includes a 3'-aptamer linked through a C18 PEG spacer to a separate zip code, and a detection oligonucleotide linked to a 5' anti-zip code, a C18 PEG spacer, and a reporter oligonucleotide (complementary to the TPP oligonucleotide) terminating in a 3' AzC group.

**FIG**. **1**6 illustrates an example of the corresponding architect where antibodies are used instead of aptamers as target binding moieties.

One advantage of the "zip coded" approach is the ability to create the reporter oligonucleotides separately, and have them assemble together with binders under conditions retaining the activities of both the binders and of the nucleic acid template-activated chemistry.

The zip-coded system is based upon two pairs of oligonucleotides, with each pair being held together by the base-pairing of a unique zip code and an anti-zip code pair. "Zip codes" are oligonucleotide sequences which bind specifically to their complementary sequences, and preferably are designed such they are not complementary to known genomic sequences (relevant if the sample may contain genomic DNA), have similar $T_m$ values, lack significant secondary structure, and do not anneal to other zip code or anti-zip code sequences in the detection system.

It is worth pointing out the methods of the invention do not require enzymatic or chemical ligation of the first and/or the second oligonucleotide sequences.

Factors that may be considered in optimizing a design of a zip-coded architecture include, for example, (1) spacer groups (e.g., oligonucleotides and/or non-base groups) between the aptamer/antibody and zip codes (spacer 1), e.g., to allow hybridization partners to reach each other, to prevent any steric hindrance; (2) Length of a zip code sequence in order to form a sufficiently stable annealing to the anti-zip code sequence to form the complex; and (3) Spacer groups (spacer 2) between the anti-zip code and the reporter sequence, e.g., to prevent any steric hindrance.

The binders (target binding moieties) attached to the oligonucleotides may be any chemical moieties that specifically bind to a target molecule and allow the design of the invention to work. Examples include a wide range of functionalities, such as (1) antibodies: e.g., IgG, IgM, IgA, IgE, Fab's, Fab', $F(ab)_2$, Dab, Fv or ScFv fragments; (2) small molecule binders, such as inhibitors, drugs, cofactors; (3) receptors for protein detection, and vice versa; (4) DNA, RNA, PNA aptamers; (5) DNA sequences for DNA-binding and regulatory proteins; (6) peptides representing protein binding motifs; (7) peptides discovered through phage display, random synthesis, mutagenesis; (8) naturally binding protein pairs and complexes; (9) antigens (for antibody detection); and (10) a single polyclonal antibody separately attached to two oligonucleotides may serve as two separate binders of different specificity.

The target binding moieties attached to the oligonucleotides may be of heterogeneous types directed against different sites within the same target. For example, the two binders may be two different antibodies, an antibody and a receptor, an antibody and a small molecule binder, a receptor and a peptide, an aptamer and a cofactor, or any other combination.

The target analytes can be of any type, provided the target supports two (or more) binding sites. Molecules which exist in equilibrium with a monomeric form and a homodimeric or higher polymerization phase may be detected by a pair of probes containing the same binder but different complementary DNA sequences. Suitable targets include proteins, cell surfaces, antibodies, antigens, viruses, bacteria, organic surfaces, membranes, organelles, *in situ* analysis of fixed cells, protein complexes. The invention may be particularly suited for the detection of fusion proteins (e.g., BCR-ABL in the presence of BCR and ABL).

In the design of the probes, one consideration is the $T_m$ of the two reporter sequences carrying the reactive groups. Since the $T_m$ of the duplex should be below room temperature in the absence of a target, this sequence normally should be short, for example 6-15 bases and/or A-T rich. A typical reporter length of 10 base pairs might have a $T_m$ of around 30°C at a low salt concentration. Therefore, it is often necessary even with a short sequence to add 10% to 40% volume/volume formamide to further lower the temperature below assay temperature, or to elevate the assay temperature. Very short reporter oligonucleotides may suffer from a lack of specificity and exhibit some binding to zip code sequences (when these are employed) which is undesirable.

Another factor in the design of the probes is the length of oligonucleotide in between the binding moiety and the reporter sequence, including any zip code sequences. These must be long enough for the reporter oligonucleotides to reach each other and anneal. The sequences may be interspersed with polyethylene glycol (PEG) linkers that are flexible and may afford additional protection against any steric hindrance. For example, total lengths of oligonucleotides may be around 35 bases long. Oligonucleotides containing 0, 1, or 2 C18 PEG spacers, or homopolymer tracts may also be utilized (i.e. $C_{10}$).

A third consideration is the length of zip and anti-zip sequences when these are employed (i.e. **FIG. 14** and **FIG. 17).** Aside from the need for each zip code to anneal only to its anti-zip code, and not any other zip code, anti-zip code, or reporter sequence, an important parameter is the $T_m$ of the duplex between the zip codes and anti-zip codes. The $T_m$ should be substantially higher than the highest temperature that will be used in the assay in order that the reporter oligonucleotides remain firmly attached to the binding moiety. In practice, zip codes of about twice the length of the reporter sequences (i.e. total length of 15-30 bases) are desirable and generally meet these criteria.

Regarding signal generation, nucleic acid-templated chemistry may be used to create or destroy a label that

effects an optical signal, e.g., creating or destroying a fluorescent, chemiluminescent, or colorimetric molecule. Additionally, a detection reaction may be designed to create or destroy a product that directly or indirectly creates a detectable label, for example, a product that catalyzes a reaction that creates an optical label; inhibits a reaction that creates an optical label; is a fluorescence quencher; is a fluorescent energy transfer molecule; creates a Ramen label; creates an electrochemiluminescent label (i.e. ruthernium bipyridyl); produces an electron spin label molecule.

The following examples contain important additional information, exemplification and guidance that can be adapted to the practice of this invention in its various embodiments and equivalents thereof. Practice of the invention will be more fully understood from these following examples, which are presented herein for illustrative purpose only, and should not be construed as limiting in anyway.

## EXAMPLES

### Oligonucleotide Sequences

Oligonucleotides were prepared using standard phosphoramidite chemistry (Glen Research, Sterling VA, USA) and purified by reversed-phase C18 chromatography. Oligonucleotides bearing 5'-amino groups were prepared using either 5'-Amino-Modifier 5 (antizip reporter oligonucleotides) or 5'-Amino-Modifier C6 (zip oligonucleotides). Oligonucleotides bearing 3'-amino groups were prepared using 3'-Amino-Modifier C7 CPG (Glen Research, Sterling VA, USA).

Table 2 Oligonucleotide Sequences

| Oligo | sequence (5'-3') | ID No. |
|---|---|---|
| Zip2 | TTGGTGCTCGAGTCCCCCCCCCCCCCCCCCCCCCCC-$NH_2$ | (SEQ ID NO: 66) |
| Zip3 | $NH_2$-CCCCCCCCCCCCCCCCCCCCGCTGCCATCGATGGT | (SEQ ID NO: 67) |
| Zip5 | $NH_2$-CCCCCCCCCCCCCCCCCCCGTGCCATCCATAGTCAG | (SEQ ID NO: 68) |
| Antizip2 reporter1 | GGACTCGAGCACCAATAC-X-TATAAATTCG-$NH_2$ | (SEQ ID NO: 69) |
| Antizip3 reporter | $NH_2$-CGAATTTATA-X-CTGACCATCGATGGCAGC | (SEQ ID NO: 70) |
| Antizip5 reporter mismatch | $NH_2$-CCAATTAATA-X-CTGACTATGGATGGCACG | (SEQ ID NO: 71) |
| Antizip5 reporter | $NH_2$-CGAATTTATA-X-CTGACTATGGATGGCACG | (SEQ ID NO: 72) |
| Antizip2 report2 | GGACTCGAGCACCAATACXTATAAATTCGCCC | (SEQ ID NO: 73) |

X = Spacer Phosphoramidite 18 (Glen Research, Sterling VA, USA)

**Example 1. DPC Reporter Chemistry: Rhodamine formation through phosphine reduction of DAZR**

**Scheme 1** illustrates the reduction of DAZR (diazidorhodamine, non-fluorescent) to rhodamine (fluorescent) via DNA programmed chemistry.

**Scheme 1: DPC of rhodamine formation (any phosphine capable of reducing azide can be used here.)**

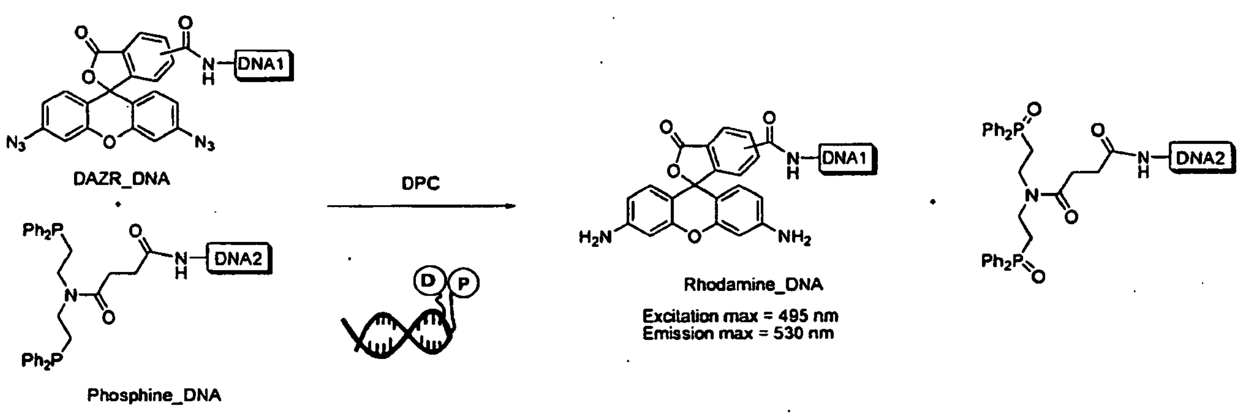


Both DAZR and phosphine are linked to oligonucleotide (DNA) through amide bond formation. First, an acid

precursor was synthesized. The acid precursor was then either converted to the active N-hydroxysuccinimide ester (NHS ester) that reacted with a DNA bearing amine functionality in solution (DNA_DAZR, **Scheme 2)** or was directly coupled to the DNA bearing amine functionality on the Controlled Pore Glass (CPG) (DNA_bisdiphenylphosphine, **Scheme 3).**

**Scheme 2** illustrates various synthetic routes to DAZR and DNA_DAZR conjugate. The acid derivatives of rhodamine (isomers) were initially synthesized following the literature procedure by condensing 3-aminophenol with 1,3-dioxo-1,3-dihydroisobenzofuran-5-carboxylic acid under very harsh conditions (e.g., high temperature, strong acid and prolonged reaction time) **(Scheme 2a).** Under those conditions, significant amount of fluorescein and rhodol compounds were generated which were difficult to separate from rhodamine acid. The yield for this reaction was also quite low. The compound isomers **(1)** are Zwitterion and cannot be purified by Silica gel column. Subsequent reaction of **1** with sodium nitrile and sodium azide afforded only 11% of DAZR (Novikova, et al., Russian Journal of Organic Chemistry, 1998, 34, 1762-1767).

An alternative approach was thus developed to provide more effective access to DAZR acid **(Scheme 2b).** In this approach, diiodo and dibromo substituted rhodamines **(3 & 4)** were synthesized and then converted to DAZR acid. Compounds **3** and **4** are not Zwitterions and can be purified easily, and the yields for generating these compounds are good (40 to 60% yield). Around 80% of DAZR acid isomers were generated from dibromo-substituted rhodamines (4).

**Scheme 2: Synthetic routes to DAZR and DNA_DAZR conjugates.**

Scheme 2a:

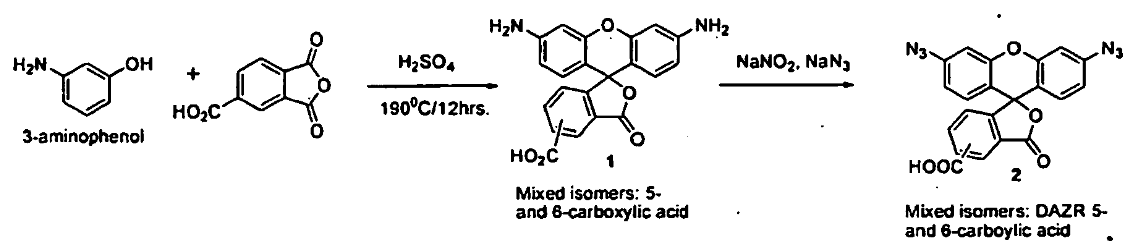


3-aminophenol

1

Mixed isomers: 5- and 6-carboxylic acid

2

Mixed isomers: DAZR 5- and 6-carboylic acid

Scheme 2b:

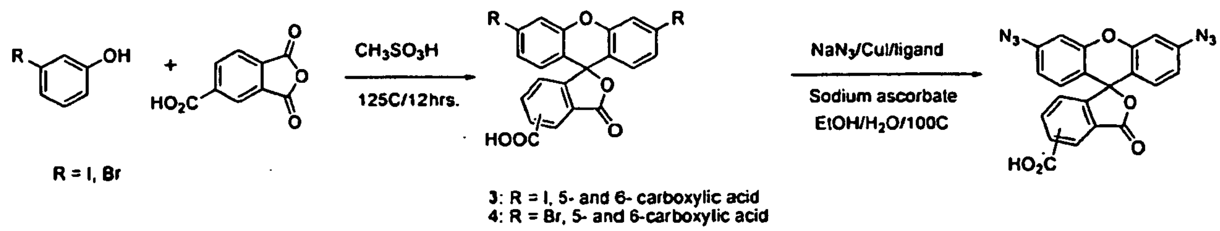


R = I, Br

3: R = I, 5- and 6- carboxylic acid
4: R = Br, 5- and 6-carboxylic acid

Scheme 2c:

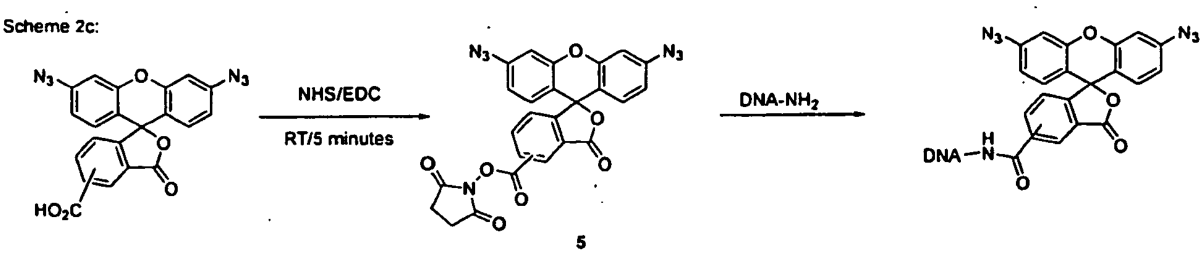


5

## Scheme 3: Synthetic routes to bisdiphenylphosphine acid and DNA_bisdiphenylphosphine conjugate.

Scheme 3a: Synthesis of acid derivatives of bsdiphenylphosphine

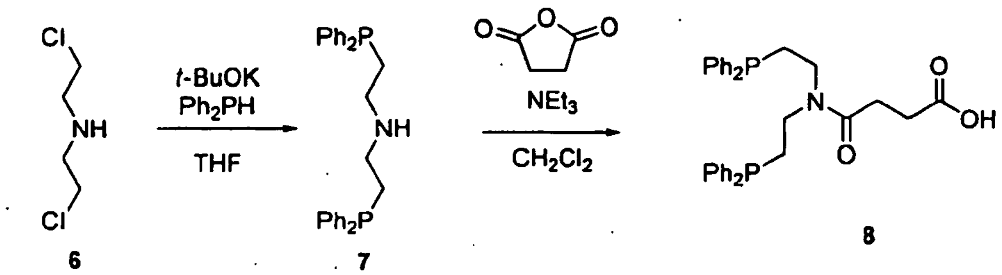


6 7 8

Scheme 3b: Synthesis of DNA_bisdiphenylphosphine

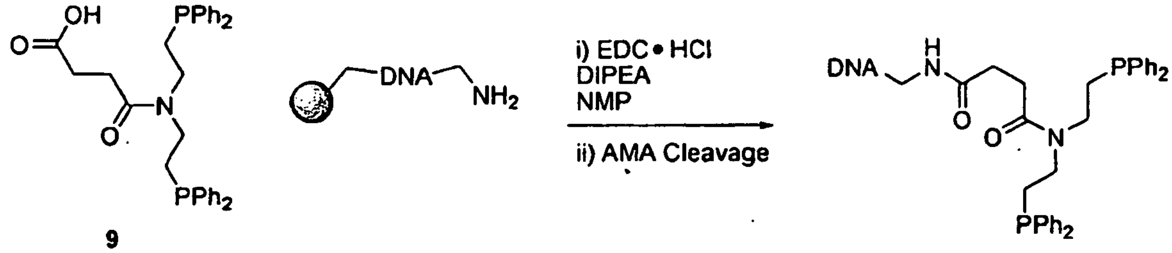


9

Synthesis of compound **2** (3',6'-diazido-3-oxo-3H-spiro[isobenzofuran-1,9'-xanthene]-5-[and -6]-carboxylic acid mixed isomers, Scheme 2a): To a solution of 1 (3',6'-diamino-3-oxo-3H-spiro[isobenzofuran-1,9'-xanthene]-5- [and -6]-carboxylic acid mixed isomers, 600 mg, 0.16 mmol) in 4 mL of 2N HCl was added sodium nitrite (276 mg, 4.01 mmol). After stirring at room temperature (RT) for 30 minutes, ice was added followed by sodium azide (625 mg, 9.62 mmol) dissolved in a minimum amount of water. Solid was precipitated out and was separated out by filtration. The crude product was purified by 4g of RediSep silica-gel column on a CombiFlash Companion chromatography system (1% triethylamine in DCM/MeOH) to afford 40 mg of mixed isomers 2(11%).

Synthesis of compound **3** (3',6'-diiodo-3-oxo-3H-spiro[isobenzofuran-1,9'-xanthene]-5-[and -6]-carboxylic acid mixed isomers): To a 100ml flask was added 2,4-benzentricarboxylicacid anhydride (4.50 g, 23.4mmoles), 3-iodophenol (10.3 g, 26.8 mmoles) and methanesulfonic acid (40 mL). The mixture was stirred at 125 °C under atmosphere of argon for 16 hrs. The cooled mixture was poured into 100 g of ice. The precipitate was dissolved in acetone/DCM and washed with water. The crude product was purified by 80g of RediSep silica-gel column on a CombiFlash Companion chromatography system (DCM/acetone) to afford 6.0 g of the product (white powder, 43.0%). Calculated exact mass for $C_{21}H_{10}I_2O_5$: 595.86
Observed: 594.8348 (M-H).

Synthesis of compound 4 (3',6'-dibromo-3-oxo-3H-spiro[isobenzofuran-1,9'-xanthene]-5- [and -6]-carboxylic acid mixed isomers): To a 100 ml flask was added 1,2,4-benzentricarboxylicacid anhydride (2 g, 10.41 mmoles), 3-bromophenol (2.341 g, 13.53 mmoles) and methanesulfonic acid (45 g, 468 mmoles). The mixture was stirred at 135 °C for 14 hours under atmosphere of Argon. The cooled mixture was poured into ice water (100g). The product was extracted with 75 mL of DCM and then washed with 100 mL of water. The solvent was removed by rotary evaporation. The solid residue was washed with DCM to remove unreacted bromophenol and other nonpolar impurities. The crude product was further purified by silica gel chrmatography (0-5% MeOH/DCM) to obtain 3.0 g of product (white powder, 57.4%). Calculated exact mass for $C_{21}H_{10}Br_2O_5$: 499.89; Observed: 500.9679 (M+H).

Synthesis of compound **2** (3',6'-diazido-3-oxo-3H-spiro[isobenzofuran-1,9'-xanthene]-5-[and -6]-carboxylic acid mixed isomers, **Scheme 2b)**: To a 100ml flask was added iodofluoran (3) (0.30g, 0.50mmoles), CuI (0.01 g), sodium ascorbate (0.02g), 1,2-dimethaminoethane (0.02g), sodium azide (0.10g, 1.50 moles), 15 mL of ethanol and 5 ml of water. The resulting mixture was heated under Argon at 100 °C for 30 minute. The cooled mixture was diluted with water, neutralized with HCl, and extracted with DCM. The crude product was purified by silica gel chromatography (0-5% MeOH/DCM) to obtain 0.13 g of product (white powder, 60.6%).

Synthesis of compound 5 (NHS ester of DAZR 5- [and 6-] isomers): To a 100ml flask was added compound **2** (0.18 g, 0.422 mmol) and DCM (5 ml), followed by **1-**hydroxypyrrolidine-2,5-dione (0.15 g, 1.303 mmol), and N1-((ethylimino)methylene)-N3,N3-dimethylpropane-1,3-diamine (0.15 g, 0.966 mmol). The mixture was stirred at room temperature for 5 minutes. The TLC showed completion of the reaction (DCM/EtOAc, 9:1). The mixture was loaded directly onto a 12 g of RediSep silica gel column and eluted out with gradient 0-15% of DCM/EtOAc solvent system. Obtained was 0.19 g of product (white powder, 86%). Calculated exact mass for $C_{25}H_{13}N_7O_7$: 523.09; Observed: 524.1212 (M+H).

Synthesis of DAZR_DNA conjugate: To a 1.5 mL of centrifugation vial containing 55 nmole of DNA in 50 μL of water was added 30 μL of N,N-diisopropylethylamine (DIPEA) and 3 mg of 5 (0.005 mmol) dissolved in 70 μL of N-methyl-2-pyrrolidone (NMP). After reacted for 16 h at RT, the reaction mixture was desalted by gel filtration using Sephadex G-25 and then purified by reversed-phase C18 column. Antizip2 reporter 1_DAZR (DNA: SEQ ID NO: 69): LC-MS: Calcd. for monoisotopic [M 7]-7: 1359.9580; Found 1359.9852. Antizip2 reporter 2_DAZR (DNA: SEQ ID NO: 73): LC-MS: Calcd. for monoisotopic $[M-7]^{-7}$ 1483.8350; Found 1483.8777.

Synthesis of diphenylphosphine-DNA conjugate: Oligonucleotides containing 5'-Amino-Modifier 5 (1 umole scale) were prepared using standard phosphoramidite chemistry (Glen Research, Sterling VA, USA). After trityl group of 5'-amino was removed, oligo-CPG was transferred to a 1.5 mL of centrifuge tube. A solution of **9** (81 mg, 0.15 mmol), N-(3-dimethylaminopropyl)-N'-ethylcarbodiimide hydrochloride (EDC.HCl) (28 mg, 0.15 mmol) and DIPEA (33 μL) in NMP (300 μL) was added. After reacting at 37 °C for 2 h, the solvent was removed by centrifugation and oligo-CPG was treated with ammonium methylamine (AMA) at 55 °C for 45 min. The crude oligos released from AMA cleavage was purified by reverse phase C18 HPLC. Antizip5_bisdiphenylphosphine (DNA: SEQ ID NO: 72): MS: Calcd. for monoisotopic $[M-4]^{-4}$ 1931.357; found 1931.465. Antizip5m_disdiphenylphosphine (DNA: SEQ ID NO: 71): MS: Calcd. for monoisotopic $[M-4]^{-4}$ 1925.1583; found 1925.0873.

**Example 2. DPC Reporter Chemistry: Hemicyanine formation through aldol-type condensation between indolinium and aldehyde**

**Scheme 4** illustrates the condensation of an indolinium and an aldehyde to form hemicyanine accomplished using diamine catalyzed DNA programmed chemistry. Both the aldehyde and indolinium precursors were conjugated to DNA through amide bond formation.

**Scheme 5** gives one example of synthesizing a DNA-conjugated indolinium compound. The acid functionality is introduced to indoline ring through *N*-quaternization. **Scheme 6** provides an example of synthesizing DNA conjugated aldehyde. The acid functionality in aldehyde precursor is introduced through hydrolysis of a cyano group by hydrogen peroxide (Brady, et al. J. Bio. Chem. 2001, 276, 18812-18818.). **Scheme 7** provides another example of DNA labeled α,β-unsaturated aldehyde **1**. Wittig reagent was used for the two-carbon homologation of aldehydes into the corresponding α, β-enals (Eitel, et al. Synthesis (1989) 364-367). The acid functionality in aldehyde precursor is introduced through hydrolysis of a cyano group by concentrated HCl (Bratenko, et al. Chemistry of Heterocyclic Compounds (2004) 40, 1279-1282).

**Scheme 4: DPC of hemicyanine formation.**

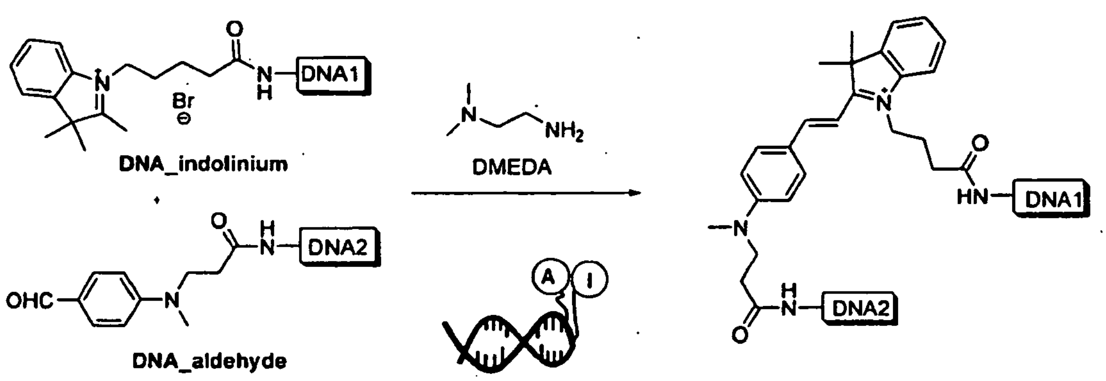


**The sequences of DNA1 and DNA2 are complementary to each other**

Additional examples of catalysts are shown in **FIG. 6.**

**Scheme 5: Example of synthesizing DNA and indolinium conjugate.**

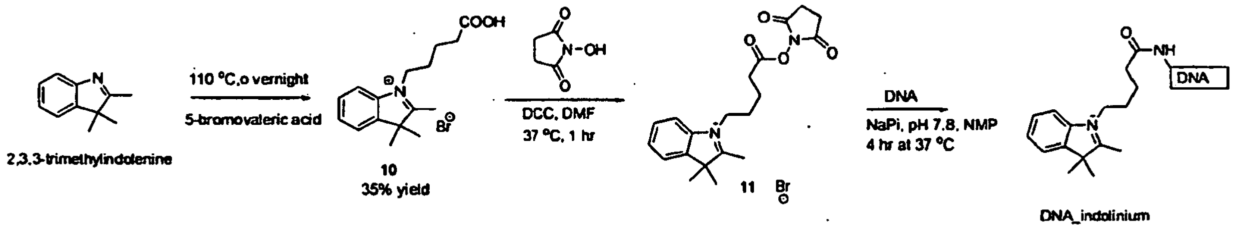


**Scheme 6: Example of synthesizing DNA and aldehyde conjugate (DNA_A0).**

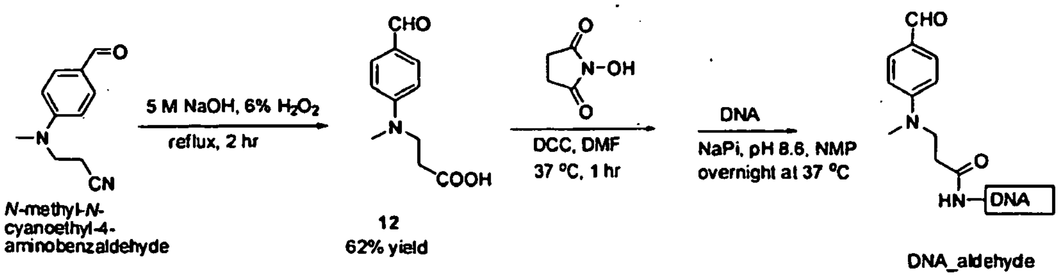


**Scheme 7: Example of synthesizing DNA conjugated aldehyde (DNA_A1).**

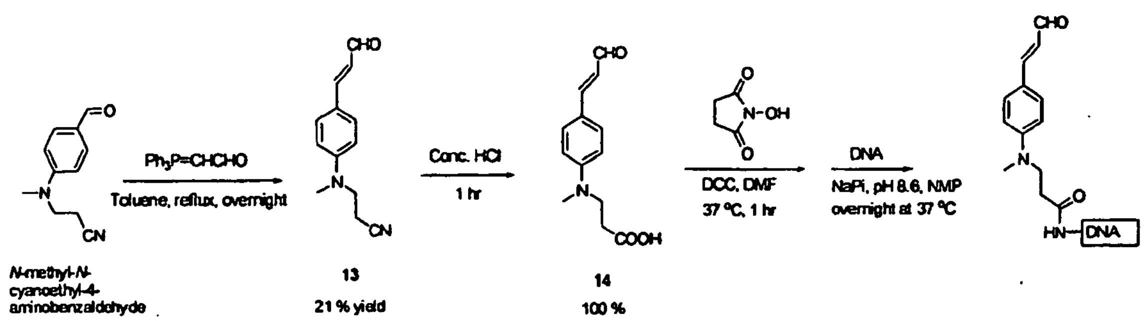


Synthesis of compound **10**: To 5-bromovaleric acid (2.435 g, 13.45 mmole) was added 2,3,3-trimethylindolenine (2.141 g, 13.45 mmole). The reaction mixture was heated with rigorous stirring at 110 °C overnight. The dark red sticky oil obtained was transferred to a Gregar extractor and extracted with EtOAc overnight. A light red solid was obtained. The solid was redissolved in 30 mL of MeOH. MeOH was removed under reduced pressure and the remaining residue was treated with 10 mL of EtOAc. Brownish solid was precipitated out and filtrated. The solid was washed with 2 x 50 mL of acetone and 2 x 100 mL of EtOAc. Total 1.590 g of light brownish solid was obtained (35% yield). $^1$H NMR (DMSO) $\delta_{ppm}$: 7.98 (m, 1H), 7.84 (m, 1H), 7.61 (m, 2H), 4.49 (t, 2H), 2.84 (s, 3H), 2.30 (t, 2H), 1.84 (m, 2H), 1.63 (m, 2H), 1.53 (s, 6H). MALDI-MS (positive mode): 260.2419.

Synthesis of compound **11**: Compound 11 (0.1 g, 0.294 mmole), N-hydroxy succimide (0.068 g, 0.588 mmole) and *N,N'*-dicyclohexylcarbodiimide (DCC) (0.085 g, 0.411 mmole) were dissolved in 1.5 mL of dimethyl formamide (DMF). The reaction mixture was stirred at 37 °C for 1 hr. The precipitated dicyclohexylurea (DCU) was removed by filtration, and the filtrate was treated with 15 mL of ether. Light orange solid was washed three times with 10 mL of ether and dried under vacuum for several hours. The solid obtained was used directly for the next reaction. MALDI-MS (positive mode): 357.1590.

Labeling DNA with indolinium compound: To a 1.5 mL of centrifugation vial containing 20 nmole of DNA was added 41.6 μL of 0.1 M sodium phosphate buffer (NaPi), pH 7.8, 41.6 μL of compound **10** in NMP (96 mM) and 41.6 μL of NMP. The vial was placed in a shaker and shaked for 4 hr at 37 °C. The reaction mixture was desalted by gel filtration using Sephadex G-25 and then purified by reversed-phase C8 column. Antizip5_indolinium (DNA: SEQ ID NO:

72): 10% yield. LC-MS (negative mode): Calcd for $C_{307}H_{401}N_{108}O_{180}P_{29}$ (average): 1340.2614 $[M-8H]^{7-}$; Found: 1340.2705 $[M-8H]^{7-}$. Antizip5m_indolinium (DNA: SEQ ID NO 71): 10% yield. LC-MS (negative mode): Calcd for $C_{308}H_{404}N_{109}O_{177}P_{29}$ (monoisotopic): 1336.4969 [M-8H]7-; Found: 1336.673 $[M-8H]^{7-}$ Antizip3_indolinium (DNA: SEQ ID NO: 70): 5% yield. LC-MS (negative mode): Calcd for C307H404N107O178P29 (monoisotopic): 1332.4034 [M-8H] 7-; Found: 1332.6293 [M-8H]7-.

Synthesis of compound **12**: In a 50 mL of round-shaped flask containing N-methyl-N cyanoethyl-4-aminobenzaldehyde (1.024 g, 5.44 mmole) was added 27.2 mL of 5 N NaOH solution and 6.8 mL of 30% $H_2O_2$. The reaction mixture was refluxed for 2 hr. After cooling, the reaction mixture was neutralized by the addition of concentrated HCl (37% w.t.) and extracted with 2 x 100 mL of EtOAc and 1 x 100 mL of $CH_2Cl_2$. The organic layers were combined and washed once with 50 mL of brine and concentrated to dryness. The crude product was purified by a 40 g RediSep silica-gel column on a CombiFlash Companion chromatography system (EtOAc/MeOH). Total 0.702 g of light pinkish solid was obtained (62%). Electrospray MS: M+H 208.0735. (Brady, et al., J. Biol. Chem. 2001, 276, 18812-18818).

Labeling DNA with aldehyde: The NHS ester of **12** was synthesized following the same procedure of compound **11.** After removing the DCU by filtration, the filtrate was used directly for DNA conjugation (calculated as 0.2 M product in DMF). To a 1.5 mL of centrifugation vial containing 50 nmole of DNA was added 104 μL of 0.1 M NaPi, pH 8.6, 125 μL of the above filtrate and 83 μL of NMP. The vial was placed in a shaker and shaken overnight at 37 °C. The reaction mixture was desalted by gel filtration using Sephadex G-25 and then purified by reversed-phase C8 column. Antizip2 reporter1_A0 (DNA: SEQ ID NO: 69) (30% yield). LC-MS: Calcd for $C_{303}H_{396}N_{110}O_{177}P_{29}$ (monoisotopic): 1328.2458 $[M-7H]7^{-}$; Found: 1328.3051 $[M-7H]^{7-}$.

Synthesis of compound **13:** In a 100 mL of round-shaped flask containing N-methyl-N-cyanoethyl-4-aminobenzaldehyde (1.116 g, 5.9 mmole) and ylide (2.71 g, 8.9 mmole) was added 57 mL of dry toluene. The reaction mixture was heated under reflux for overnight, allowed to cool, and filtered through filter paper. After removing the solvent from the filtrate, the residue was first purified by a 40 g RediSep silica-gel column on a CombiFlash Companion chromatography system (Toluene/Ether) and then preparative HPLC C18 column (Agilent Prep-C18, 30 x 100 mm, 10 um) to afford 0.27 g of pure product (21%). MALDI-MS (positive mode): 215.226.

Synthesis of compound **14:** In a 50 mL of round-shaped flask containing compound **13** (0.1 g, 0.47 mmole) was added 30 mL of concentrated HCl. The reaction mixture was heating to boiling and left at room temperature for 1 hr. HPLC analysis indicated that only one product was formed and no starting material remained in the reaction mixture. After removing most of the HCl, the compound was dissolved in water and lyophilized to dryness to afford the product.

Labeling DNA with α,β-unsaturated aldehyde: The NHS ester of compound **14** was synthesized following the same procedure as compound **11,** however was purified by silica-gel chromatography (EtOAc/Hexanes) instead. After drying under vacuum for several hours, the NHS ester of compound **14** was dissolved in NMP (96 mM) and was used for labeling DNA following the same procedure as labeling DNA_A0 Antizip2 reporter1_A1 (DNA: SEQ ID NO: 69): yield 40 %. Calcd for $C_{305}H_{398}N_{110}O_{177}P_{29}$ (monoisotopic): 1331.96239 $[M-7H]^{-7}$; Found: 1332.0778 $[M-7H]^{-7}$.

**Example 3. Generation of two fluorescent hemicyanine dyes via DPC**

Two hemicyanine products were formed by mixing antizip3_indolinium with antizip2 reporter1-A0 and antizip2 reporter1_A1 respectively (DNA: SEQ ID NO: 69). The product (P1) formed between antizip3_indolinium (DNA: SEQ ID NO: 70) and antizip2 reporter1_A1 (DNA: SEQ ID NO: 69) has excitation maximum at 540 nm and emission maximum at 600 nm, while the product (P3) formed between antizip3_indolinium (DNA: SEQ ID NO: 70) and antizip2 reporter1_A1 (DNA: SEQ ID NO: 69) has excitation maximum at 600 nm and emission maximum at 670 nm **(FIG. 18).**

DPC reaction: Reactions were performed with 200 nM each of reagent in 15 mM N,N-dimethyl ethylenediamine (DMEDA), 50 mM sodium phosphate buffer, pH 8.0, 2.5 mM MgCl2 at 30 °C. Total reaction volume was 50 μL. Catalyst DMEDA was added after mixing both reagents together in reaction buffer. Fluorescence was recorded immediately after the addition of catalyst DMEDA.

**Example 4. Synthesis of Affibody Oligonucleotide Conjugate**

DNAs are covalently attached to antibody or affibody via cross-linkers. Varieties of heterobifunctional cross-linkers can be used to synthesize DNA-antibody conjugate. The most commonly used are: 1) amine-reactive and sulfhydryl-reactive cross-linkers such as SMCC (succinimidyl-4-(*N*-maleimidomethyl)cyclohexane-1-carboxylate); 2) aldehyde-reactive and sulfhydryl-reactive cross-linkers such as hydrazine/hydroxylamine and maleimide/iodoacetate functional group containing compounds; 3) aldehyde-reactive and amine-reactive cross-linkers such as hydrazine/hydroxylamine and succinimidyl functional group containing compounds. See, *e.g.*, Hermanson, G. T. Bioconjugate Techniques, Academic Press 1996. For a precursor to be able to attach to a DNA, it is usually functionalized with a carboxylic acid group and then reacts with an amine-containing DNA. Other functional groups such as aldehyde and sulfhydryl can also be incorporated into DNA. The precursor is functionalized with hydrazone/hydroxylamine and maleimide group accordingly. **Scheme 8** provides an example of synthesizing affibody_oligonucleotide conjugate through BMPS. Zip2_ErbB2 (DNA: SEQ ID: NO 66) affibody has been synthesized following this scheme.

**Scheme 8**: NHS ester–maleimide heterobifuntional cross-linker-mediated affibody-Oligonucleotide Conjugation

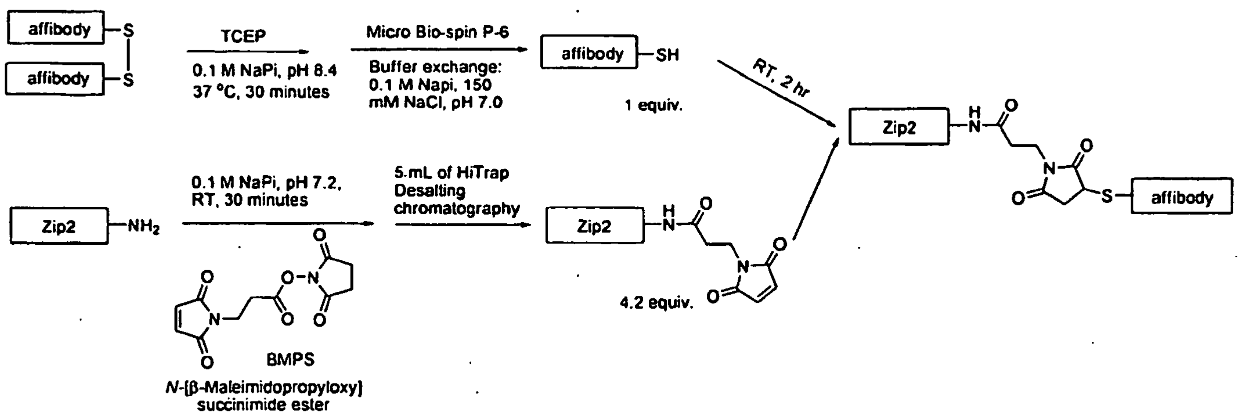


**Example 5. Detection of EGFR Homodimers by Hemicyanine DPC Assay**

Suspensions of cells (A431, BT474; or N87) expressing various levels of EGFR were analyzed for the presence of EGFR homodimers by an assay utilizing the DPC-based hemicyanine synthesis. Cells were incubated with EGF (epidermal growth factor, 200 ng/mL) in phosphate buffered saline (PBS) at 4 ˚C for up to 1 hr. Control cells (untreated) were incubated under the same conditions without EGF. After treatment, cells were fixed by incubation in 3% formaldehyde in PBS for 30 min. at 4 ˚C. Cells were then washed with PBS and blocked by incubation with PBS containing 2% bovine serum albumin (BSA), 100 μg/ml goat IgG, and 10 μM yeast tRNA at 4 ˚C for 45 min. The blocked cells were then incubated with anti-EGFR antibody-zip oligonucleotide conjugates (egfr1-zip2 (DNA: SEQ ID NO: 66) and egfr1-zip5 (DNA: SEQ ID NO: 68), 30 nM each) and antizip oligonucleotides (antizip5_A0, (DNA: SEQ ID NO: 72), and antizip2 reporter1_indolinium, DNA: (SEQ ID NO: 69), 60 nM each) for 1 hr. at room temperature. Reactants were removed and the cells washed with PBS containing 1 μM yeast tRNA. Cells were suspended in PBS containing 5 mM N,N-dimethyl ethylenediamine (DMEDA) and incubated for 4 hrs. at room temperature. Cells were then washed in PBS containing 2% BSA, suspended in PBS and analyzed by flow cytometry for fluorescence generated by the DPC reaction. The results of the DPC assay for EGFR homodimers expressed as the product of the percent positive cells times the mean fluorescence intensity (MFI) are shown in **FIG. 19.**

For cells untreated with EGF, a positive DPC signal (i.e., the fluorescent signal resulting from the DPC reaction) was detected indicating the presence of EGFR homodimers. The magnitude of the DPC signal was proportional to the expression level of EGFR (A431 cells express significantly higher levels of EGFR than BT474 or N87 cells). Treatment of cells with EGF resulted in an approximately two fold increase in the magnitude of the DPC signal. These results are consistent with a model of EGFR mechanism of action in which in the absence of EGF, unactivated EGFR homodimers are reversibly formed and the equilibrium level of these dimers is increased upon ligation of EGF, also resulting in the activation of these homodimers as evidenced by autophosphorylation of specific tyrosines in the cytoplasmic domain of the receptor (Citri, et al. Nature Rev. Mol. Biol. 2006, 7, 505-516).

**Example 6. Detection of erbB2 Dimers by DAZR Reduction DPC Assay.**

Both homodimers of ErbB2 and EGFR-ErbB2 heterodimers on N87 cells were detected by a DPC assay employing the DAZR reduction reaction. Suspensions of N87 cell were fixed by incubation with 2% formaldehyde in PBS at 4 ˚C for 30 min. Fixed cells were washed with PBS and then blocked by incubation with PBS containing 2% BSA, 10 μM yeast tRNA, 100 μg/mL goat IgG, and 5% dextran sulfate at 4 ˚C for 45 min. The blocked cells were then incubated with antibody-zipcodes (anti-erbB2 9G6-zip2 (DNA: SEQ ID NO: 66) and 9G6-zip5 (DNA: SEQ ID NO: 68) for the ErbB2 homodimer assay or anti-EGFR egfr1-zip5 (DNA: SEQ ID NO: 68) for the heterodimer assay, 30 nM concentration for antibody conjugages) and 60 nM antizip2 reporter 2_DAZR (DNA: SEQ ID NO: 73), for 1 hr at 30 ˚C. Cells were centrifuged, reactants removed, and washed twice prior to incubation with 80 nM antizip5_bisdiphenylphosphine (DNA: SEQ ID NO: 72) at 37˚C for 1 hr. Cells were then washed twice and suspended in PBS prior to analysis by flow cytometry for fluorescent cells. The results of the DPC assays for ErbB2 dimers.are

shown in **FIG. 20**, expressed as the fraction of positively stained cells times the mean fluorescent intensity.

The background signal in the assay was assessed by omitting the 9G6-zip5 conjugate, described above. The total amount of ErbB2 (monomer, homodimer, and heterodimer) was assessed by performing the DPC under conditions in which the DPC reaction was obtained independent of the state of EerbB2. The DPC signal obtained for ErbB2-ErbB2 homodimer was significantly greater than that obtained for the EGFR-ErbB2 heterodimer and both dimer DPC signals were significantly lower than that of the total ErbB2. N87 cells express high levels of ErbB2 and significantly lower levels of EGFR, thus the relative magnitudes of the DPC signals are consistent with these expression levels. As in the case of the EGFR homodimers on A431 cells, these results indicate the presence of homo and heterodimers on untreated cells.

**Example 7. Detection of ErbB2 Dimers on A431 cells by Indolinium Condensation DPC Assay.**

A DPC assay was performed on suspensions of A431 cells as described in Example 5 except that instead of EGFR homodimers, ErbB2 homodimers and EGFR-ErbB2 heterodimers were detected. In this assay, cells were either untreated, treated with 200 ng/ml EGF for 15 min at 4 ˚C, or pretreated with the EGFR specific tyrosine kinase inhibitor AG1478 (1 μM, 15 min at 37 ˚C) prior to treatment with EGF. After the various treatments, cells were fixed in 2% formaldehyde in PBS for 30 min at 4 ˚C. Cells were then washed with PBS and blocked by incubation with PBS containing 2% BSA, 5% dextran sulfate, 100 μg/ml goat IgG, and 10 μM yeast tRNA at 4 ˚C for 45 min. The blocked cells were then incubated with antibody-zip conjugates, antizip2 reporter1_A0, and antizip5_indolinium for 1hr at 30 ˚C. For ErbB2 homodimer detection, anti-ErbB2 9G6-zip5 and an anti-ErbB2 affibody-zip2 conjugates were used, while for EGFR-ErbB2 heterodimer detection, anti-EGFR egfr1-zip5 and anti-erBB2 affibody-zip2 conjugates were used. Reactants were removed and the cells washed with PBS containing 1 μM yeast tRNA. Cells were suspended in PBS containing 5 mM DMEDA and incubated for 3 hrs at room temperature. Cells were then washed in PBS containing 2% BSA , suspended in PBS, and analyzed by flow cytometry for fluorescence generated by the DPC reaction. The results of the DPC assay for ErbB2 dimers expressed as the product of the fraction of positively stained cells times the mean fluorescence intensity are shown in FIG. 21.

The background signal in the assay was assessed by omitting the one of the zip conjugates. The DPC signals for the ErbB2 homodimers were quite low yet above the background signal (signal/background of 3-4). Such a low DPC signal is consistent with the low level of expression of ErbB2 in A431 cells. The magnitude of the DPC signal obtained in the assay for EGFR-ErbB2 heterodimer was larger and varied significantly in response to treatment. As in the case of EGFR homodimers, there was a signal in the assay of untreated cells indicating a basal level of heterodimer. The DPC signal increased nearly two fold in cells treated with EGF and approximately three fold when the cells were pretreated with AG 1478 prior to EGF treatment.

The results of these DPC assays are consistent with many studies (e.g. immunoprecipitation followed by Western blotting) that have shown the existence of EGFR-ErbB2 heterodimers in untreated cells and the induction of additional heterodimers in response to EGF. Treatment with AG1478 alone has been shown to induce formation of EGFR-ErbB2 heterodimers (Arteaga, et al., J. Biol. Chem. (1997) 272, 23247-23254). Furthermore, pretreatment with the tyrosine kinase inhibitor prevents the autophosphorylation of the cytoplasmic domain of EGFR and prevents the down regulation of the receptor by endocytosis allowing its accumulation (Gannett, et al. J. Biol. Chem. (1997) 272, 12052-12056.)

## <u>GENERAL EXAMPLES ON DPC-BASED PROTEIN DETECTION</u>

**Example 8. Creation of Fluorescence by Hybridization Induced Azidocoumarin Reduction**

Five oligonucleotides were prepared using standard phosphoramidite chemistry (Glen Research, Sterling VA, USA). Oligonucleotides bearing 5'-amino groups (Oligo2 and Oligo6) were prepared using 5'-Amino-Modifier 5 and Oligonucleotides bearing 3'-aminogroups (Oligo4 and Oligo5) were prepared using 3'-Amino-Modifier C7 CPG (Glen Research, Sterling VA, USA)

| | | |
|---|---|---|
| Oligo1 | 5'-GTGGTAGTTGGAGCTGGTGGCGTAGGCAAGA-3' | (SEQ. ID. NO. 19) |
| Oligo2 | 5'-H2N-AGCTCCAACTACCAC-3' | (SEQ. ID. NO. 20) |
| Oligo4 | 5'-GTGGTAGTTGGAGCT-NH2-3' | (SEQ. ID. NO. 21) |
| Oligo5 | 5'-TCTTGCCTACGCCAC-NH2-3' | (SEQ. ID. NO. 22) |
| Oligo6 | 5'-H2N-AGATCCCACTAGCAC-3' | (SEQ. ID. NO. 23) |

Oligo1, Oligo4 and Oligo5 were removed from the synthesis support and purified by reversed-phase HPLC.

The amino groups of Oligo2 and Oligo6 were converted while resin-bound to their triphenyl phosphine derivatives and these were purified and isolated (Sakurai et al., J. Amer. Chem. Soc., 2005, 127, pp1660-1667) to give Oligo2-TPP and Oligo-6TPP, respectively.

Amino group bearing Oligo4 and Oligo5 were converted to their azidocoumarin derivatives (Oligo4-AzC and Oligo5-AzC, respectively) by reaction of each oligo with the N-hydroxysuccinimide ester of 7-azido-4-methylcoumarin-3-acetic acid (Thevenin et al., Eur. J. Biochem (1992) Vol. 206, pp-471-477). The reaction was performed by adding 1 μL of triflouroacetic acid to 5 μL of N-methylmorpholine to prepare a buffer to which was added 10 μL of water containing 6.6 mmol of Oligo 4 or Oligo 5, followed by addition of 30 μL of a 0.16 M solution of the coumarin NHS-ester in dimethylformamide. Each reaction was allowed to proceed for 2 hours at room temperature, whereupon 50 μL of 0.1 M aqueous triethylammonium acetate was added. The mixtures were applied to a NAP-5 desalting columns (Amersham Biosciences, Piscataway NJ USA) and eluted according to the manufacturers instructions the eluate was purified by RP-HPLC to provide Oligo4-AzC and Oligo5-AzC, in yields of 77% and 70%, respectively. Product identity was confirmed by Maldi-ToF mass spectrometry.

To demonstrate the hybridization-specific creation of fluorescence, various combinations of complementary and non-complementary oligonucleotides bearing azidocoumarin and triphenyl phosphine moieties were allowed to react at room temperature in a buffer comprised of 30% aqueous formamide, 50 mM NaCl, and 10 mM sodium phosphate, pH 7.2. The reaction progress was monitored over time using a Victor Multilabel fluorimeter (EG&G Wallach, Turku Finlnad) set to excite the sample at 360 nm and monitor light emission at 455 nm

FIG. 22 shows that when Oligo4-AzC and Oligo2-TPP are combined to final concentrations of 200 nM and 400 nM respectively, a rapid increase in fluorescence is observed. In this figure 004 denotes Oligo4-AzC, 002 denote Oligo2-TPP, and 006 denotes Oligo6-TPP. The fluorescence does not occur when Oligo6-TPP is substituted for Oligo2-TPP. Whereas Oligo2-TPP is perfectly complementary in its base-pairing ability to Oligo4-AzC, Oligo6-TPP is not, as it contains three mismatched nucleotides. The results support the conclusion that the creation of fluorescence is due to the ability of Oligo2-TPP to hybridize to Oligo4-AzC thus facilitating a reaction between the TPP and azidocoumarin moieties in the resulting hybrid. The lack of signal in the case of reaction of Oligo6-TPP with Oligo4-AzC is consistent with inability of these two oligonucleotides to form a duplex, therefore the reaction is not facilitated. Control reactions containing each single oligonucleotide were performed to rule out any non-specific effects.

Results of additional experiments involving ternary complexes are shown in **FIG. 23.** In these experiments Oligo1 is tested for its ability to bring together by hybridization two perfectly complementary oligonucleotides (Oligo5-AzC and Oligo-2TPP) versus its ability to bring together one perfectly complementary oligonucleotide (Oligo5-AzC) and one partially-complementary oligonucleotide (Oligo6-TPP). Oligo1 and Oligo5-AzC were at 200 nM final concentration, whereas Oligo2-TPP and Oligo6-TPP were employed at 400nM final concentration. In **FIG. 23,** 001 denotes Oligo1, 002 denotes Oligo2-TPP, 005 denotes Oligo5-AzC, and 006 denotes Oligo6-TPP. The results show that fluorescence is generated only when the combination of fully complementary oligonucleotides is present (Oligo1, Oligo5-AzC and Oligo2-TPP).

**Example 9. Oligonucleotide Hybridization, Concentration and Melting Temperatures**

A model system was prepared which included two twenty-mer oligonucleotides with a ten-base complementary region and ten-base single stranded spacer arms, further linked to a six carbon spacer arm. These oligos were synthesized both with and without a 5'-biotin (with a 6-carbon spacer arm). As shown below, the complementary region is underlined. A third oligo was identical to the (-) strand oligo but with 4 base mismatches (*italicized*) to the (+) strand.

| | | | | |
|---|---|---|---|---|
| Oligo 26 | (+) strand | 5' | CTTCGGCCCAGATATCGT | (SEQ. ID. NO. 24) |
| Oligo 27 | (-) strand | 3' | GTCTATAGCATCGACATC | (SEQ. ID. NO. 25) |
| Oligo 28 | (-) mismatch | 3' | TACTATAGTGTCGACATC | (SEQ. ID. NO. 26) |

Melting curves of the 10-base pair oligonucleotide pair (oligo 26+oligo 27) were examined by measuring fluorescence of SYBR dye binding to double stranded DNA in a Bio-Rad iCycler (Lipsky, et al., Clinical Chemistry 2001, 47 [4], 635-44). The binding curves are presented as the first derivative of the slope of the melting curve, such that a maximum value represents a point of inflection in the curve (a $T_m$, or in a mixed population of double stranded sites, a "local" $T_m$). Binding curves can be obtained up to at least 70° C as avidin retains biotin binding activity up to this temperature and beyond.

To check the dependence of this particular pair of oligonucleotides upon concentration, melting curves were generated for the oligonucleotide pair varied over the range from 500 to 20 nM **(FIG. 24).** (See, e.g., Lipsky, et al., Clinical Chemistry 2001, 47[4], 635-44). The observed $T_m$ dropped at the rate of about 10°C per each ten-fold reduction in concentration (where RFU indicates relative fluorescence units) of the oligonucleotide pair, similar to prediction in the

graph of **FIG. 24.** The melting curves were essentially identical for biotinylated and non biotinylated oligonucleotide pairs. The four base mismatched pair showed essentially no double stranded structure.

To test whether binding the (+) and (-) strands to a protein target would cause an increase in $T_m$, the biotinylated version of these oligonucleotides were incubated in the presence of avidin. Avidin contains 4 equivalent binding sites, which are spaced relatively close together and bind to biotin very tightly ($K_a \sim< 10^{-15}$ M) and non-cooperatively.

Presented with equal molar concentrations of oligonucleotides #26 and #27 in biotinylated form, it would be expected that about half of the biotin binding sites are occupied by complementary pairs of oligonucleotides, and about half with the same oligonucleotide (non-complementary pairs). The prediction is that one would observe two melting curve peaks in the presence of avidin. One peak would be the result of any pairs of oligonucleotides which were either not bound to avidin (free in solution) or which had only one partner of the two bound to avidin, which should not exhibit a proximity effect upon $T_m$. A second peak of significantly higher $T_m$ would represent a pair of biotinylated oligos both bound to avidin, which should exhibit a proximity effect.

Such an experiment was conducted as shown in **FIG. 25**. The oligonucleotides were added to a solution in the presence or absence of avidin held at 60° C, a so-called hot start. In a "hot start," the oligonucleotides bind to the biotin binding sites at a temperature well above their $T_m$ in solution, assuring that they are single stranded. The solution was then ramped down to 10° C and a melting curve analysis performed ascending to 70° C. As shown in **FIG. 25**, the melting curves of non-biotinylated oligo pair in the presence or absence of avidin showed a $T_m$ of 30-32° C (where RFU indicates relative fluorescence units). In the presence of avidin, however, two well separated $T_m$ peaks were generated with $T_m$ values of 33° C and 52° C. The elevated temperature peak ($T_m$ raised almost 20° C) was observed only in the presence of two complementary biotinylated oligonucleotides in the presence of avidin. The difference in $T_m$ +/- biotin tended to be greatest at lower salt concentrations **(FIG. 26)** and slightly higher in the presence of 10 mM magnesium chloride **(FIG. 27)** (where RFU indicates relative fluorescence units). The optimal molar ratio of biotinylated oligonucleotides to avidin was found to be about 3.5:1, (with total concentration of oligos + avidin = 0.7 μM) consistent with avidin possessing four equivalent binding sites **(FIG. 28)** (RFU indicates relative fluorescence units). This is important because it substantiates that the requirement that the oligonucleotides bind to the same molecule of avidin for the $T_m$ effect to occur. The substitution of a 3' biotinylated (-) strand oligo for a 5' biotinylated strand oligonucleotide showed little difference in $T_m$ values **(FIG. 29)** (RFU indicates relative fluorescence units) with previous results in which both oligonucleotides were 5' biotinylated.

Results were essentially identical if the experiment was conducted by adding equimolar amounts of both the oligonucleotides at room temperature, ramping to 60° C, and then obtaining the melting curves. In this method (as well as the hot start method) suitable melting curves can be generated by adding an excess molar of each oligo relative to avidin if desired. (Large excesses of pairs of oligos increases the size of the low $T_m$ peak, however, as predicted.) This was not detrimental in forming high $T_m$ hybrid DNA since the pairs of oligos competed equally for biotin binding sites as long as they were added together in equal molar amounts. If oligos were added one at a time, it was important to add about a 2:1 molar ratio of the first oligo to avidin followed by a 2:1 ratio of the second oligo. With sequential addition, adding an excess molar amount of either oligo relative to avidin occupies all the binding sites of the avidin with the first oligo and prevents occupying adjacent sites with the second, complementary oligo and exhibiting the elevated $T_m$ effect. These observations are consistent with the mechanism being binding of adjacent pairs of complementary oligos to two adjacent biotin binding sites to obtain hybrids exhibiting the elevated $T_m$ peaks.

Experiments were also conducted with a 10-base self-complementary oligonucleotide which was composed entirely of A and T. (Oligo 31: 5'-biotin-spacer arm-TTTTTTTTTTTTTAATTAAA) (SEQ. ID. NO. 27). Because this oligonucleotide was homogeneous in base composition and composed entirely of AT, it melted at a lower $T_m$ than the above-described model system and produced a fairly sharp melting curve. In the presence of avidin, its $T_m$ was increased from 30.5° C to 61.5° C **(FIG. 30)** (where RFU indicates relative fluorescence units). Since this oligonucleotide was self-complementary, all binding events lead to complementary strands, rather than only ½ of the events. Thus, only a single peak of increased $T_m$ was observed.

These experiments were repeated using anti-biotin antibody as a target rather than avidin. Anti-biotin antibody contains two biotin binding sites located near the ends of the Fab portion of the antibody, but the binding sites are much further apart than the biotin binding sites on avidin.

### Example 10. Detection of Protein Targets - Aptamers as Target Binders

Here, an exemplary system was designed to utilize nucleic acid-templated azidocoumarin (AzC) -triphenylphosphine (TPP) chemistry to detect a protein target upon aptamer binding and annealing of the two complementary DNA probes.

Materials

Human PDGF-BB and PDGF-AA was obtained from R&D Systems (220-BB and 220-AA, respectively). Anti-human PDGF-B Subunit monoclonal antibody was obtained from R&D Systems (MAB2201). Buffers included Tris/Mg buffer, at 50 mM Tris/HCl, pH 8.0 - 10 mM $MgCl_2$. Oligonucleotides used were as follows:

Oligonucleotide Sequences Used in this Example

| Oligo #/ (SEQ. ID #) | Sequence (5' to 3') | 5' -Mod'f | 3'Mod'f | Description |
|---|---|---|---|---|
| 201 (28) | CAGGCTACGGCACGTAGAGCATCACC ATGATCCTGCCCCCCCCCCATATTTAA GC | TPP | none | DPC-aptamer probe |
| 202 (29) | GCTTAAATATCCCCCCCCCCCAGGCT ACGGCACGTAGAGCATCACCATGATC CTG | none | AZC | DPC-aptamer probe |
| 203 (30) | GTGGGAATGGTGCCCCCCCCCCCAGG CTACGGCACGTAGAGCATCACCATGA TCCTG | none | AZC | DPC-aptamer probe-mismatch |
| 204 (31) | GTGGTAGTTGGAGTCGTGGCGTAGGC AAGA | none | none | target |
| 205 (32) | GTGGTAGTTGGAGTCACACGTGGCGT AGGCAAGA | none | none | target |
| 206 (33) | GTGGTAGTTGGAGCTCACACCACACG TGGCGTAGGCAAGA | none | none | target |
| 207 (34) | GTGGTAGTTGGAGTCACACACACCAC ACACAGTGGCGTAGGCAAGA | none | none | target |
| 208 (35) | GTGGTAGTTGGAGCTCACACCACACC AACCACACCACACCACACACACCACA CGTGGCGTAGGCAAGA | none | none | target |
| 209 (36) | | | | splint |
| 210 (37) | GTGGCGTAGGCAAGAGTGGTAGTTGG AGCT | none | none | K-ras target outward facing |
| 211 (38) | GTGGGAATGGTG | none | TPP | TPP probe |
| 212 (39) | AGATCCCACTAGCAC | TPP | none | TPP probe |
| 213 (40) | AGCTCCAACTACCAC | TPP | none | TPP "mismatch" |
| 214 (41) | TCTTGCCTACGCCAC | none | AZC | AZC probe |
| 215 (42) | CAGGCTACGGCACGTAGAGCATCACC ATGATCCTG | none | none | aptamer |

Methods

*DPC Reaction conditions.* Except as-noted, each 100 microliter reaction contained, in a total volume of 100 μl, 1 xTris/Mg buffer, 40 picomoles of TPP and AzC reaction probes, 40 picomoles of target oligonucleotide or of target protein, and typically 25-30% v/v of formamide. Samples were incubated at 25° C in a Wallac Victor 1420 spectrophotometer and the increase in fluorescence monitored with excitation at 355 nm and emission at 460 nm.

Results: Detection of PDGF-BB by Aptamer-DPC Probes

As illustrated in **FIG. 31,** an aptamer sequence directed against platelet-derived growth factor (PDGF) B-subunits was selected (Fang, et al., Chem. BioChem. 2003, 4, 829-34.). This belongs to a family of aptamers with strong affinity for PDGF B subunit (~$10^{-9}$ M), and about ten-fold reduced affinity for PDGF A subunit. (Green, et al., Biochemistry 35, 14413-24. 1996) The probe sequences were synthesized, each containing a complementary 10-mer DNA sequence, a $C_{10}$ spacer sequence, and the same 35-mer aptamer sequence. (Oligos #201, #202). Each sequence contained a 5'-TPP or 3'-AZC group with the aptamer linked 3' or 5', respectively. A second AzC probe, oligo #203, was the same as oligo #202 except that its annealing sequence was entirely mismatched to the TPP oligo (#201).

As shown in **FIG. 32,** in the presence of 30% (volume) formamide, the reaction of the TPP and AzC probes with each other was entirely dependent upon the presence of PDGF-BB and complementary DNA sequences on the probes. The reaction failed in the absence of either probe.

The DNA-dependence of the reaction was critically dependent upon the melting temperature of the DNA relative to the assay temperature. In the presence of 0% formamide (with the calculated and observed $T_m > T_{assay}$, the reaction took place in the presence or absence of the target protein PDGF-BB **(FIG. 33A).** In fact, under these conditions, addition of PDGF-BB did not increase, but reduced the reaction rate by about 50%. In 10% formamide, PDGF-BB was less inhibitory **(FIG. 33B).** In 20% formamide **(FIG. 34A),** the situation was completely reversed - the reaction rate was now weak except in the presence of PDGF-BB. In 30% formamide **(FIG. 34B)** the reaction was completely dependent upon the presence of PDGF-BB. In 40% formamide, the reaction was very slow with any set of reactants **(FIG. 35).** In all cases, the mismatched probes produced little or no reaction.

DNA melting experiments with the complementary sequences, monitored with SYBR Green had indicated a $T_m$ of the sequence of about 30° C in the Tris/Mg buffer in the absence of formamide, and about 7° C lower for every 10% increase in formamide. $T_m$ in the optimal formamide concentration for the detection assay, 30%, was 10° C.

In 0% formamide, the oligonucleotides can form at least a partial duplex even in the absence of PDGF-BB ($T_m$ slightly higher than $T_{assay}$). The DNA target-dependence of the reactions in 20% and 30% formamide is explained by the assay being conducted at a temperature greater than the $T_m$ in the absence of protein target. No reaction occurs unless the $T_m$ of the complex is increased by the binding of the two probes to the PDGF-BB target. At 40% formamide, the reaction doesn't occur with any set of reactions. The likely explanation is that either the $T_m$ had been reduced so low that binding to PDGF-BB could not raise it above $T_{assay}$, or that formamide had inhibited PDGF-BB binding to the aptamers. A more complex situation is the observed inhibition of reaction rate upon addition of PDGF-BB in the absence of formamide. Since half of the duplexes formed by PDGF-BB are non-productive (50% will be homoduplexes) the reduction in rate is likely due to PDGF-BB binding preventing these homoduplexes from disassociating and then reassociating in solution with complementary pairs to form heteroduplexes. This situation should not occur using pairs of probes specifically directed against different binding sites in a heterodimeric target.

The sensitivity of the assay (FIG. 36) was calculated by measuring reaction rates generated from a dilution series of PDGF-BB concentrations. The minimum detection level on the Wallac instrument was estimated at 0.8 picomoles in a 100 microliter assay volume, based upon the calculated value of three times the standard deviation of the background noise of the assay.

The assay sensitivity was also determined using PDGF-AA as a target. The aptamer monomer is expected to have an affinity for PDGF-AA about ten times weaker than for PDGF-BB. However, since the assay involves forming a complex of two aptamer-dimers to either type of PDGF, the avidity of binding of the dimer is expected to be tighter than the affinity of the monomer, and its affinity should be substantially tighter (lower $K_i$) than the concentrations tested of the target PDGFs (down to about 1 nanomolar). As shown in **FIG. 37,** the reaction rates of the aptamer DPC probes to PDGF-AA at low or high concentrations (0, 1.25, 2.5, 5, 10, 20, and 40 pmole of PDGF-AA) were not substantially different than the reaction rates with PDGF-BB. This is consistent with the model of an aptamer pair binding as a dimer and exhibiting increased avidity.

*Ratios of TPP to AzC Probes.* To confirm the model of the reaction mechanism **(FIG. 9,** the optimal ratio of TPP to AzC probes would be expected to be 1:1), **FIG. 38** was an experiment in which the total amount of the two probes was kept constant, at 800 nMoles probes/reaction, while the ratio of the two probes was varied. The ratio producing the highest reaction rate was approximately 1:1, consistent with the expected mechanism.

Thus, in this model system fluorescence was not generated unless the aptamers bound and the complementary sequences in the two probes annealed to each other.

**Example 11. Zip-Coded Architecture for Nucleic Acid-templated Chemistry Based-Biodetection with Aptamer Binders**

**FIG. 15** illustrates in more detail an exemplary zip-code architect. The TPP pair contained, first, a PDGF-aptamer on the 5'-end, a C18 polyethylene-glycol based spacer, and an 18-mer zip code sequence. The TPP reporter sequence contained a complementary anti-zip code sequence on its 3' terminus, a C18 PEG spacer, and a ten base pair reporter sequence terminating in a 5' TPP group. The pair of oligonucleotides comprising the AzC detection probe contained a 3'-aptamer linked through a C 18 PEG spacer to a separate zip code, and a detection oligonucleotide linked to a 5' anti-zip code, a C18 PEG spacer, and a reporter oligonucleotide (complementary to the TPP oligonucleotide) terminating in a 3' AzC group.

The reaction, in 35% formamide at 22˚C, was dependent upon the presence of both of the reporter oligonucleotides, both of the aptamer oligonucleotides, and the target, PDGF-BB **(FIG. 39).** At 22˚C in the absence of formamide, the reaction proceeded independently of the presence of PDGF. This is consistent with the behavior of the above-described "one-piece" architech, and reflects that the mechanism of fluorescence generation in 35% formamide is dependent the increased thermal stability of the reporter sequence duplex in formamide upon addition of PDGF. In the absence of formamide at 22˚C, the reporter oligonucleotide duplex is stable both in the presence and absence of PDGF.

Confirmation of the correctness of the model was obtained with experiments varying the ratio of the TPP and AzC aptamer oligos **(FIG. 40).** These experiments indicated that the optimal ratio of the aptamer oligos was the expected 1:1 ratio (i.e. 50% TPP oligo with a total concentration of PDGF and aptamer oligos of 0.4 μM). The optimal ratio of total reporter oligonucleotides to total aptamer oligos was also 1:1. No PDGF-dependent reaction occurred in the complete absence of either one of the reporter or aptamer oligonucleotides. At higher than stoicheometric concentrations of reporter oligonucleotides, the PDGF-independent signal increased (background) but the PDGF-dependent signal remained about constant. Both of these observations are consistent with the model that the complex is assembled in the ratio of 1:1:1 for each of the aptamer oligos, each of the reporter oligos, and PDGF.

These experiments indicate that the complex can self-assemble in solution, such that each zip code and its anti-zip code anneal to each other with minimal interference with the aptamer sequence or the reporter sequences.

Experiments were also conducted to determine if the order of addition, and thus assembly of the aptamer and reporter probes, was of any importance. Slightly slower reaction rates were obtained if the aptamer oligonucleotides were first incubated with PDGF before adding the reporter oligonucleotides, compared with adding all probes together as a mixture. Somewhat greater reaction rates were obtained if each pair of aptamer oligonucleotides and reporter oligonucleotides was first incubated and allowed to assemble with each other before the two sets were mixed together and incubated with PDGF. The reason for this may be that there is some steric hindrance to zip code-anti zip code annealing to aptamer probe if the aptamer probe is already bound to target.

As a control, a set of one-piece TPP and AzC probes was compared which contained only the zip code sequences and no zip code-anti zip code sequences **(FIG. 41)**. The reaction rates of this one-piece system were similar to that of the two-piece system, except that the rate enhancement due to the addition of PDGF was typically slightly better than that of the two-piece system.

The sequence of the aptamer-containing TPP and AzC probes was also systematically varied to determine any constraints on the design. The aptamer-containing TPP and AzC oligos were synthesized, both having the same sequences as described in **FIG. 15** but with the following changes: (1) omission of the C18-PEG spacer. (Oligos 119 & 122); (2) replacement of the C18-PEG spacer with the sequence $C_{10}$. (oligos 120 & 123); (3) replacement of the C18-PEG spacer with the sequence $C_{20}$. (oligos 121 & 124); (4) Omission of the C18-PEG spacer and omitting 3 3'-bases in the zip code region (reduction to 15 bases in length). (oligos 127 & 129); and (5) omission of the C 18-PEG spacer and omitting 6 3'-bases in the zip code region (reduction to 12 bases in length). (oligos 128 & 130).

Oligonucleotides used in this example included:

<u>**Oligo#/ Sequence (5'-3')**</u>
<u>**Modification**</u>

(SEQ. ID NO. 43)
106 GGACTCGAGCACCAATAC-X-TATAAATTCG-AZC    X=C18 PEG; AZC = 3'-AzC.
(SEQ. ID NO. 44)
109 CGAATTTATA-X-CTGACCATCGATGGCAGC    X=C18 PEG, 5'-TPP
(SEQ. ID NO. 45)
112 CAGGCTACGGCACGTAGAGCATCACCATGATCCTG-X-GCTGCCATCGATGGTCAG    X=C18 PEG
(SEQ. ID NO. 46)
113 GTATTGGTGCTCGAGTCC-X-CAGGCTACGGCACGTAGAGCATCACCATGATCCTG    X=C18 PEG
(SEQ. ID NO. 47)

119 GTATTGGTGCTCGAGTCCCAGGCTACGGCACGTAGAGCATCACCATGATCCTG
(SEQ. ID NO. 48)
120 GTATTGGTGCTCGAGTCCCCCCCCCCCCCAGGCTACGGCACGTAGAGCATCACCATGATCCTG
(SEQ. ID NO. 49)
121

**GTATTGGTGCTCGAGTCCCCCCCCCCCCCCCCCCCCCCCAGGCTACGGCACGTAGAGCATCACCATG ATCCTG**

(SEQ. ID NO. 50
122 CAGGCTACGGCACGTAGAGCATCACCATGATCCTGGCTGCCATCGATGGTCAG
(SEQ. ID NO. 51)
123 CAGGCTACGGCACGTAGAGCATCACCATGATCCTGCCCCCCCCCCGCTGCCATCGATGGTCAG
(SEQ. ID NO. 52)
124

**CAGGCTACGGCACGTAGAGCATCACCATGATCCTGCCCCCCCCCCCCCCCCCCCCGCTGCCATCGAT GGTCAG**

(SEQ. ID NO. 53)
127 CAGGCTACGGCACGTAGAGCATCACCATGATCCTGGCTGCCATCGATGGT
(SEQ. ID NO. 54)
128 CAGGCTACGGCACGTAGAGCATCACCATGATCCTGGCTGCCATCGAT
(SEQ. ID NO. 55)
129 TTGGTGCTCGAGTCCCAGGCTACGGCACGTAGAGCATCACCATGATCCTG
(SEQ. ID NO. 56)
130 GTGCTCGAGTCCCAGGCTACGGCACGTAGAGCATCACCATGATCCTG

None of these changes resulted in a significant difference in the performance of the system. Experiments 4) and 5) also resulted in a 3 and 6-base single stranded (not annealed to zip code) structure immediately upstream of the C 18 spacer in the reporter oligonucleotides.

The results of these experiments indicate that the aptamer-based PDGF detection system can be assembled separating the binding and DPC functions into two separate oligonucleotides. Through the selection of appropriate zip code sequences, the detection format described in **FIG. 14** self-assembled into pairs of annealed oligonucleotides which will function similarly to oligonucleotides synthesized in a single piece. The reporter and aptamer oligonucleotides may be separately assembled prior to introduction of target, or all species may be added together in almost any order. This process may be extended to the solution-phase assembly of more than one pair of annealed detection oligos, for example, to detect multiple targets. Detection of multiple targets may require using different reporter oligonucleotides which generate separately discernable signals (for example, different wavelengths of emitted light).

These results indicate that a zip-coded reporting approach can be effectively designed, for example, using aptamer-containing oligonucleotides.

While the results with the aptamer system indicate that a stable complex between binding and reporter sequences can be formed simply by annealing the zip code and anti-zip code regions, it should be noted that there are technologies to covalently and irreversibly link the two oligonucleotides together, with a high likelihood of retaining activity of the reporter reactive groups. For example, the oligonucleotides may be incubated in pairs (a binder oligonucleotide and a reactive oligonucleotide for nucleic acid-template chemistry) at a temperature at which the zip codes and anti-zip codes are mostly double stranded, but the rest of the sequences are single-stranded. Adding an intercalating, photoactivatable cross-linker such as Trioxalen, followed by UV irradiation, may irreversibly crosslink the two strands. Similarly, UV irradiation may introduce thymidine dimers between separate strands of annealed sequences. Alternately, a sequence may be introduced complementary to a short target (splice) DNA, abutting 3' and 5', which may then be ligated with DNA ligase. The splice oligonucleotide may alternately be composed of RNA, and removed after ligation with RNase H, which hydrolyzes RNA annealed to DNA. This can result in converting the two oligonucleotides into a single piece of single-stranded DNA. These methods can lead to cost-effective production of oligonucleotide reagents in detection kits against specific targets.

Relevant references for this example include Capaldi, et al., Nucleic Acid Res. 2000, 28[7], e21.; Castiglioni, et al., Appl. and Exper. Microbio. 2004, 7161-72; Fang, et al., Chem.BioChem. 2003, 4, 829-34.; Gerry, et al., J. Mol. Biol. 1999, 292, 251-62.

**Example 12. Zip-Coded Architecture for DPC-based Biodetection - Antibody Binders**

In another embodiment, the aptamer sequences are replaced with non-DNA binders such as antibodies. For PDGF and other protein targets, the aptamer sequences are replaced with chemically active groups, such as aldehydes, and reacted with non-DNA binder sequences such as antibodies or receptors to the protein targets **(FIG. 17)**. The optimal design for the binder and reporter oligonucleotides may be achieved with considerations on the size and geometry of the binder and size and geometry of the binding sites of the target. A longer, or shorter spacer arms, for example, may be used to optimally span the distance between binding sites on the target and avoid steric hindrance due to the binders themselves.

Referring to **FIG. 17**, the zip-coded oligonucleotide designed to hybridize to the TPP reporter molecule was synthesized containing a 5'-amino group. The zip-coded oligonucleotide designed to hybridize to the AzC reporter molecule contained a 3'-amino group. Synthesis of the conjugates between the oligonucleotides and anti-PDGF-BB antibody were performed by SoluLink Biosciences (San Diego, CA).

The SoluLink technology for conjugation of the antibody and oligonucleotides first requires modification of the primary amino groups of the antibody with succinimidyl 2-hydrazinonicotinate acetone hydrazone) to incorporate an acetone hydrazone onto the antibody. The primary amines of the oligonucleotides are separately activated with succinimdyl 4-formylbenzoate. The two activated molecules are mixed in the desired ratio (typically 6:1) and reacted at a mildly acidic pH to form a stable hydrazone linkage. The details of this chemistry are available at www.SoluLink.com. Two conjugates were prepared: one containing the zip code to anneal to the AzC-containing reporter oligonucleotide, and the other containing the zip code to anneal to the TPP-containing reporter oligonucleotide.

The antibody-oligonucleotide conjugates received from SoluLink were further purified by gel chromatography on a 1.6 x 60 cm column of Superdex S-200 (Amersham Biosciences) in PBS buffer (0.01 M potassium phosphate, pH 7.4 - 0.138 M sodium chloride). The main antibody peak, eluting at about 0.6 times the column volume, was collected and a later eluting peak of contaminating non-conjugated oligonucleotide was discarded. The antibody conjugate was concentrated by reversed dialysis with a Pierce (Rockford, IL) 30 K molecular weight cutoff Slide-A-Lyzer using Pierce Concentrating Solution. The protein content was determined using the Bio-Rad Micro BCA Reagent Kit and the oligonucleotide content determined using SYBR Gold DNA binding dye (Molecular Probes (Eugene, OR). The conjugates were both determined to contain an average of approximately 3 oligonucleotides per protein molecule.

Recombinant human PDGF-BB (220-BB) and mouse monoclonal anti-PDGF-BB (MAB220) were obtained from R&D Systems (Minneapolis MN).

Sequences used in this study included (where AzC indicates azidocoumarin and TPP indicates triphenylphosphine):

| <u>Name</u> | <u>Sequence (5'-3')</u> |
|---|---|
| TPP reporter | TPP-(amino modifier C6)-CGAATTTATA-C18PEG-TCAGCATCGTACCTCAGC (SEQ ID NO.: 9) (SEQ ID NO.: 58) |
| AzC reporter | GGACTCGAGCACCAATAC-C18 PEG-TATAAATTCG-(amino modifier C7)-AzC (SEQ ID NO.: 14) (SEQ ID NO.: 10) |
| AzC zip code | TTGGTGCTCGAGTCCCCCCCCCCCCCCCCCCCCCC-(amino modifier C7) (SEQ ID NO.: 59) |
| TPP zip code | (amino modifier C6)-CCCCCCCCCCCCCCCCCCCCCGCTGAGGTACGATGCTGA (SEQ ID NO.: 60) |

In addition, the 5' amino modifier C6 was obtained from Glen Research (from Glen Research phosphoramidite 110- 1906). The 3'-amino modifier C7 was obtained from Glen Research (from Glen Research CPG 20-2957). The C18 PEG was obtained from Glen Research (from Glen Research phosphoramidite 10-1918).

<u>Assembly of antibody-oligo conjugates with reporter oligonucleotides.</u>

The two antibody-oligo conjugates with their reporter were first assembled separately in a volume of 10 μl. Each assembly contained 0.5 μM (5 picomoles) of antibody-oligonucleotide conjugate and 0.15 μM of (15 pmoles) of complementary reporter oligonucleotide in 0.05 M Tris/HCl pH 8 - 0.01 M magnesium chloride. Each was incubated for at least 15 minutes at 4° C before use in the detection reaction mixture.

Detection Reaction of anti-PDGF-BB DPC Conjugate/Reporters with PDGF-BB

To conduct detection reaction, each reaction may contain in a volume of 50 μl: 10 μl of each conjugate assembly, prepared as described above, and variable amounts of PDGF-BB, in a buffer of 0.05 M Tris/HCl pH 8 - 0.01 M magnesium chloride-40% volume/volume formamide. The conjugates are present in this reaction mixture at 0.2 μM. Samples are incubated in the wells of a black 96-well microplate in a Wallac Victor Luminometer at 25° C. Fluorescence can be followed vs. time with excitation at 355 nm and emission at 460 nm.

Reactions typically may be carried out at 25° C, monitoring fluorescence generation at the wavelength optimums of the reaction product, 7-amino coumarin.

## Claims

**1.** A method for measuring the dimerization profile of a family of receptors, the method comprising:

(a) providing a pair of probes,

(i) the first probe comprising a first binding moiety having specific binding affinity to a first member of the receptor dimers to be profiled, wherein the first binding moiety is conjugated, optionally via a first linker, to a first oligonucleotide that is associated with a first reactive group;
(ii) the second probe comprising a second binding moiety having specific binding affinity to a second receptor of the receptor dimers to be profiled, wherein the second binding moiety is conjugated, optionally via a second linker, to a second oligonucleotide that is associated with a second reactive group;

wherein the second oligonucleotide is capable of hybridizing to the first oligonucleotide and the second reactive group is reactive with the first reactive group when brought into reactive proximity of one another;
(b) combining the first probe and the second probe with a sample to be measured for the dimerization of the first and second receptor members under conditions where the first and the second binding moieties bind to the first and second receptor members, respectively;
(c) allowing the second oligonucleotide to hybridize to the first oligonucleotide to bring into reactive proximity the first and the second reactive groups; and
(d) detecting a reaction between the first and the second reactive groups thereby determining the dimerization profile of the first and second receptor members.

**2.** A method for measuring the dimerization profile of a family of receptors, the method comprising:

(a) providing a pair of probes,

(i) the first probe comprising (1) a first binding moiety having specific binding affinity to a first member of the receptor dimers to be profiled, and (2) a first oligonucleotide zip code sequence;
(ii) the second probe comprising (1) a second binding moiety having specific binding affinity to a second member of the receptor dimers to be profiled, and (2) a second oligonucleotide zip code sequence; wherein the first probe is hybridized to a first reporter probe comprising (1) an anti-zip code oligonucleotide sequence complementary to the first oligonucleotide zip code sequence, (2) a first reporter oligonucleotide, and (3) a first reactive group; wherein the second probe is hybridized to a second reporter probe comprising (1) an anti-zip code oligonucleotide sequence complementary to the second oligonucleotide zip code sequence, (2) a second reporter oligonucleotide, and (3) a second reactive group;

wherein the second reporter oligonucleotide is capable of hybridizing to the first reporter oligonucleotide and the second reactive group is reactive with the first reactive group when brought into reactive proximity of one another;
(b) combining the first and second probes with a sample to be measured for the dimerization of the first and second receptor members under conditions where the first and the second binding moieties bind to the first and second receptor members, respectively;
(c) allowing the second reporter oligonucleotide to hybridize to the first reporter oligonucleotide to bring into reactive proximity the first and the second reactive groups; and
(d) detecting a reaction between the first and the second reactive groups thereby determining the dimerization

profile of the first and second receptor members.

3. The method of Claim 1 or Claim 2; wherein the family of receptors is the ErbB receptor family.

4. The method of Claim 3; wherein the ErbB receptor dimers are selected from the group consisting essentially of homo-dimers of ErbB1, ErbB2, ErbB3 and ErbB4, or from the group consisting essentially of hetero-dimers of ErbB1, ErbB2, ErbB3 and ErbB4.

5. First and second probes that are suitable for use in an assay for measuring the dimerization profile of a family of receptors; wherein:

(a) the first probe comprises a first binding moiety having specific binding affinity to a first member of the receptor dimers to be profiled, wherein the first binding moiety is conjugated, optionally via a first linker, to a first oligonucleotide that is associated with a first reactive group; and
(b) the second probe comprises a second binding moiety having specific binding affinity to a second receptor of the receptor dimers to be profiled, wherein the second binding moiety is conjugated, optionally via a second linker, to a second oligonucleotide that is associated with a second reactive group; wherein the second oligonucleotide is capable of hybridizing to the first oligonucleotide and the second reactive group is reactive with the first reactive group when brought into reactive proximity of one another.

6. First and second probes that are suitable for use in an assay for measuring the dimerization profile of a family of receptors; wherein:

(a) the first probe comprises (1) a first binding moiety having specific binding affinity to a first member of the receptor dimers to be profiled, and (2) a first oligonucleotide zip code sequence;and
(b) the second probe comprises (1) a second binding moiety having specific binding affinity to a second member of the receptor dimers to be profiled, and (2) a second oligonucleotide zip code sequence; wherein the first probe is hybridized to a first reporter probe comprising (1) an anti-zip code oligonucleotide sequence complementary to the first oligonucleotide zip code sequence, (2) a first reporter oligonucleotide, and (3) a first reactive group; wherein the second probe is hybridized to a second reporter probe comprising (1) an oligonucleotide sequence complementary to the second oligonucleotide zip code sequence, (2) a second reporter oligonucleotide, and (3) a second reactive group; and wherein the second reporter oligonucleotide is capable of hybridizing to the first reporter oligonucleotide sequence and the second reactive group is reactive with the first reactive group when brought into reactive proximity of one another.

7. First and second probes according to Claim 5 or Claim 6; wherein the family of receptors is the ErbB receptor family.

8. First and second probes according to Claim 7; wherein the ErbB receptor dimers are selected from the group consisting essentially of homo-dimers of ErbB1, ErbB2, ErbB3 and ErbB4, or from the group consisting essentially of hetero-dimers of ErbB1, ErbB2, ErbB3 and ErbB4.

9. A method for detecting a biological target, the method comprising:

(a) providing a first probe, the first probe comprising (1) a first binding moiety having binding affinity to the biological target, (2) a first oligonucleotide sequence, and (3) a first reactive group associated with the first oligonucleotide sequence;
(b) providing a second probe, the second probe comprising (1) a second binding moiety having binding affinity to the biological target, (2) a second oligonucleotide sequence, and (3) a second reactive group associated with the second oligonucleotide sequence, wherein the second oligonucleotide sequence is capable of hybridizing to the first oligonucleotide sequence and the second reactive group is reactive with the first reactive group when brought into reactive proximity of one another;
(c) combining the first probe and the second probe with a sample to be tested for the presence of the biological target under conditions where the first and the second binding moieties bind to the biological target;
(d) allowing the second oligonucleotide sequence to hybridize to the first oligonucleotide sequence to bring into reactive proximity the first and the second reactive groups; and
(e) detecting a reaction between the first and the second reactive groups thereby determining the presence of the biological target, wherein the reaction between the first and second reactive groups generates a rhodamine or an analog thereof.

10. The method of claim 9; wherein the first reactive group comprises a DAZR moiety or an analog thereof.

11. The method of claim 9; wherein the second reactive group comprises a phosphine moiety or an analog thereof.

## Patentansprüche

1. Verfahren zum Messen des Dimerisationsprofils einer Familie von Rezeptoren, wobei das Verfahren umfasst:

(a) Bereitstellen eines Paars von Sonden,

(i) wobei die erste Sonde eine erste Bindungsgruppierung mit spezifischer Bindungsaffinität zu einem ersten Mitglied der Rezeptordimere umfasst, von denen ein Profil erstellt werden soll, wobei die erste Bindungsgruppierung gegebenenfalls über einen ersten Linker an ein erstes Oligonukleotid konjugiert ist, das mit einer ersten reaktiven Gruppe assoziiert ist;
(ii) wobei die zweite Sonde eine zweite Bindungsgruppierung mit spezifischer Bindungsaffinität zu einem zweiten Rezeptor der Rezeptordimere umfasst, von denen ein Profil erstellt werden soll, wobei die zweite Bindungsgruppierung gegebenenfalls über einen zweiten Linker an ein zweites Oligonukleotid konjugiert ist, das mit einer zweiten reaktiven Gruppe assoziiert ist;

wobei das zweite Oligonukleotid an das erste Oligonukleotid hybridisieren kann und die zweite reaktive Gruppe mit der ersten reaktiven Gruppe reagiert, wenn sie in Reaktionsnähe zueinander gebracht werden;
(b) Vereinigen der ersten Sonde und der zweiten Sonde mit einer Probe, die auf die Dimerisation der ersten und zweiten Rezeptormitglieder untersucht werden soll, unter Bedingungen, bei denen die ersten und zweiten Bindungsgruppierungen an die ersten bzw. zweiten Rezeptormitglieder binden
(c) Ermöglichen, dass das zweite Oligonukleotid an das erste Oligonukleotid hyrbidisiert, so dass die erste und zweite reaktive Gruppen in Reaktionsnähe zueinander gebracht werden; und
(d) Nachweisen einer Reaktion zwischen der ersten und zweiten reaktiven Gruppe, wodurch das Dimerisationsprofil der ersten und zweiten Rezeptormitglieder bestimmt wird.

2. Verfahren zum Messen des Dimerisationsprofils einer Familie von Rezeptoren, wobei das Verfahren umfasst:

(a) Bereitstellen eines Paars von Sonden,

(i) wobei die erste Sonde (1) eine erste Bindungsgruppierung mit spezifischer Bindungsaffinität zu einem ersten Mitglied der Rezeptordimere, von denen ein Profil erstellt werden soll, und (2) eine erste Oligonukleotid-Zip-Code-Sequenz umfasst;
(ii) wobei die zweite Sonde (1) eine zweite Bindungsgruppierung mit spezifischer Bindungsaffinität zu einem zweiten Mitglied der Rezeptordimere, von denen ein Profil erstellt werden soll, und (2) eine zweite Oligonukleotid-Zip-Code-Sequenz umfasst, wobei die erste Sonde an eine erste Reportersonde hybridisiert wird, die (1) eine Anti-Zip-Code-Oligonukleotid-Sequenz, die zu der ersten Oligonukleotid-Zip-Code-Sequenz komplementär ist, (2) ein erstes Reporter-Oligonukleotid, und (3) eine erste reaktive Gruppe umfasst; wobei die zweite Sonde an eine zweite Reportersonde hybridisiert wird, die (1) eine Anti-Zip-Code-Oligonukleotid-Sequenz, die zu der zweiten Oligonukleotid-Zip-Code-Sequenz komplementär ist, (2) ein zweites Reporter-Oligonukleotid, und (3) eine zweite reaktive Gruppe umfasst;

wobei das zweite Reporter-Oligonukleotid an das erste Reporter-Oligonukleotid hybridisieren kann und die zweite reaktive Gruppe mit der ersten reaktiven Gruppe reagiert, wenn sie in Reaktionsnähe zueinander gebracht werden;
(b) Vereinigen der ersten Sonde und der zweiten Sonde mit einer Probe, die auf die Dimerisation der ersten und zweiten Rezeptor-Mitglieder untersucht werden soll, unter Bedingungen, bei denen die ersten und zweiten Bindungsgruppierungen an die ersten bzw. zweiten Rezeptormitglieder binden
(c) Ermöglichen, dass das zweite Reporter-Oligonukleotid an das erste Reporter-Oligonukleotid hyrbidisiert, so dass die ersten und zweiten reaktiven Gruppen in Reaktionsnähe zueinander gebracht werden; und
(d) Nachweisen einer Reaktion zwischen der ersten und zweiten reaktiven Gruppe, wodurch das Dimerisationsprofil der ersten und zweiten Rezeptormitglieder bestimmt wird.

3. Verfahren nach Anspruch 1 oder Anspruch 2, wobei die Familie der Rezeptoren die ErbB-Rezeptorfamilie ist.

4. Verfahren nach Anspruch 3, wobei die ErbB Rezeptordimere aus der Gruppe ausgewählt sind, die im Wesentlichen aus Homodimeren von ErbB1, ErbB2, ErbB3 und ErbB4 besteht oder aus der Gruppe, die im Wesentlichen aus Heterodimeren von ErbB1, ErbB2, ErbB3 und ErbB4 besteht.

5. Erste und zweite Sonden, die sich zur Verwendung in einem Assay zum Messen des Dimerisationsprofils einer Familie von Rezeptoren eignen; wobei

(a) die erste Sonde eine erste Bindungsgruppierung mit spezifischer Bindungsaffinität zu einem ersten Mitglied der Rezeptordimere umfasst, von denen ein Profil erstellt werden soll, wobei die erste Bindungsgruppierung gegebenenfalls über einen ersten Linker an ein erstes Oligonukleotid konjugiert ist, das mit einer ersten reaktiven Gruppe assoziiert ist; und
(b) die zweite Sonde eine zweite Bindungsgruppierung mit spezifischer Bindungsaffinität zu einem zweiten Rezeptor der Rezeptordimere umfasst, von denen ein Profil erstellt werden soll, wobei die zweite Bindungsgruppierung gegebenenfalls über einen zweiten Linker an ein zweites Oligonukleotid konjugiert ist, das mit einer zweiten reaktiven Gruppe assoziiert ist; wobei das zweite Oligonukleotid an das erste Oligonukleotid hybridisieren kann und die zweite reaktive Gruppe mit der ersten reaktiven Gruppe reagiert, wenn sie in Reaktionsnähe zueinander gebracht werden.

6. Erste und zweite Sonden, die sich zur Verwendung in einem Assay zum Messen des Dimerisationsprofils einer Familie von Rezeptoren eignen; wobei

(a) die erste Sonde (1) eine erste Bindungsgruppierung mit spezifischer Bindungsaffinität zu einem ersten Mitglied der Rezeptordimere, von denen ein Profil erstellt werden soll, und (2) eine erste Oligonukleotid-Zip-Code-Sequenz umfasst;
(b) die zweite Sonde (1) eine zweite Bindungsgruppierung mit spezifischer Bindungsaffinität zu einem zweiten Mitglied der Rezeptordimere, von denen ein Profil erstellt werden soll; und (2) eine zweite Oligonukleotid-Zip-Code-Sequenz umfasst, wobei die erste Sonde an eine erste Reportersonde hybridisiert wird, die (1) eine Anti-Zip-Code-Oligonukleotid-Sequenz, die zu der ersten Oligonukleotid-Zip-Code-Sequenz komplementär ist, (2) ein erstes Reporter-Oligonukleotid, und (3) eine erste reaktive Gruppe umfasst, wobei die zweite Sonde an eine zweite Reportersonde hybridisiert wird, die (1) eine Anti-Zip-Code-Oligonukleotid-Sequenz, die zu der zweiten Oligonukleotid-Zip-Code-Sequenz komplementär ist, (2) ein zweites Reporter-Oligonukleotid, und (3) eine zweite reaktive Gruppe umfasst; und

wobei das zweite Reporter-Oligonukleotid an das erste Reporter-Oligonukleotid hybridisieren kann und die zweite reaktive Gruppe mit der ersten reaktiven Gruppe reagiert, wenn sie in Reaktionsnähe zueinander gebracht werden

7. Erste und zweite Sonden nach Anspruch 5 oder 6, wobei die Familie der Rezeptoren die ErbB-RezeptorFamilie ist.

8. Erste und zweite Sonden nach Anspruch 7, wobei die ErbB Rezeptordimere aus der Gruppe ausgewählt sind, die im Wesentlichen aus Homodimeren von ErbB1, ErbB2, ErbB3 und ErbB4 besteht oder aus der Gruppe, die im Wesentlichen aus Heterodimeren von ErbB1, ErbB2, ErbB3 und ErbB4 besteht.

9. Verfahren zum Nachweisen eines biologischen Ziels, wobei das Verfahren umfasst:

(a) Bereitstellen einer ersten Sonde, wobei die erste Sonde (1) eine erste Bindungsgruppierung mit Bindungsaffinität zu dem biologischen Ziel, (2) eine erste Oligonukleotid-Sequenz, und (3) eine erste reaktive Gruppe, die mit der ersten Oligonukleotid-Sequenz assoziiert ist, umfasst;
(b) Bereitstellen einer zweiten Sonde, wobei die zweite Sonde (1) eine zweite Bindungsgruppierung mit Bindungsaffinität zu dem biologischen Ziel, (2) eine zweite Oligonukleotid-Sequenz, und (3) eine zweite reaktive Gruppe, die mit der zweiten Oligonukleotid-Sequenz assoziiert ist, umfasst, wobei die zweite Oligonukleotid-Sequenz an die erste Oligonukleotid-Sequenz hybridisieren kann, und die zweite reaktive Gruppe mit der ersten reaktiven Gruppe reagiert, wenn sie in Reaktionsnähe zueinander gebracht werden;
(c) Vereinigen der ersten Sonde und der zweiten Sonde mit einer Probe, die auf die Anwesenheit des biologischen Ziels untersucht werden soll, unter Bedingungen, bei denen die erste und die zweite Bindungsgruppierung an das biologische Ziel binden;
(d) Ermöglichen, dass die zweite Oligonukleotid-Sequenz an die erste Oligonukleotid-Sequenz hyrbidisiert, so dass die ersten und zweiten reaktiven Gruppen in Reaktionsnähe zueinander gebracht werden; und
(e) Nachweisen einer Reaktion zwischen der ersten und zweiten reaktiven Gruppe, wodurch die Anwesenheit

des biologischen Ziels nachgewiesen wird, wobei die Reaktion zwischen der ersten und zweiten reaktiven Gruppe ein Rhodamin oder ein Analogon davon erzeugt.

**10.** Verfahren nach Anspruch 9, wobei die erste reaktive Gruppe eine DAZR-Gruppierung oder ein Analogon davon umfasst.

**11.** Verfahren nach Anspruch 9, wobei die zweite reaktive Gruppe eine Phosphin-Gruppierung oder ein Analogon davon umfasst.

**Revendications**

**1.** Procédé de mesure du profil de dimérisation d'une famille de récepteurs, le procédé comprenant :

(a) la mise à disposition d'une paire de sondes,

(i) la première sonde comprenant un premier fragment de liaison, ayant une affinité de liaison spécifique avec un premier membre des récepteurs dimères devant être profilés, le premier fragment de liaison étant conjugué, de préférence par l'intermédiaire d'un premier lieur, à un premier oligonucléotide qui est associé à un premier groupe réactif ;
(ii) la deuxième sonde comprenant un deuxième fragment de liaison ayant une affinité de liaison spécifique avec un deuxième récepteur des récepteurs dimères devant être profilés, le deuxième fragment de liaison étant conjugué, en option par l'intermédiaire d'un deuxième lieur, à un deuxième oligonucléotide qui est associé à un deuxième groupe réactif,

le deuxième oligonucléotide étant capable de s'hybrider au premier oligonucléotide, et le deuxième groupe réactif étant réactif vis-à-vis du premier groupe réactif quand ils sont mis à proximité réactive l'un de l'autre ;
(b) la combinaison de la première sonde et de la deuxième sonde à un échantillon devant faire l'objet d'une mesure de la dimérisation du premier et du deuxième éléments récepteurs, dans des conditions dans lesquelles le premier et le deuxième fragments de liaison se lient respectivement au premier et au deuxième membres récepteurs ;
(c) le fait de permettre au deuxième oligonucléotide de s'hybrider au premier oligonucléotide, pour mettre à proximité réactive le premier ou le deuxième groupes réactifs ; et
(d) la détection d'une réaction entre le premier et le deuxième groupes réactifs, de façon à déterminer le profil de dimérisation du premier et du deuxième membres récepteurs.

**2.** Procédé de mesure du profil de dimérisation d'une famille de récepteurs, le procédé comprenant :

(a) la mise à disposition d'une paire de sondes,

(i) la première sonde comprenant (1) un premier fragment de liaison ayant une affinité de liaison spécifique avec un premier membre des récepteurs dimères devant être profilés, et (2) une première séquence de localisation nucléotidique ;
(ii) la deuxième sonde comprenant (1) un deuxième fragment de liaison ayant une affinité de liaison spécifique avec un deuxième membre des récepteurs dimères devant être profilés, et (2) une deuxième séquence de localisation oligonucléotidique ; la première sonde étant hybridée à une première sonde rapporteur comprenant (1) une séquence oligonucléotidique anti-localisation, complémentaire de la première séquence de localisation oligonucléotidique, (2) un premier oligonucléotide rapporteur, et (3) un premier groupe réactif ; la deuxième sonde étant hybridée à une deuxième sonde rapporteur comprenant (1) une séquence oligonucléotidique anti-localisation, complémentaire de la deuxième séquence de localisation oligonucléotidique, (2) un deuxième oligonucléotide rapporteur, et (3) un deuxième groupe réactif ;

le deuxième oligonucléotide rapporteur étant capable de s'hybrider au premier oligonucléotide rapporteur, et le deuxième groupe réactif étant réactif vis-à-vis du premier groupe réactif quand ils sont mis à proximité réactive l'un de l'autre ;
(b) la combinaison de la première et de la deuxième sondes à un échantillon devant être mesuré pour ce qui est de la dimérisation du premier et du deuxième membres récepteurs, dans des conditions dans lesquelles le premier et le deuxième fragments de liaison se lient respectivement au premier et au deuxième membre

récepteurs ;
(c) le fait de permettre au deuxième oligonucléotide rapporteur de s'hybrider au premier oligonucléotide rapporteur pour mettre à proximité réactive le premier et le deuxième groupes réactifs ; et
(d) la détection d'une réaction entre le premier et le deuxième groupes réactifs, de façon à déterminer le profil de dimérisation du premier et du deuxième membres récepteurs.

**3.** Procédé de la revendication 1 ou 2, dans lequel la famille de récepteurs est la famille de récepteurs ErbB.

**4.** Procédé de la revendication 3, dans lequel les récepteurs dimères ErbB sont choisis dans le groupe consistant essentiellement en les homodimères de l'ErbB1, de l'ErbB2, de l'ErbB3 et de l'ErbB4, ou dans le groupe consistant essentiellement en les hétérodimères de l'ErbB1, de l'ErbB2, de l'ErbB3 et de l'ErbB4.

**5.** Première et deuxième sondes, qui peuvent être utilisées dans un essai de mesure du profil de dimérisation d'une famille de récepteurs, où :

(a) la première sonde comprend un premier fragment de liaison ayant une affinité de liaison spécifique avec un premier membre des récepteurs dimères devant être profilés, le premier fragment de liaison étant conjugué, en option par l'intermédiaire d'un premier lieur, à un premier oligonucléotide qui est associé à un premier groupe réactif ; et
(b) la deuxième sonde comprend un deuxième fragment de liaison ayant une activité de liaison spécifique avec un deuxième récepteur des récepteurs dimères devant être profilés, le deuxième fragment de liaison étant conjugué, en option par l'intermédiaire d'un deuxième lieur, à un deuxième oligonucléotide qui est associé à un deuxième groupe réactif ; le deuxième oligonucléotide étant capable de s'hybrider au premier oligonucléotide, et le deuxième groupe réactif étant réactif vis-à-vis du premier groupe réactif quand ils sont mis à proximité réactive l'un de l'autre.

**6.** Première et deuxième sondes, qui conviennent à une utilisation dans un essai de mesure du profil de dimérisation d'une famille de récepteurs, où :

(a) la première sonde comprend (1) un premier fragment de liaison ayant une affinité de liaison spécifique avec un premier membre des récepteurs dimères devant être profilés, et (2) une première séquence de localisation d'oligonucléotides ; et
(b) la deuxième sonde comprend (1) un premier fragment de liaison ayant une affinité de liaison spécifique avec un deuxième membre des récepteurs dimères devant être profilés, et (2) une deuxième séquence de localisation oligonucléotidique, la première sonde étant hybridée à une première sonde rapporteur comprenant (1) une séquence oligonucléotidique anti-localisation, complémentaire de la première séquence de localisation d'oligonucléotides, (2) un premier oligonucléotide rapporteur, et (3) un premier groupe réactif ; la deuxième sonde étant hybridée à une deuxième sonde rapporteur comprenant (1) une séquence oligonucléotidique anti-localisation, complémentaire de la première séquence de localisation oligonucléotidique, (2) un deuxième oligonucléotide rapporteur, et (3) un deuxième groupe réactif ; et le deuxième oligonucléotide rapporteur étant capable de s'hybrider à la première séquence oligonucléotidique rapporteur, et le deuxième groupe étant réactif vis-à-vis du premier groupe réactif quand ils sont mis à proximité réactive l'un avec l'autre.

**7.** Première et deuxième sondes selon la revendication 5 ou 6, où la famille de récepteurs est la famille des récepteurs ErbB.

**8.** Première et deuxième sondes selon la revendication 7, où les récepteurs dimères ErbB sont choisis dans le groupe consistant essentiellement en les homodimères de l'ErbB1, de l'ErbB2, de l'ErbB3 et de l'ErbB4, ou dans le groupe consistant essentiellement en les hétérodimères de l'ErbB1, de l'ErbB2, de l'ErbB3 et de l'ErbB4.

**9.** Procédé de détection d'une cible biologique, le procédé comprenant :

(a) la mise à disposition d'une première sonde, la première sonde comprenant (1) un premier fragment de liaison ayant une affinité de liaison avec la cible biologique, (2) une première séquence oligonucléotidique, et (3) un premier groupe réactif associé à la première séquence oligonucléotidique ;
(b) la mise à disposition d'une deuxième sonde, la deuxième sonde comprenant (1) un premier fragment de liaison ayant une affinité de liaison avec la cible biologique, (2) une deuxième séquence oligonucléotidique, et (3) un deuxième groupe réactif, associé à la deuxième séquence oligonucléotidique, la deuxième séquence

oligonucléotidique étant capable de s'hybrider à la première séquence oligonucléotidique, et le deuxième groupe réactif étant réactif vis-à-vis du premier groupe réactif quand ils sont mis à proximité réactive l'un de l'autre ;
(c) la combinaison de la première sonde et de la deuxième sonde à un échantillon devant être soumis à un test portant sur la présence de la cible biologique dans des conditions dans lesquelles le premier et le deuxième fragments de liaison se lient à la cible biologique ;
(d) le fait de permettre à la deuxième séquence oligonucléotidique de s'hybrider à la première séquence oligonucléotidique, pour mettre à proximité réactive le premier et le deuxième groupes réactifs ; et
(e) la détection d'une réaction entre le premier et le deuxième groupes réactifs, de façon à déterminer la présence d'une cible biologique, la réaction entre le premier et le deuxième groupes réactifs produisant une rhodamine ou un analogue de cette dernière.

**10.** Procédé de la revendication 9, dans lequel le premier groupe réactif comprend un fragment DAZR ou un analogue de ce dernier.

**11.** Procédé de la revendication 9, dans lequel le deuxième groupe réactif comprend un fragment phosphine ou un analogue de ce dernier.

FIG. 1

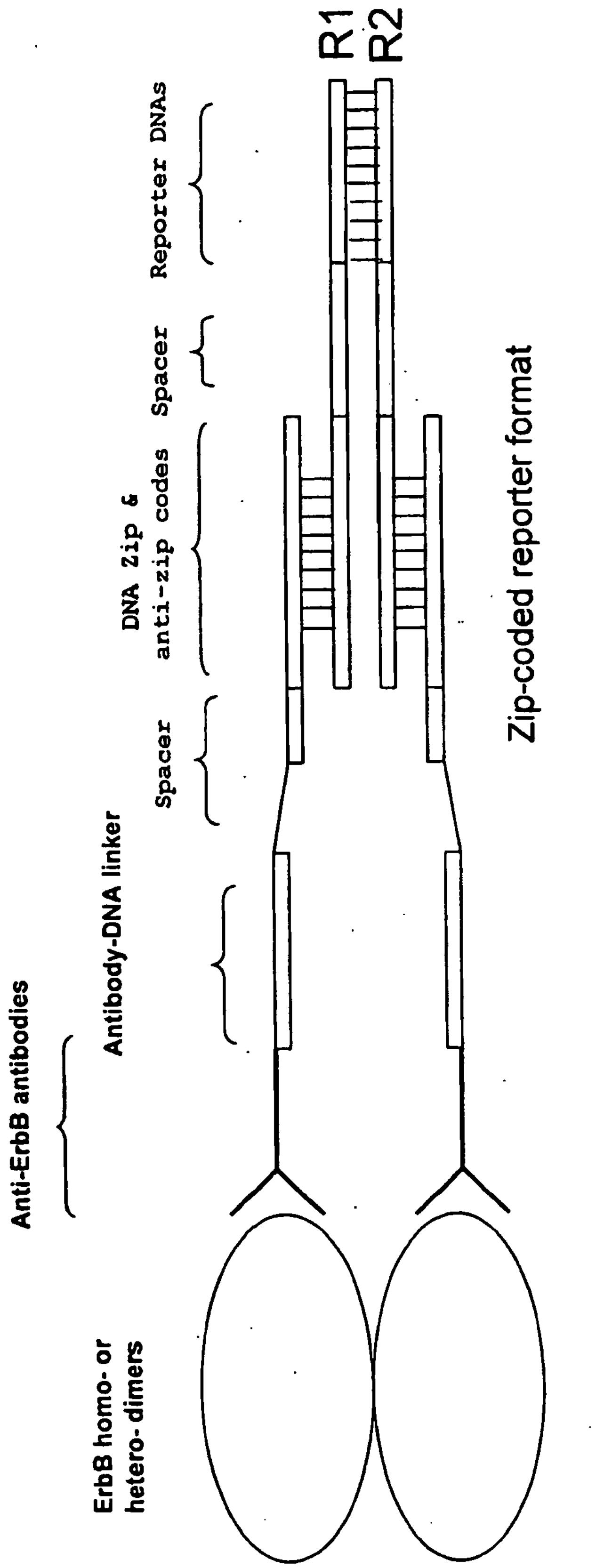


Zip-coded reporter format

FIG. 2

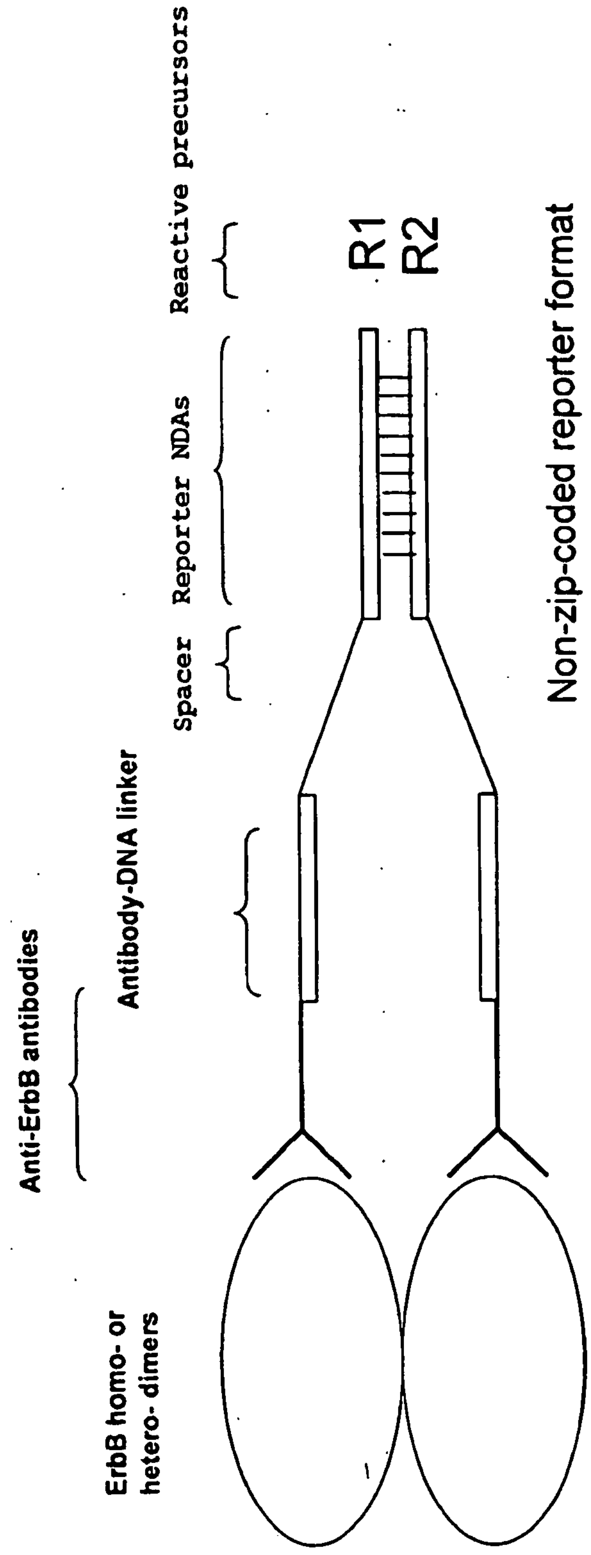
Reactive precursors
Reporter NDAs
Spacer
Antibody-DNA linker
Anti-ErbB antibodies
ErbB homo- or hetero- dimers
R1
R2
Non-zip-coded reporter format

FIG. 3

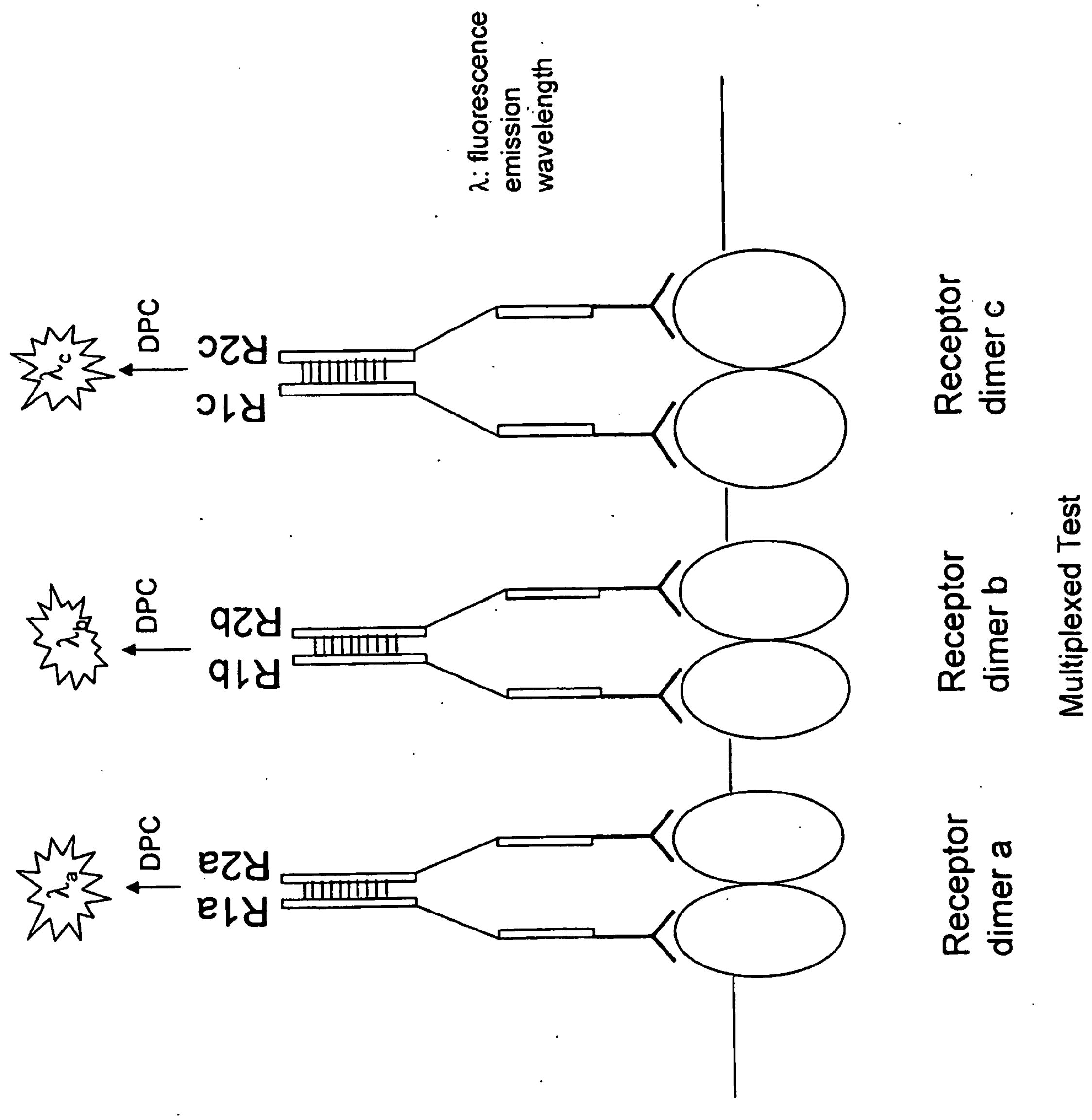
λ: fluorescence emission wavelength
$\lambda_a$
$\lambda_b$
$\lambda_c$
DPC
DPC
DPC
R1a
R2a
R1b
R2b
R1c
R2c
Receptor dimer a
Receptor dimer b
Receptor dimer c
Multiplexed Test

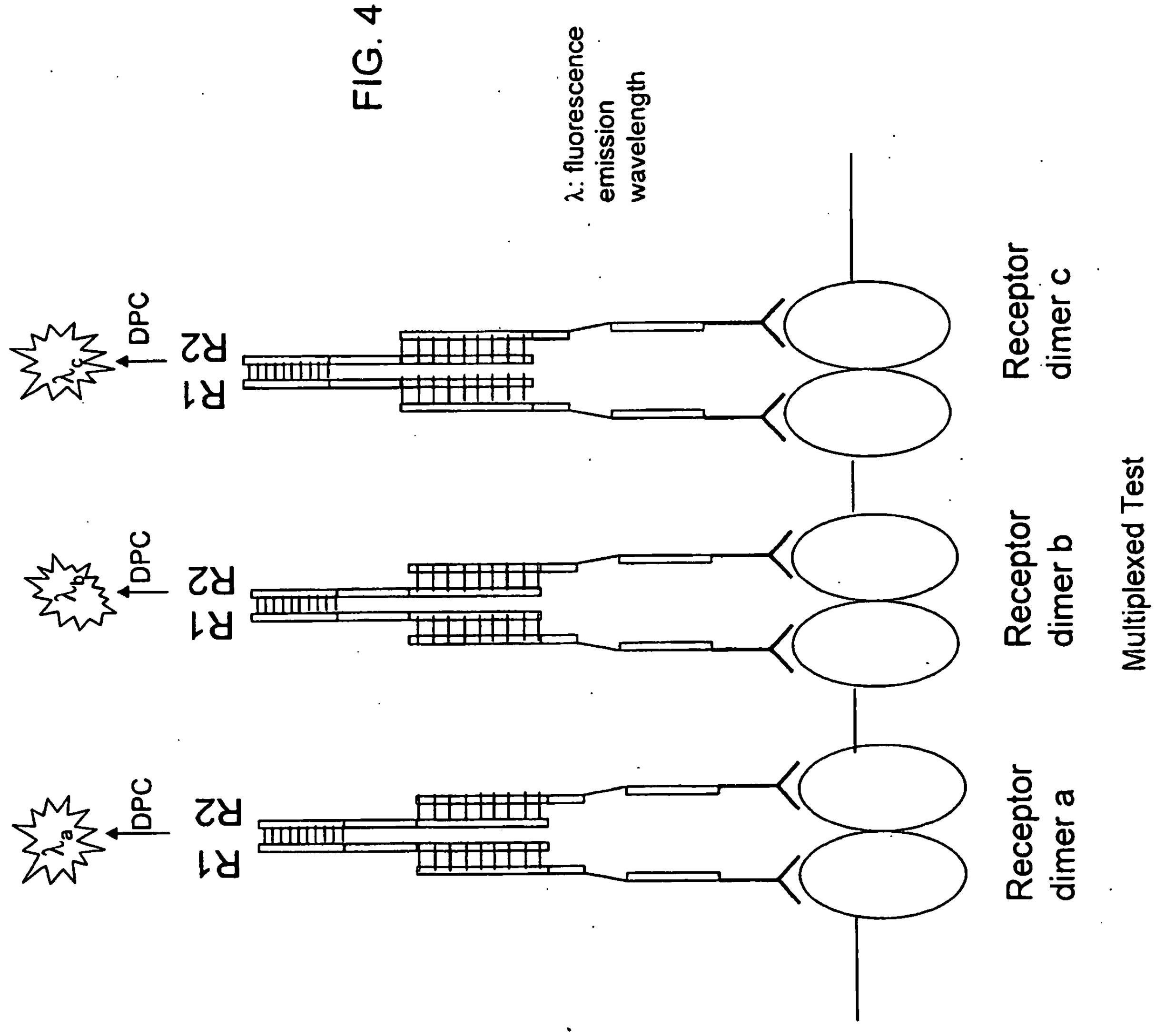
λ: fluorescence emission wavelength
DPC
R1
R2
Receptor dimer a
Receptor dimer b
Receptor dimer c
Multiplexed Test

FIG. 4

FIG. 5

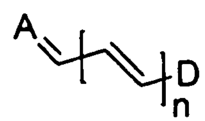

Polymethine Dyes

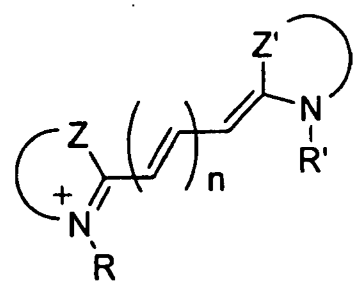

Cyanine

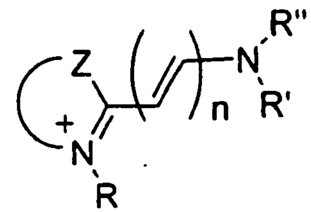

Hemicyanine

A = aceceptor D = donor Z, Z' = O, S, N, C ... n = 0, 1, 2 ... R, R', R''= alkyl chain

*Typical A and D for polymethine dyes: thiazoles, pyrroles, pyrrolines, indoles, 1,3,3-trimethylindolines, tetrazoles, pyrimidine, pyridines, quinolines and higher fused N-heterocycles or any substituted benzyl rings.*

General structure of polymethine dye, cyanine and hemicyanine

# FIG. 6

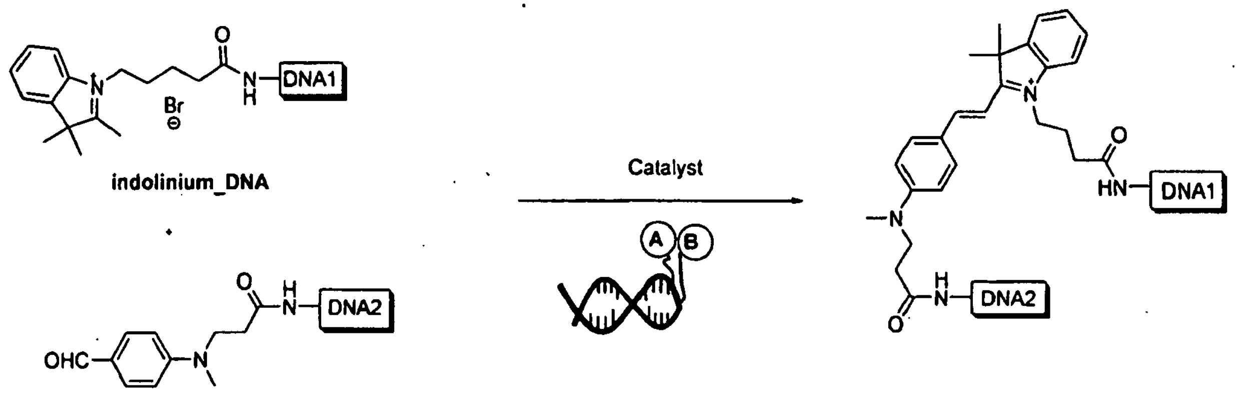


indolinium_DNA

aldehyde_DNA

Examples of catalysts:

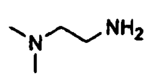

$N^1$,$N^1$-dimethylethane-1,2-diamine
DMEDA

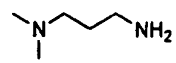

$N^1$,$N^1$-dimethylpropane-1,3-diamine

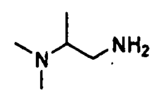

$N^2$,$N^2$-dimethylpropane-1,2-diamine

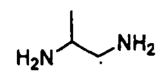

propane-1,2-diamine

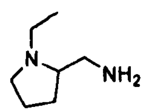

(*R*)-(1-ethylpyrrolidin-2-yl)methanamine

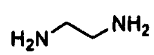

ethylenediamine

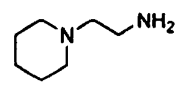

1-(2-aminoethyl)-piperidine

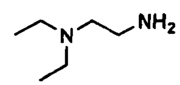

$N^1$,$N^1$-diethylethylene-1,2-diamine

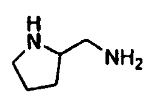

(*S*)-2-aminomethyl pyrrolidine

Example of synthesizing hemicyanine through DPC

FIG. 7

Hemicyanine dye:

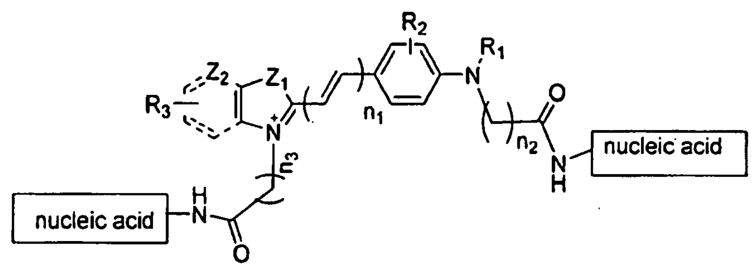

n1 = 1, 2, 3, 4, 5
n2 and n3 = 0 to 16
Z1 = O, S, Se, P, $NH_2$, $NR_1$, $C(CH_3)_2$ where $R_1$ is alkyl group
$R_3$ = any substituted benzyl or higher fused benzyl rings, H, alkyl, $SO_3H$, OH, CN, Cl, Br, $NO_2$, $NH_2$, $N(R_1)_2$, $OR_1$ while $R_1$ is alkyl group
$Z_2$ = benzene or any N-heterocycles
R1 = H, alkyl
$R_2$ = Ph or N-heterocycle, H, alkyl, $SO_3H$, OH, CN, Cl, Br, $NO_2$, $NH_2$, $N(R_1)_2$, $OR_1$ while $R_1$ is alkyl group

Quaternary salt precursor:

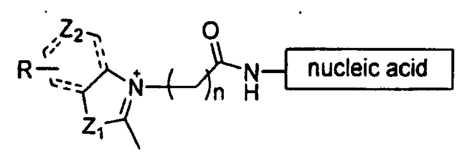

n = 0 to 16
Z1 = O, S, Se, P, $NH_2$, $NR_1$, $C(CH_3)_2$ where $R_1$ is alkyl group
R = any substituted benzyl or higher fused benzyl rings, H, alkyl, $SO_3H$, OH, CN, Cl, Br, $NO_2$, $NH_2$, $N(R_1)_2$, $OR_1$ while $R_1$ is alkyl group
$Z_2$ = benzene or any N-heterocycles

Aldehyde precursor:

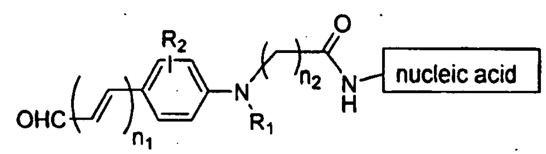

n1 = 1, 2, 3, 4, 5
n2 = 0 to 16
R1 = H, alkyl
$R_2$ = Ph or N-heterocycle, H, alkyl, $SO_3H$, OH, CN, Cl, Br, $NO_2$, $NH_2$, $N(R_1)_2$, $OR_1$ while $R_1$ is alkyl group

General chemical structures of hemicyanine dyes useful for multiplex and their aldehyde and quaternary salt precursors.

FIG. 8

| Quaternary salt bearing active hydrogen component | Aldehyde | Hemicyanine Dye | λabs (nm) | λem (nm) |
|---|---|---|---|---|
| 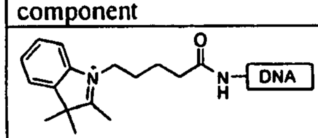 Indolinium (I) | 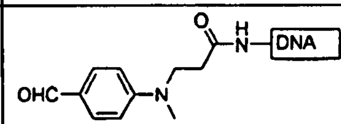 Aldehyde (A0) | 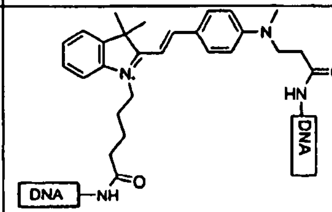 P1 | 560 | 585 |
| 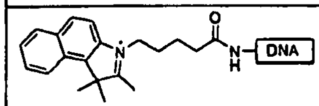 Benzoindolinium (BI) | 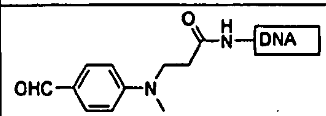 Aldehyde (A0) | 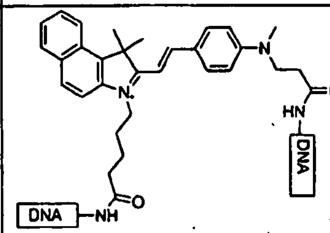 P2 | 560 | 602 |
| 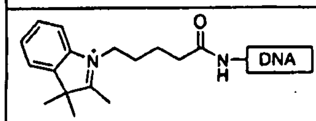 Indolinium (I) | 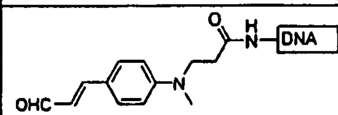 Aldehyde 1 (A1) | 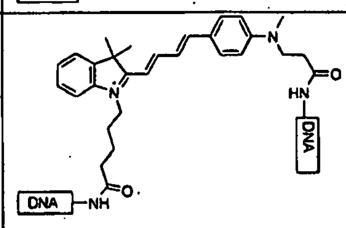 P3 | 600 | 670 |
| 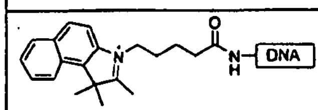 Benzoindolinium (BI) | 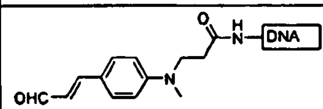 Aldehyde 1 (A1) | 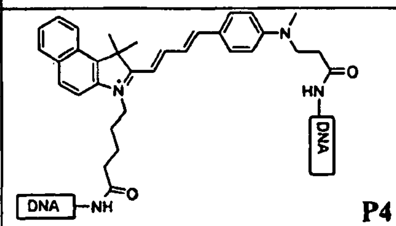 P4 | 620 | 685 |

Chemical structures of a four-plex hemicyanine_DNA dyes and their spectroscopic properties

FIG. 9

Protein Detection

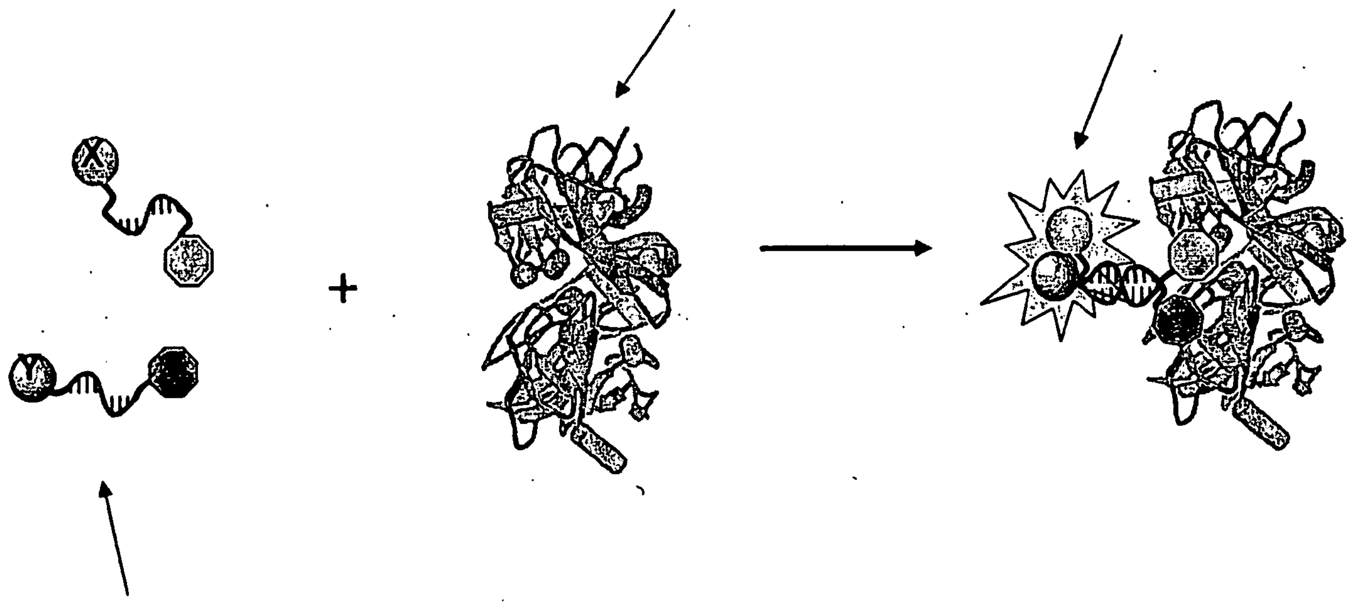

FIG. 10

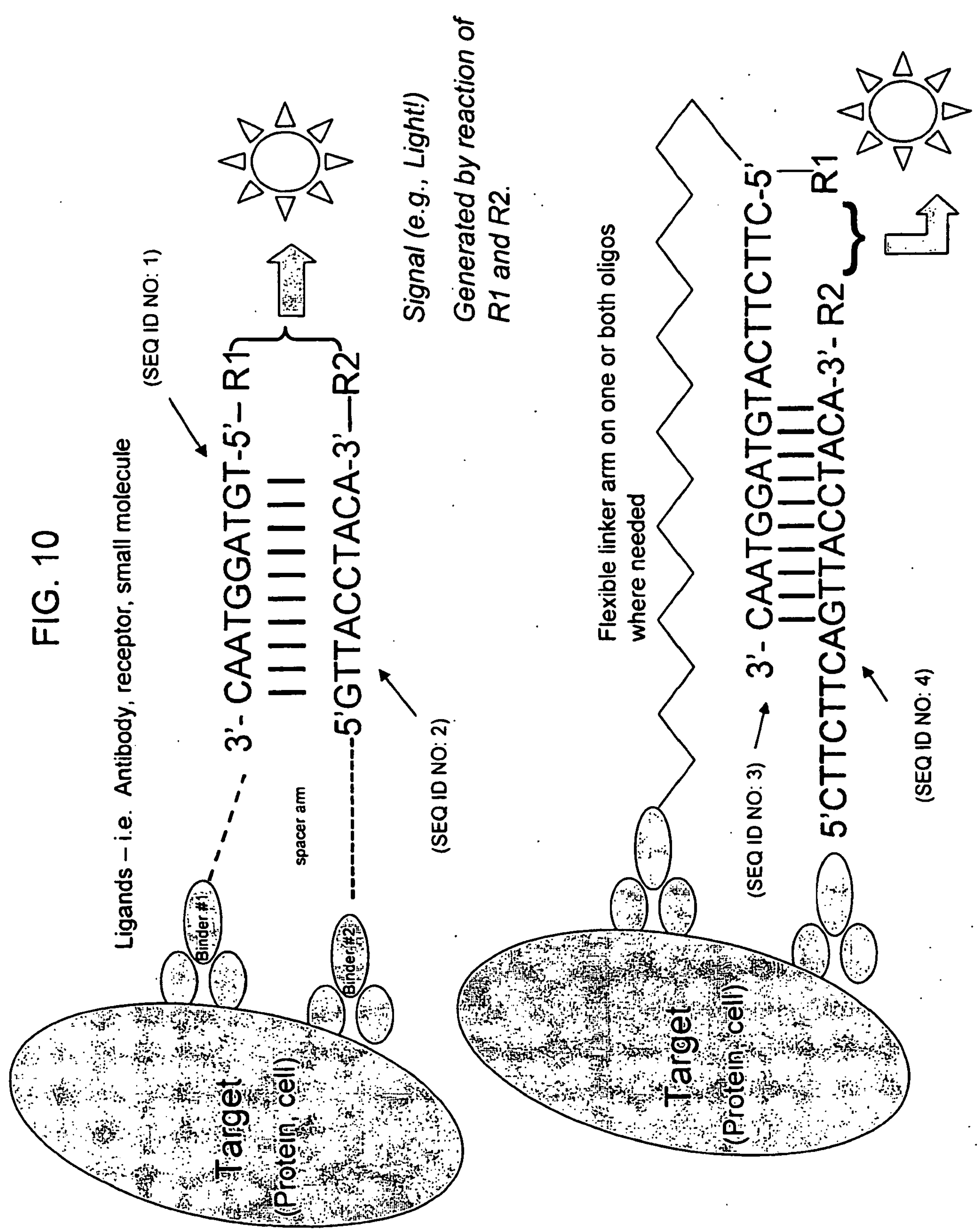
Ligands – i.e. Antibody, receptor, small molecule
(SEQ ID NO: 1)
3'- CAATGGATGT-5'– R1
5'GTTACCTACA-3'—R2
spacer arm
(SEQ ID NO: 2)
Binder #1
Binder #2
Target
(Protein, cell)
Signal (e.g., Light!)
Generated by reaction of R1 and R2.
Flexible linker arm on one or both oligos where needed
(SEQ ID NO: 3)
3'- CAATGGATGTACTTCTTC-5'
R1
5'CTTCTTCAGTTACCTACA-3'- R2
(SEQ ID NO: 4)
Target
(Protein, cell)

FIG. 11

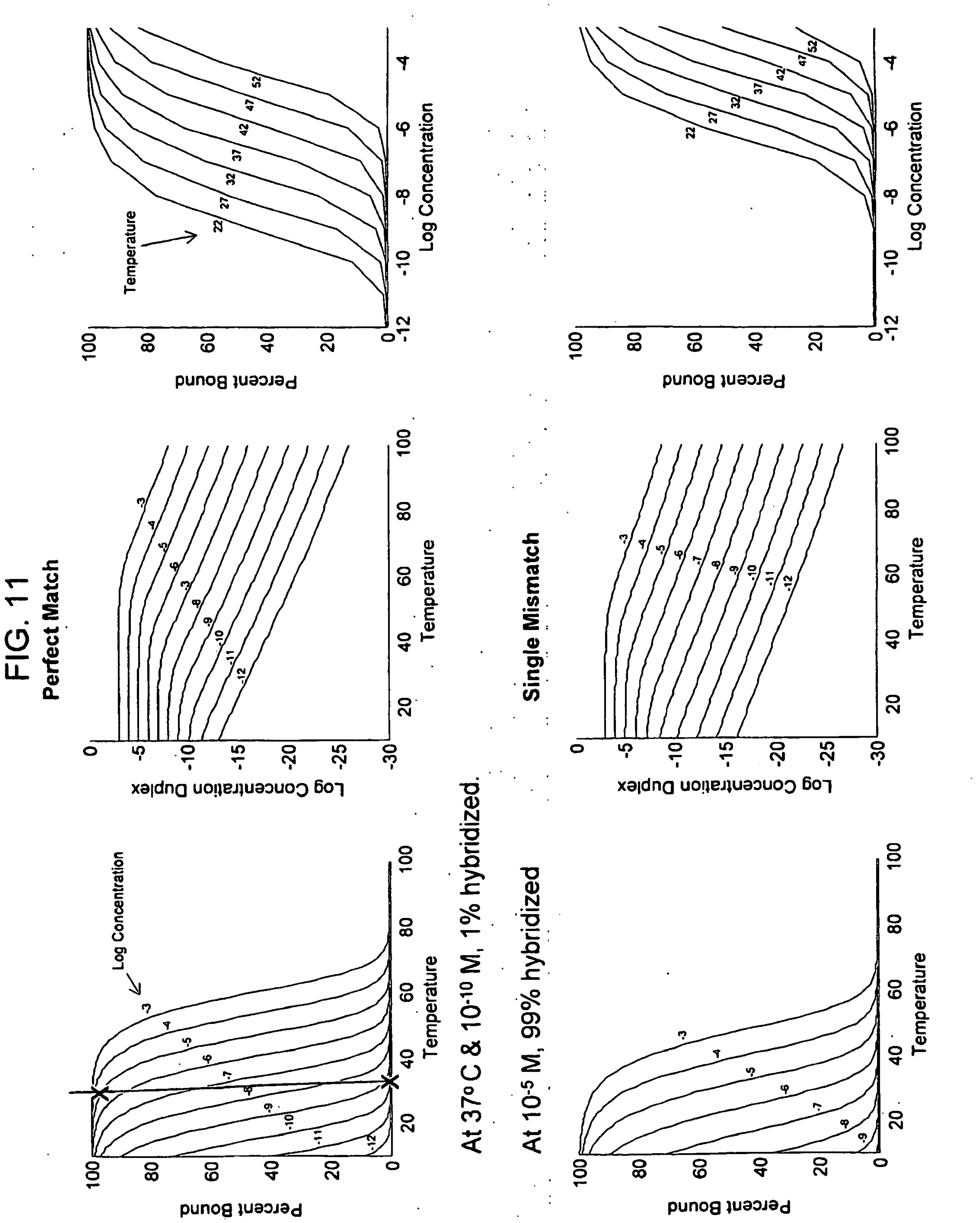
Perfect Match
Single Mismatch
At 37° C & 10-10 M, 1% hybridized.
At 10-5 M, 99% hybridized
Percent Bound
Temperature
Log Concentration
Log Concentration Duplex
Log Concentration
Temperature

FIG. 12

Oligos Used to Construct Melting Curves

Perfect Match:

```
5' (+) CAATGGATGTACTTCTTC      (SEQ ID NO: 5)
       ||||||||||
(-) CTTCTTCAGTTACCTACA 5'      (SEQ ID NO: 6)
```

Single Mismatch:

```
5' (+) CAATGGATGTACTTCTTC      (SEQ ID NO: 7)
       ||| ||||||
(-) CTTCTTCAGTTTCCTACA 5'      (SEQ ID NO: 8)
```

FIG. 13

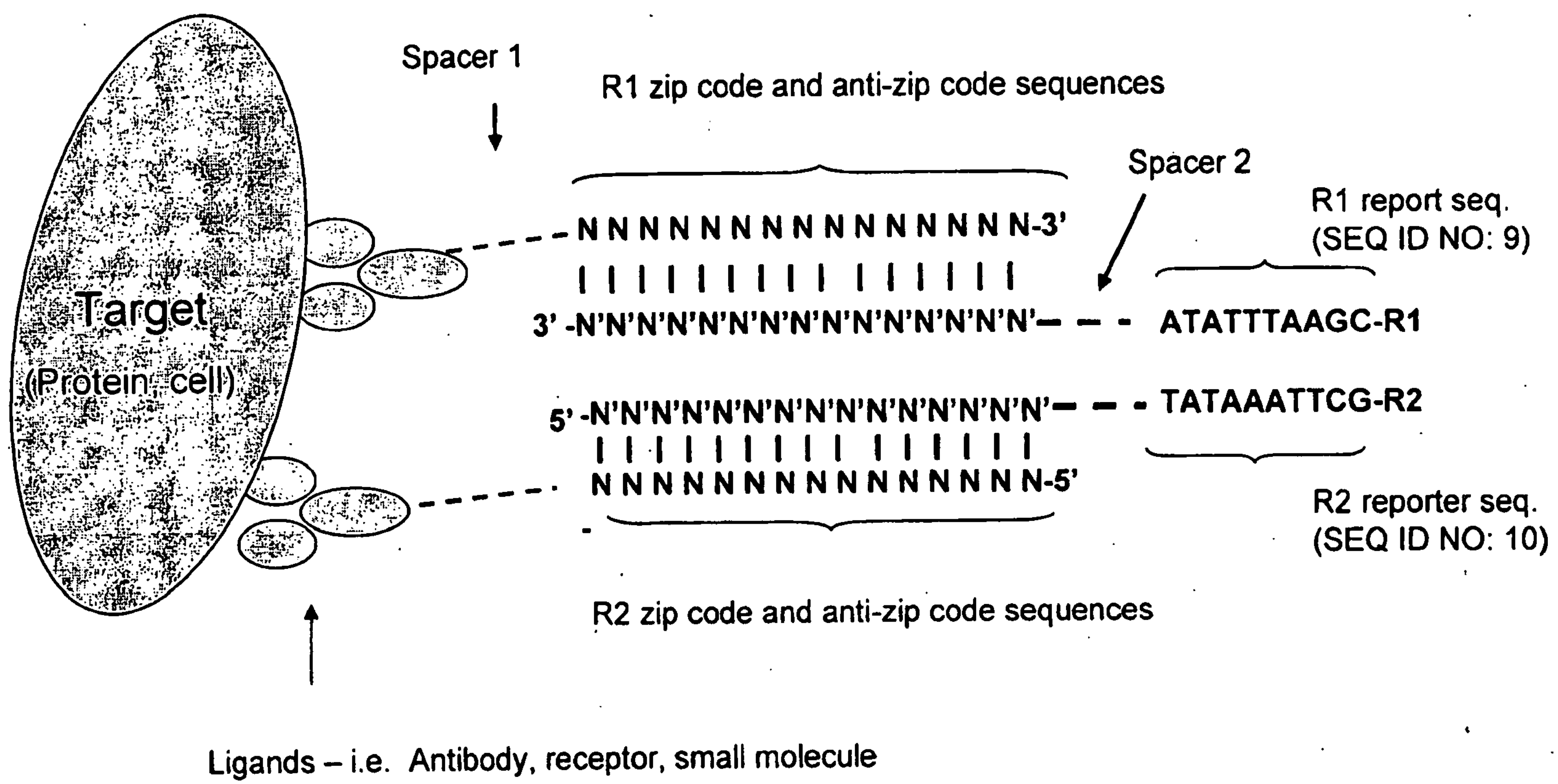
Spacer 1
R1 zip code and anti-zip code sequences
Spacer 2
R1 report seq.
(SEQ ID NO: 9)
Target
(Protein, cell)
N N N N N N N N N N N N N N N-3'
3' -N'N'N'N'N'N'N'N'N'N'N'N'N'N'
ATATTTAAGC-R1
5' -N'N'N'N'N'N'N'N'N'N'N'N'N'N'
TATAAATTCG-R2
N N N N N N N N N N N N N N N-5'
R2 reporter seq.
(SEQ ID NO: 10)
R2 zip code and anti-zip code sequences
Ligands – i.e. Antibody, receptor, small molecule

FIG. 14

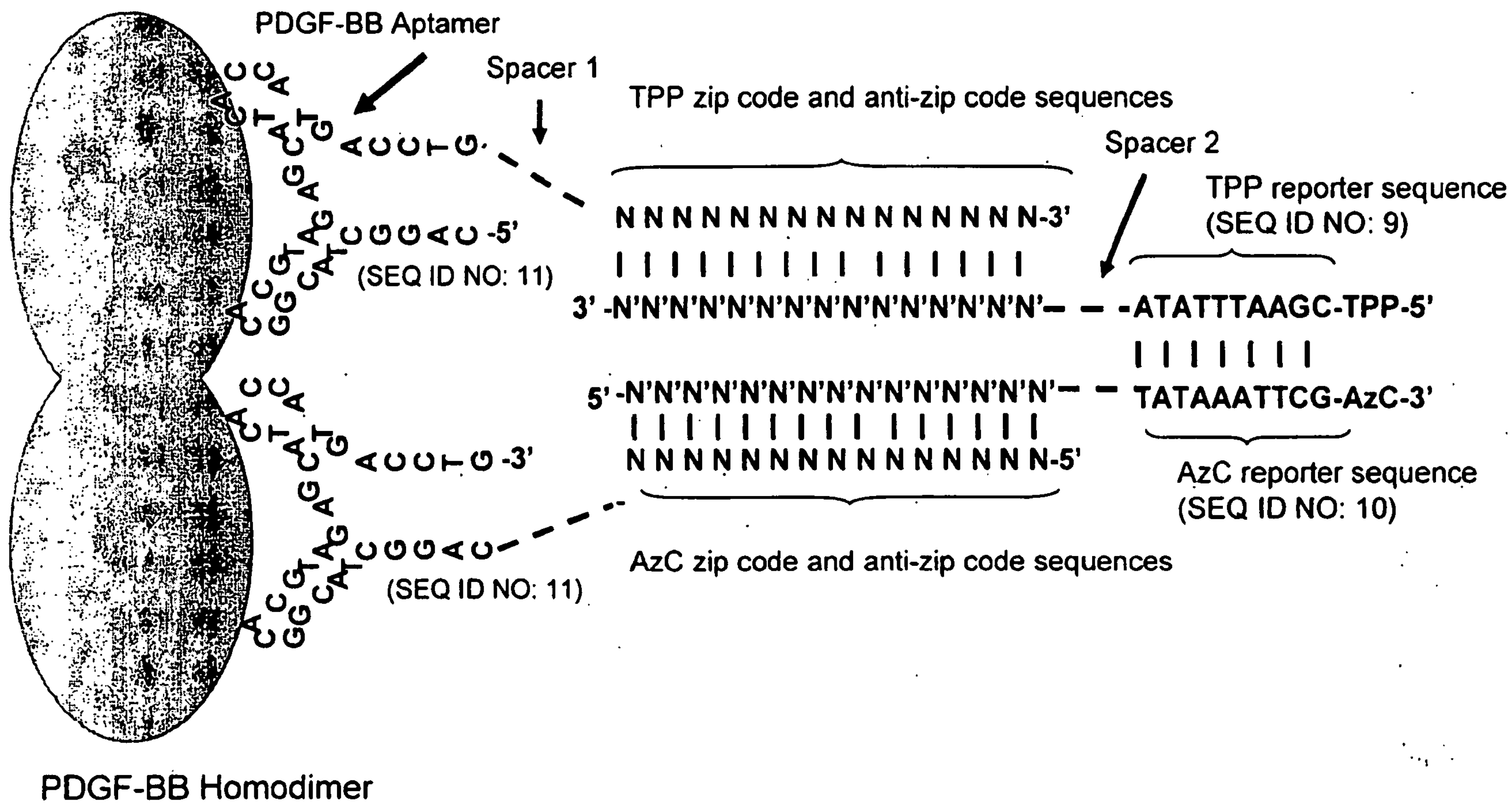


•Illustrative exemplary of a Zip-Coded Splinted Architecture

•NNN... represents zip code sequence, and N'N'N'... represent complement of the zip code sequence (anti-zip codes)

FIG. 15

```
                                                                                    (SEQ ID NO: 9)
(SEQ ID NO: 11)
                                                spacer   zip & anti-zip codes  spacer  DPC reporter
                     Aptamer
5'-CAGGCTACGGCACGTAGAGCATCACCATGACCTG-C18PEG-GCTGCCATCGATGGTCAG  #112
                                     #109      CGACGGTAGCTACCAGTC-18PEG-ATATTTAAGC-TPP-5'
                                     #106      GGACTCGAGCACCAATAC-C18PEG-TATAAATTCG-AzC-3'
3'-GTCCAGTACCACTACGAGATGCACGGCATCGGAC-C18PEG-CCTGAGCTCGTGGTTATG   #113
                                                (SEQ ID NO: 12)
                                                (SEQ ID NO: 13)                     (SEQ ID NO: 10)
                                                (SEQ ID NO: 14)
(SEQ ID NO: 11)                                 (SEQ ID NO: 15)
```

**Example of splinted, zip-coded detection probes using aptamer binders**

- The upper and lower two oligonucleotides include aptamer sequence linked to separate zip code sequences with a C18 PEG spacer.
- The inner two oligonucleotides (reporter oligonucleotides) include anti-zip codes (sequences each complementary to a zip code in one of the upper and lower oligoncleotides) linked through a C18 PEG spacer to a reporter oligonucleotide.
- One reporter oligonucleotide contains a 5'-terminal TPP residue, the other a 3' terminal AzC residue. Each zip code is complementary only to its anti-zip code.
- The reporter sequences are complementary only to each other.

FIG. 16

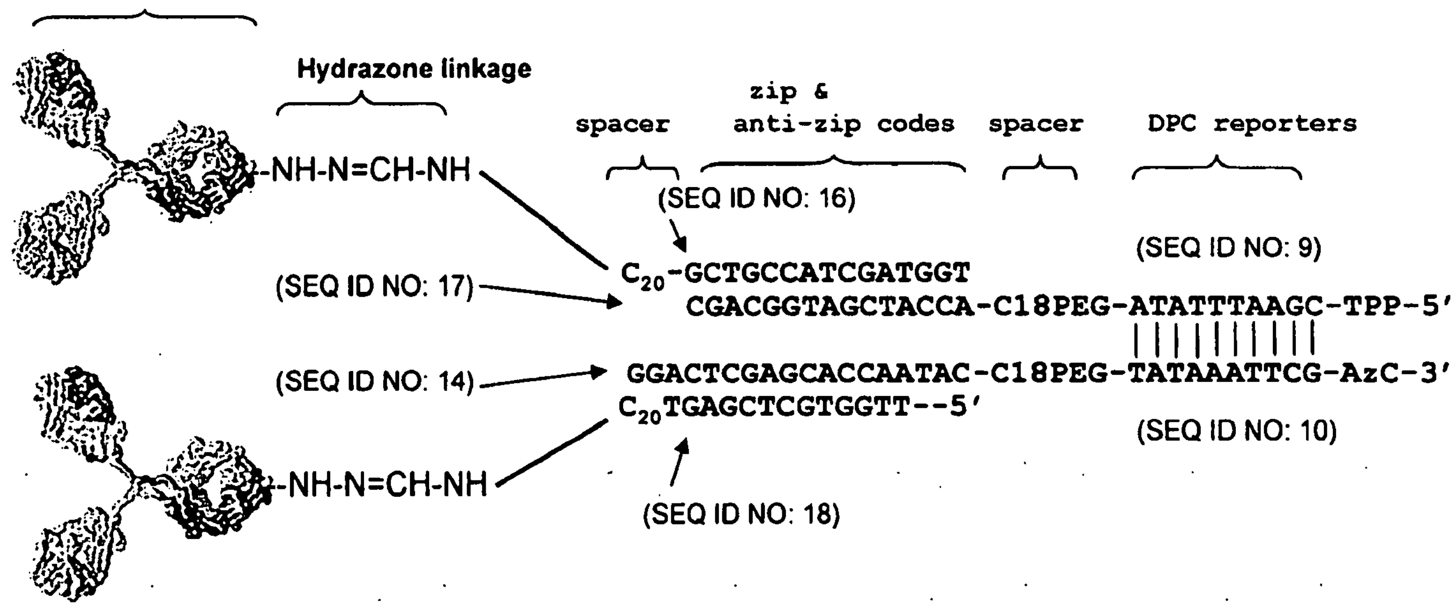


**Example of splinted, zip-coded detection probes using antibodies**

•Anti-PDGF-BB antibody covalently labeled via hydrazone linkages to a 15-base zip code sequence with a 20-base spacer of cytosine (C20).

•TPP reporter oligonucleotide with a complementary 15-base anti-zip code sequence linked via a C18 PEG spacer to a 10-base reporter sequence.

•AzC reporter oligonucleotide with an anti-zip code sequence and a 10-base reporter sequence complementary to the TPP reporter sequence. Anti-zip code sequence was 20 bases long, with 15 bases complementary to the zip code sequence.

FIG. 17

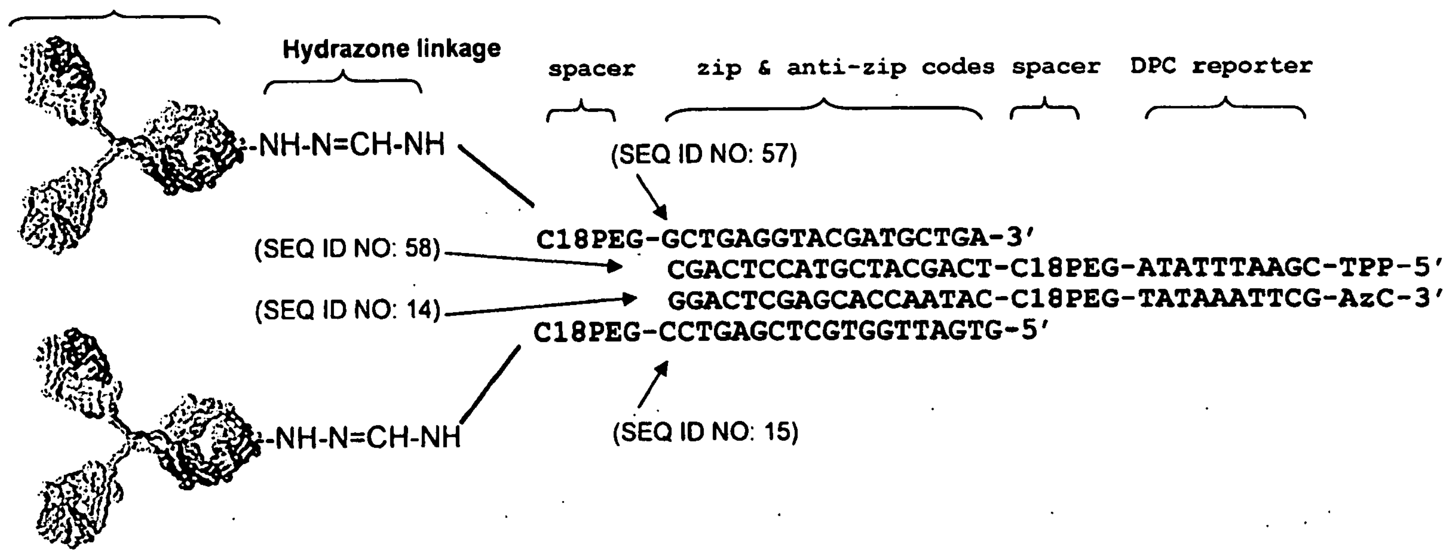


Exemplary assembly of Antibody-linked, zip-coded Detection Conjugates.

•Primary amino groups in the antibodies are activated to hydrazines and reacted with 3' and 5'-aldehyde-containing oligonucleotides to form hydrazone linkages.

•The upper and lower two oligonucleotides linked to the antibodies also contain separate zip code sequences separated from the antibody by a C18 spacer arm.

•The inner two oligonucleotides (reporter oligonucleotides) consist of anti-zip code sequences linked through a C18 PEG spacer to a reporter oligonucleotide.

•One reporter oligonucleotide contains a 5'-terminal TPP residue, the other a 3' terminal AzC residue. Each zip code is complementary only to its anti-zip code, and the DPC reporter sequences are complementary only to each other.

FIG. 18

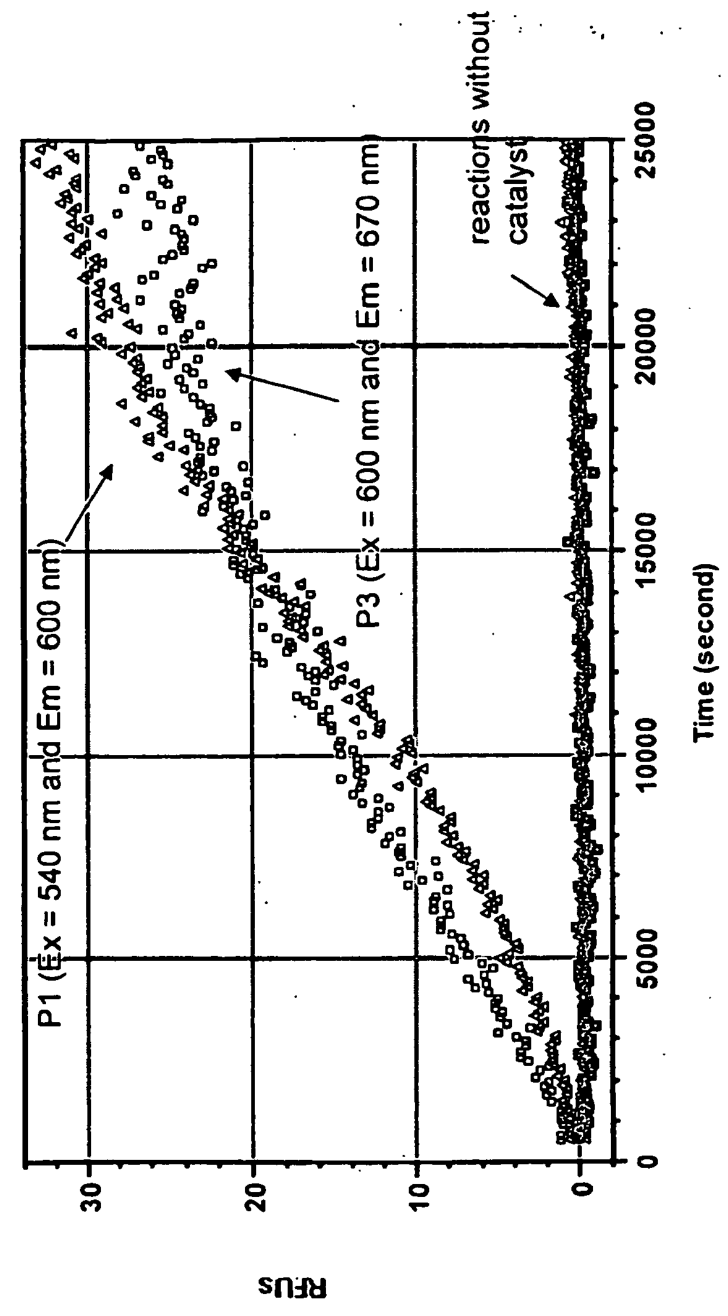


Fluorescence kinetic analysis of DPC reactions of Antizip3_indolinium/Antizip2 reporter1_A0 (triangle) and Antizip3_indolinium/ Antizip2 reporter1_A1 (circle).

FIG. 19

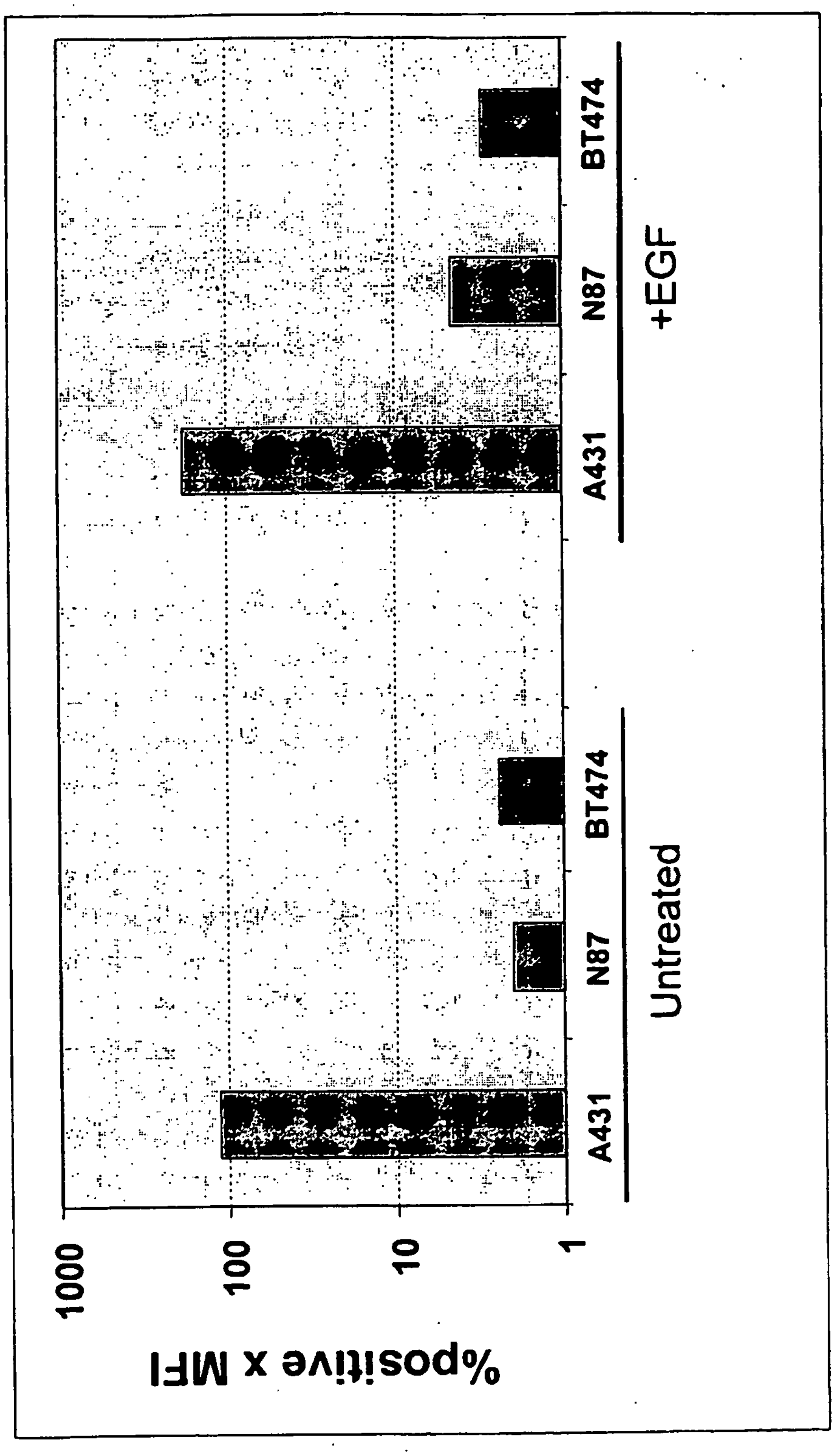
%positive x MFI
1000
100
10
1
A431
N87
BT474
A431
N87
BT474
Untreated
+EGF

FIG. 20

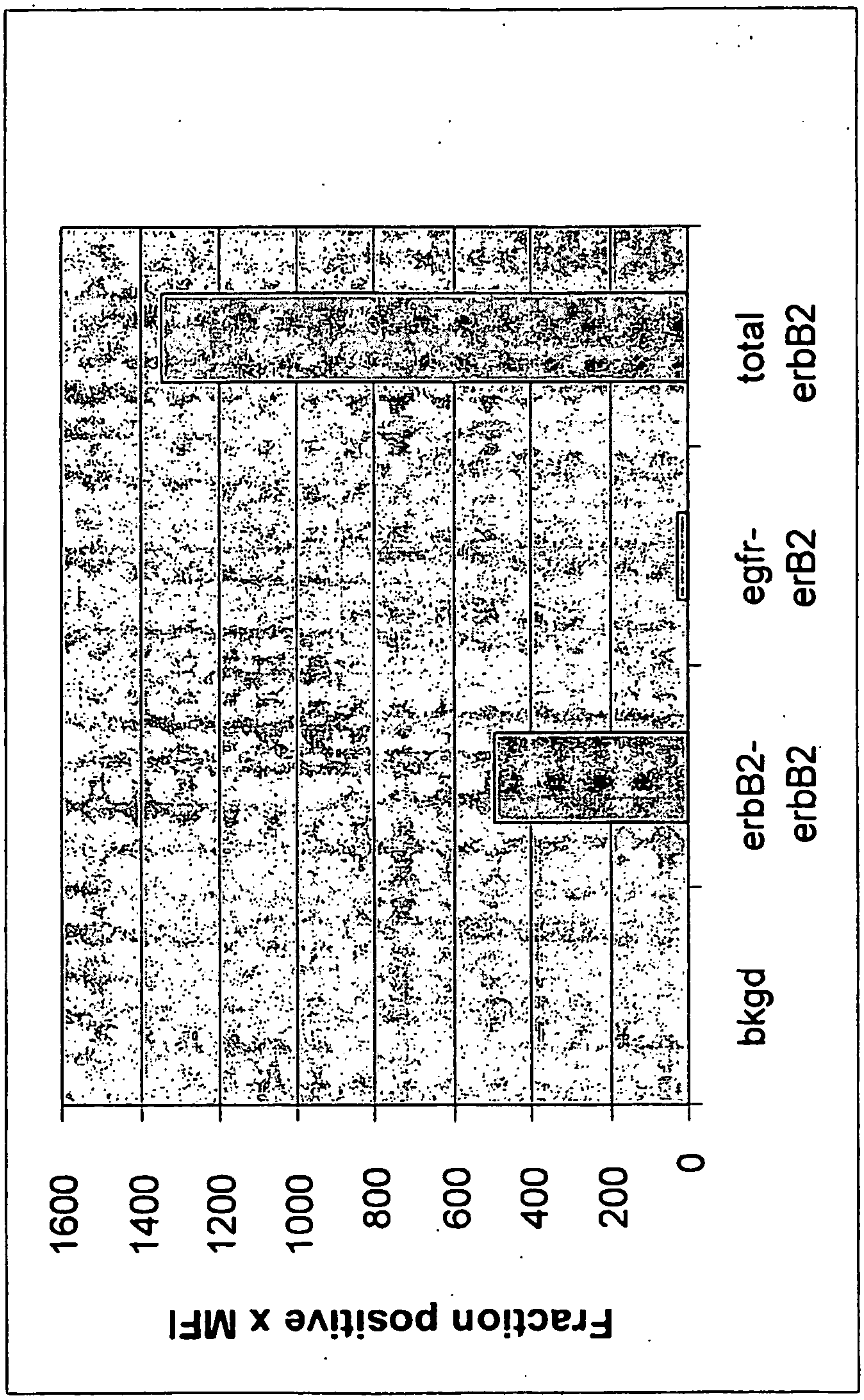
Fraction positive x MFI
1600
1400
1200
1000
800
600
400
200
0
bkgd
erbB2-erbB2
egfr-erbB2
total erbB2

FIG. 21

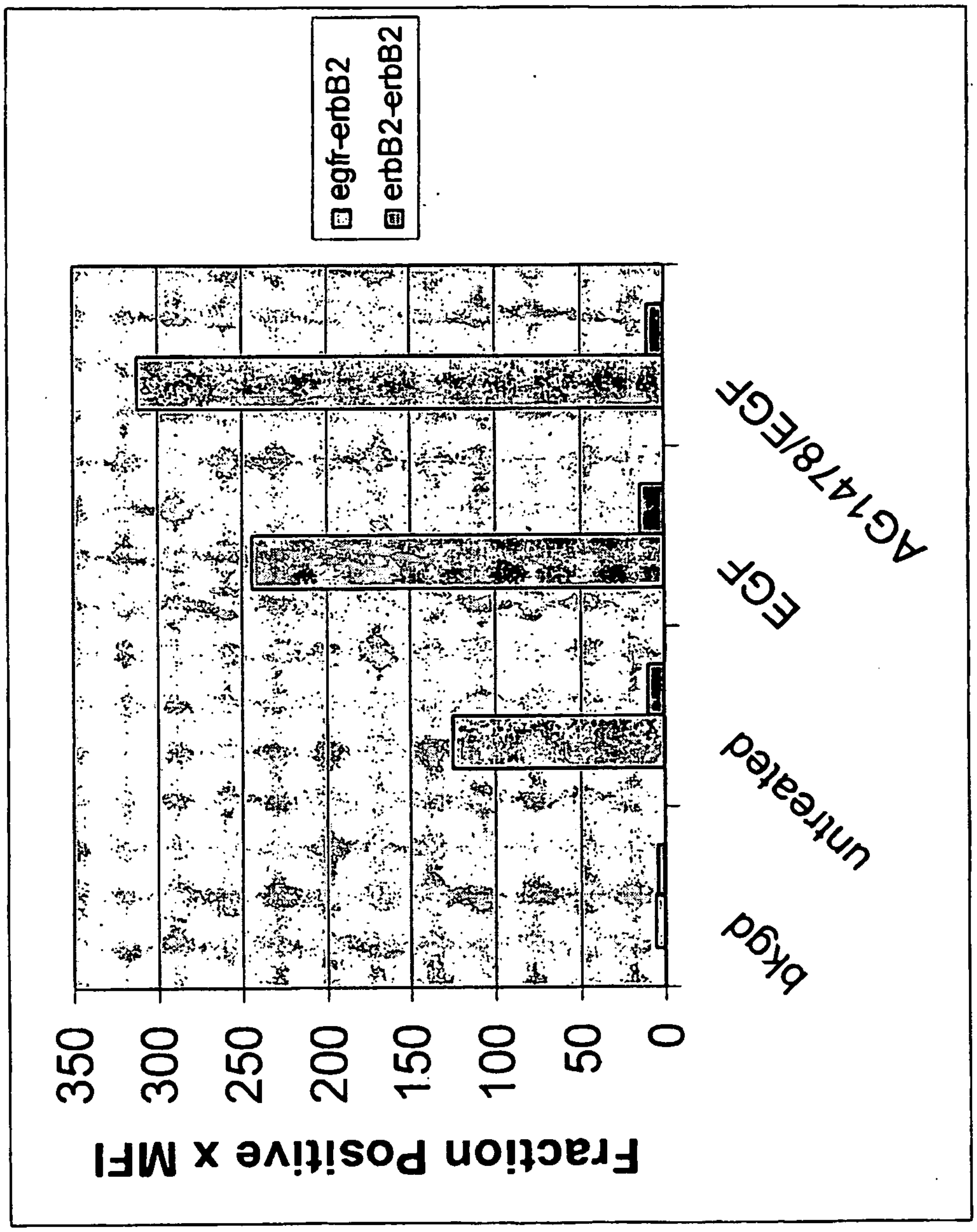
egfr-erbB2
erbB2-erbB2
Fraction Positive x MFI
350
300
250
200
150
100
50
0
bkgd
untreated
EGF
AG1478/EGF

FIG. 22

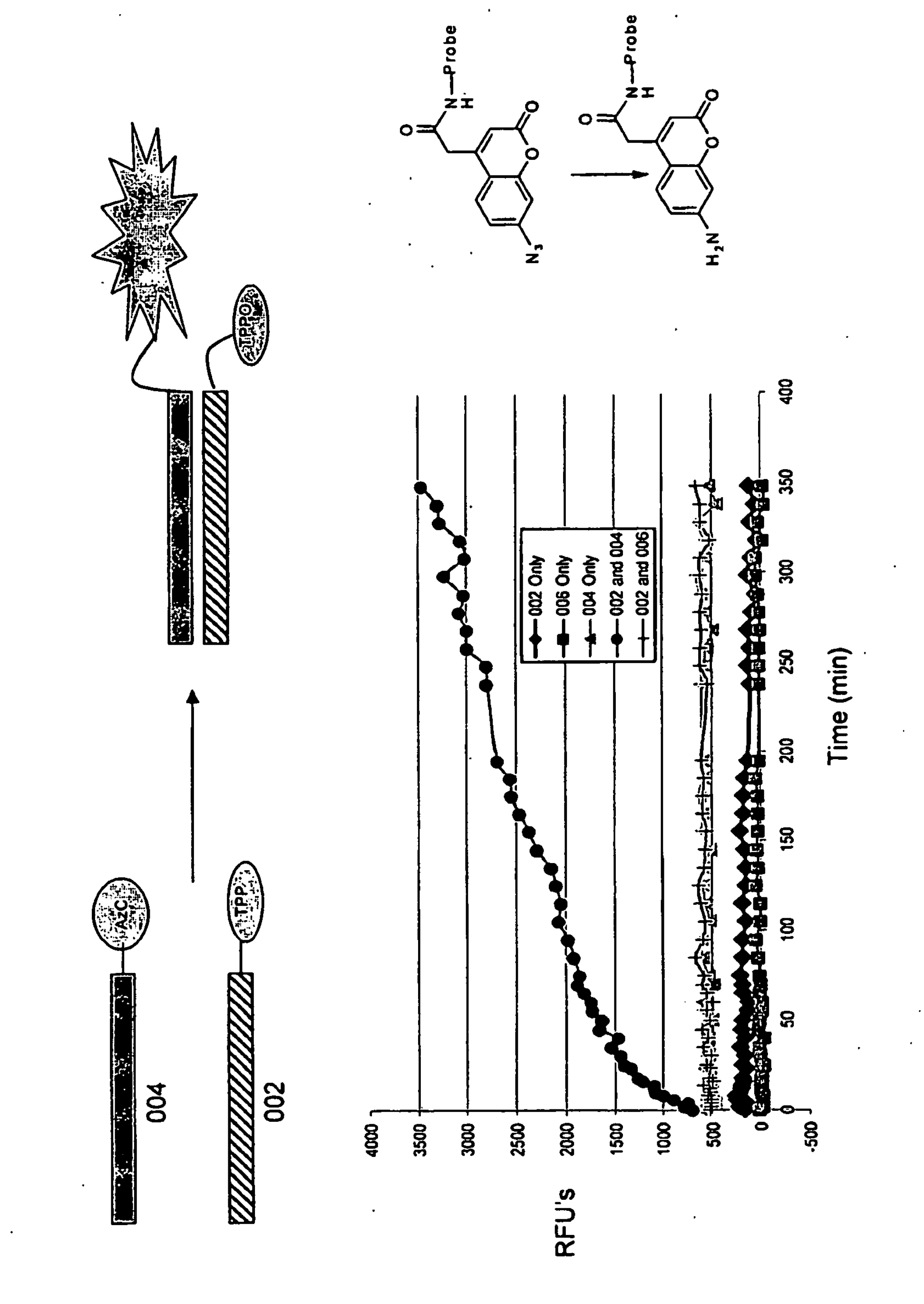


RFU = Relative Fluorescent Units

FIG. 23

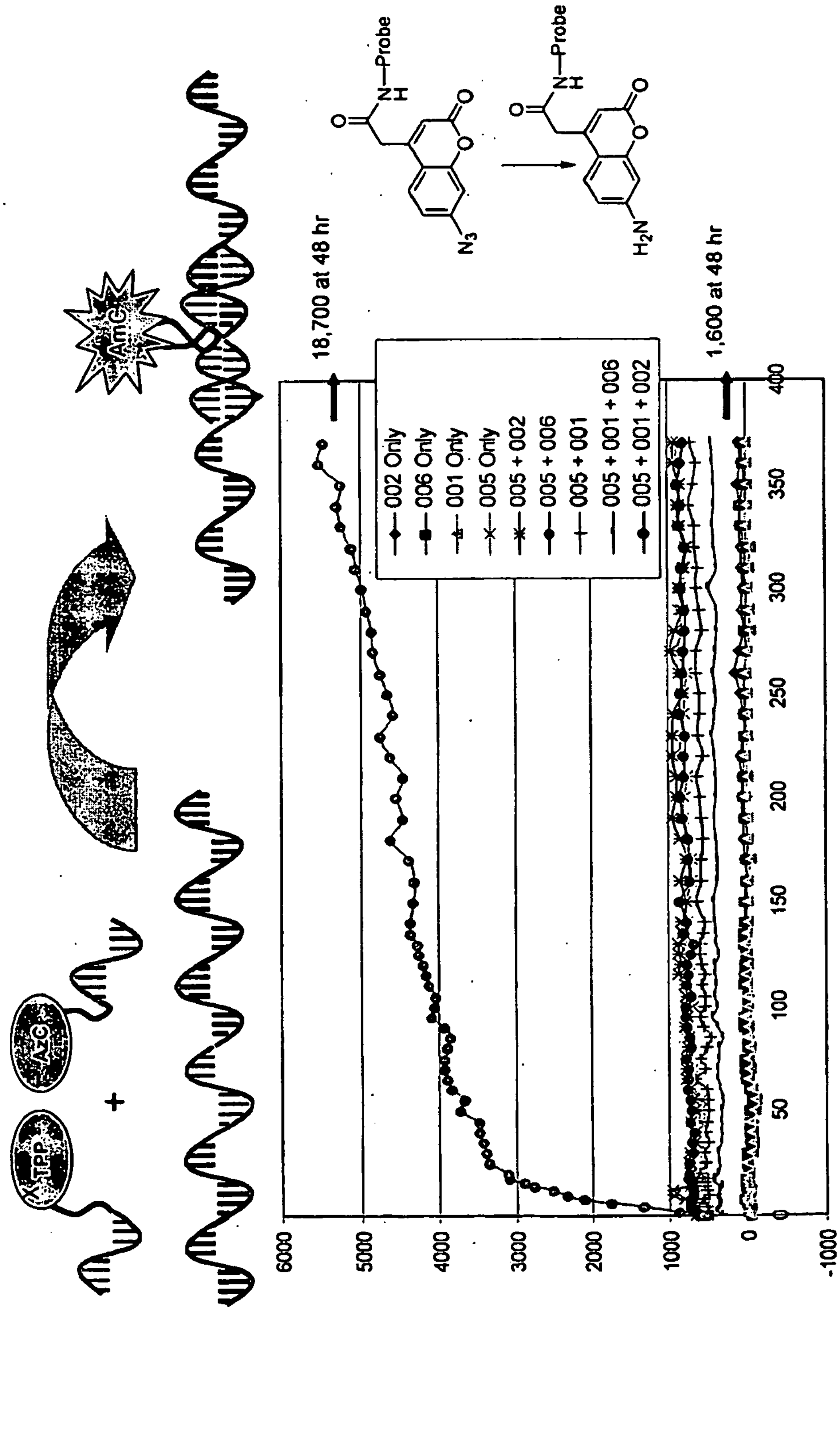
TPP
AzC
AmC
18,700 at 48 hr
1,600 at 48 hr
002 Only
006 Only
001 Only
005 Only
005 + 002
005 + 006
005 + 001
005 + 001 + 006
005 + 001 + 002
6000
5000
4000
3000
2000
1000
0
-1000
0
50
100
150
200
250
300
350
400

FIG. 24

Melt Curves, 10-base Complementary Oligos

1.0 M NaCl, 500 nM -20 nM Oligos

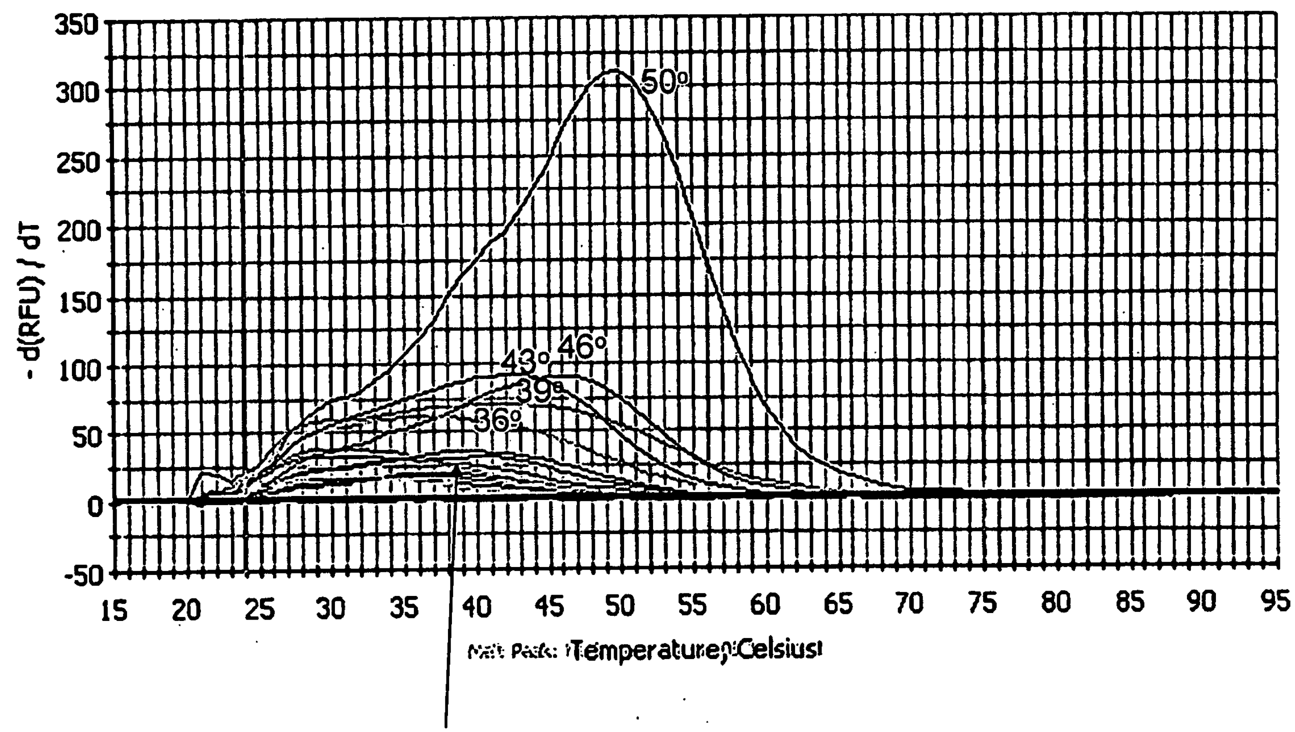


2-base mismatches – $T_m$'s 42° C to 31° C

Drop in $T_m$ ~ 10° C per 10-fold concentration of oligos

ENS-022PR EV839548687US

FIG. 25

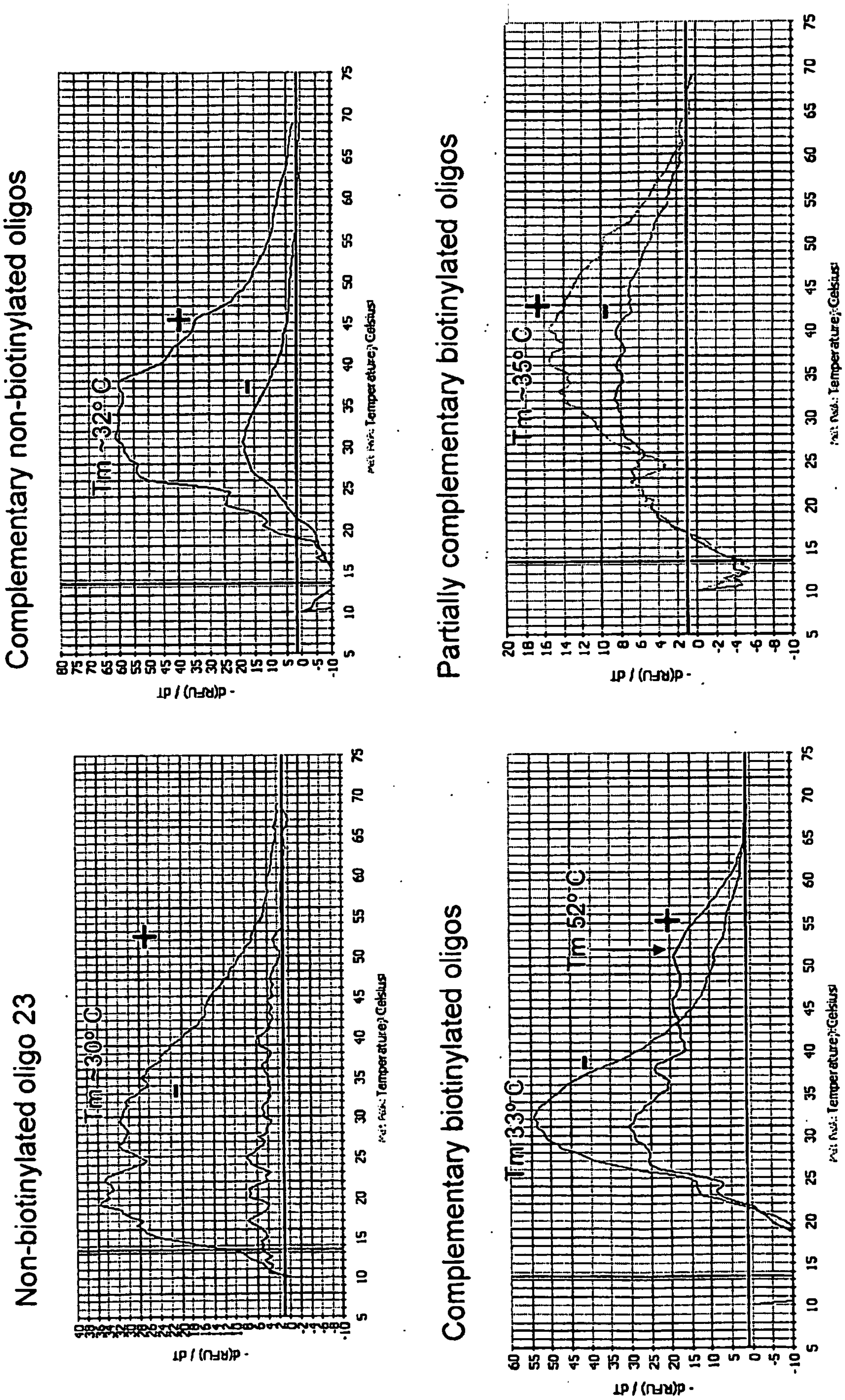


"Hot Start" DNA Hybrid Melting Curves +/- Avidin
100 nM oligos, 100 nM avidin, 25 mM salt

FIG. 26

**Complementary Biotinylated Oligos**

**$T_m$ Increases from ~35°C to ~54° C Upon Binding to Avidin.**

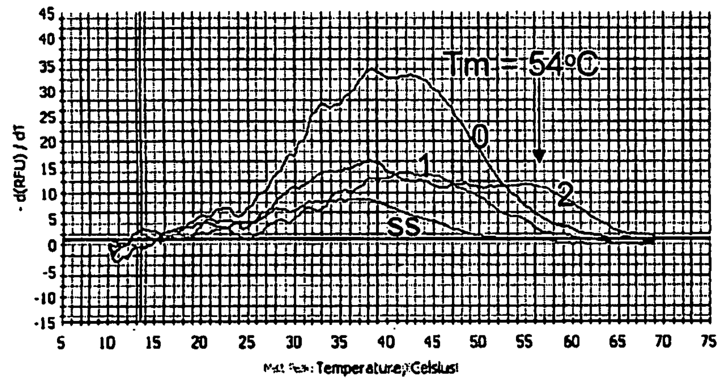


100 mM Salt, 50 nM avidin

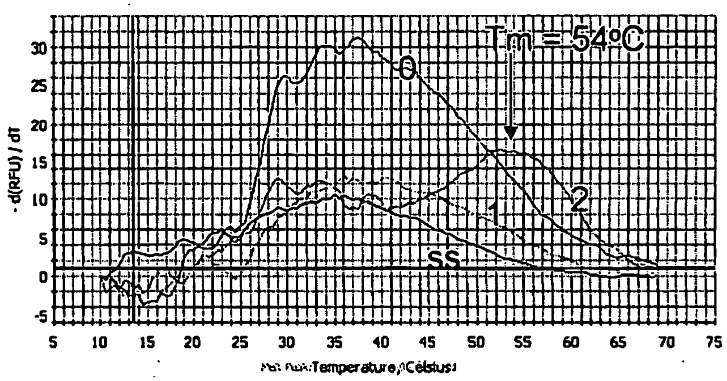


25 mM salt, 50 nM avidin

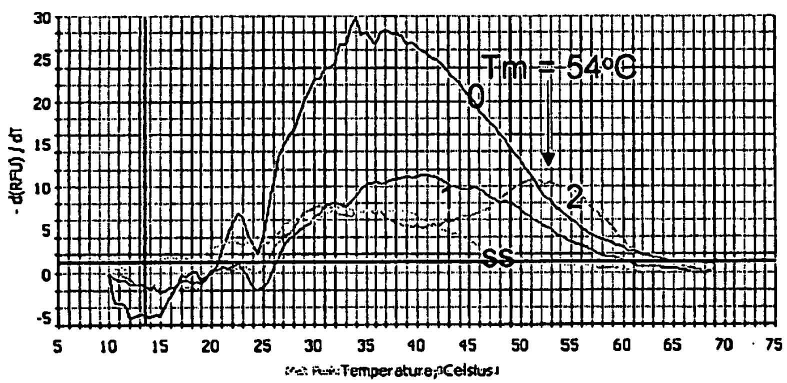


50 mM salt, 50 nM avidin

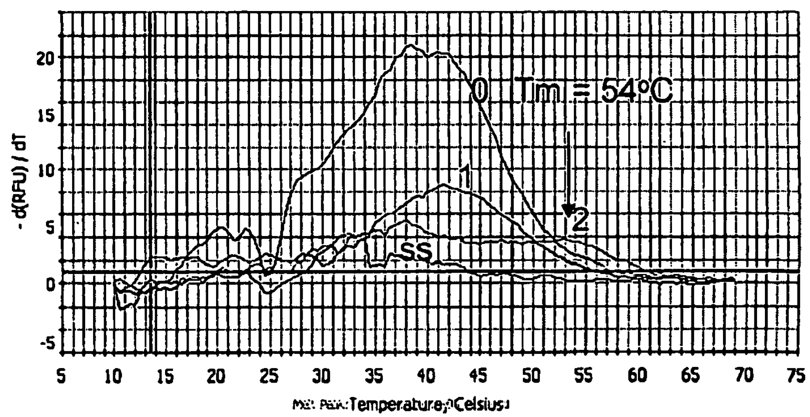


200 mM salt, 50 nM avidin

2= both strands biotinylated, 1= 1 strand of two biotinylated 0 = both strands not biotinylated ss= one biotinylated strand only

FIG. 27

Salt Concentrations – Effect upon $T_m$

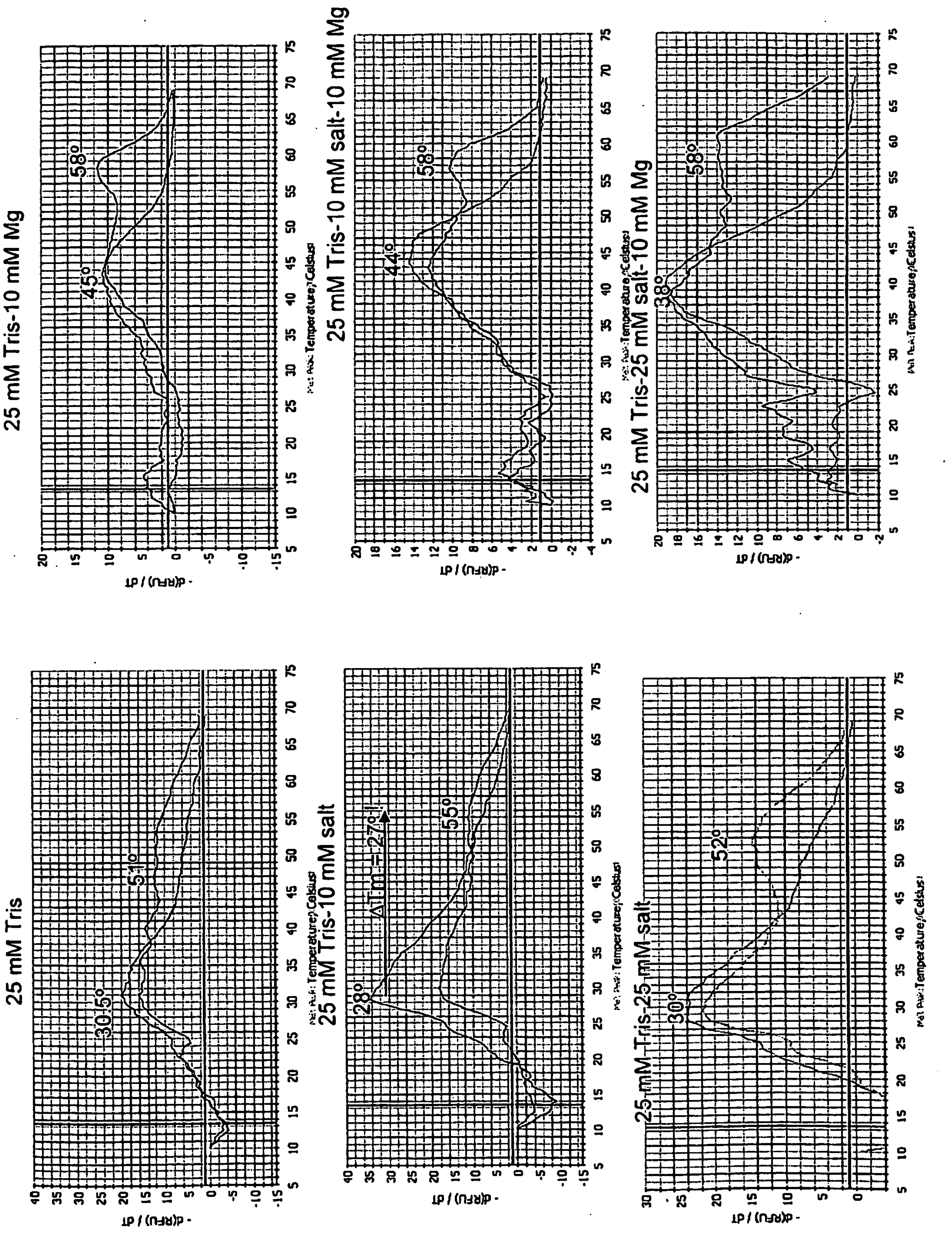

FIG. 28

Ratios of Biotinylated Oligos to Avidin

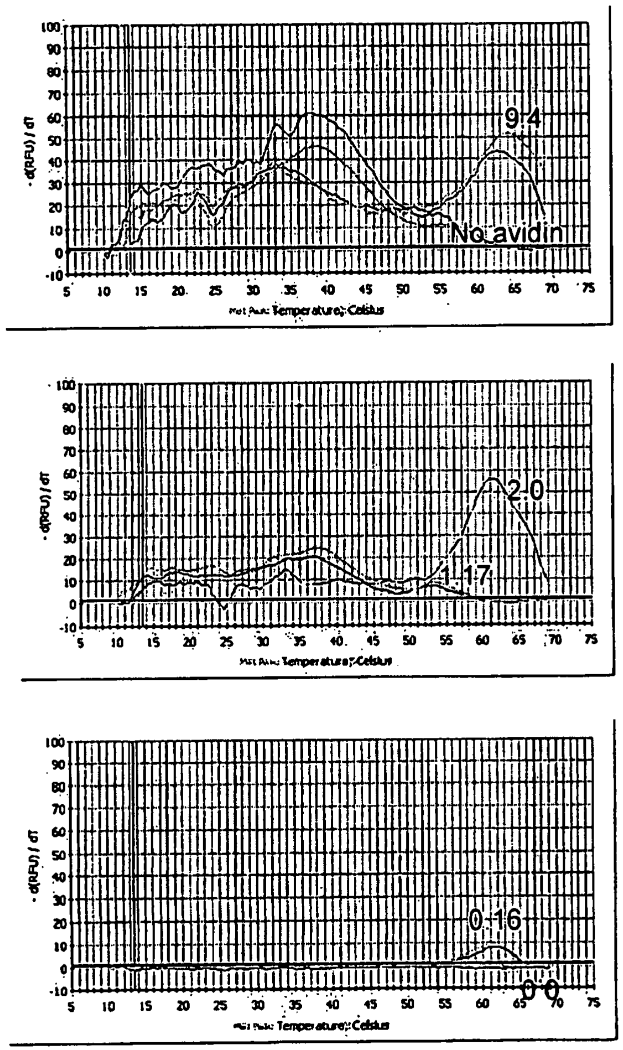


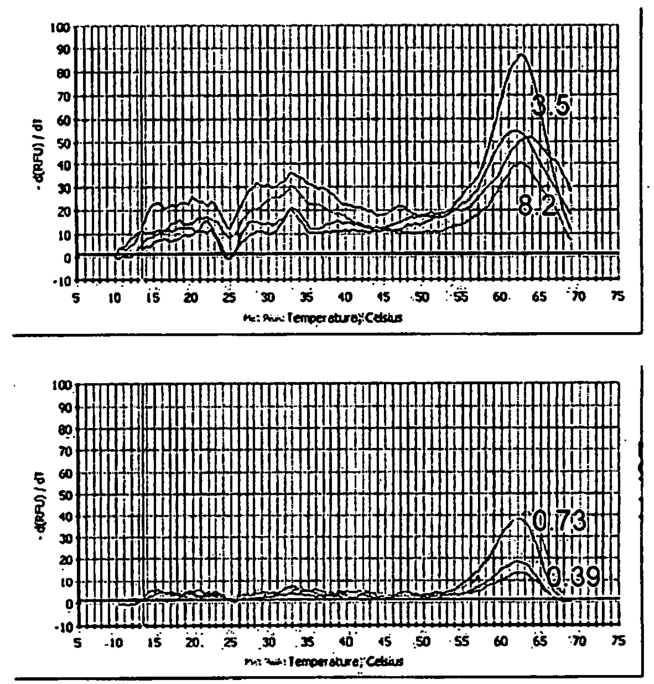


1st derivative of Melting Curves for

A-T rich biotinylated oligos

Molar ratio of oligos to Avidin

Oligos + avidin = constant of 7 µM

FIG. 29

Melting Curves of 5' and 3' (-) Biotin-Strand Oligos duplexed with Biotin-5' (+) Strand Oligo

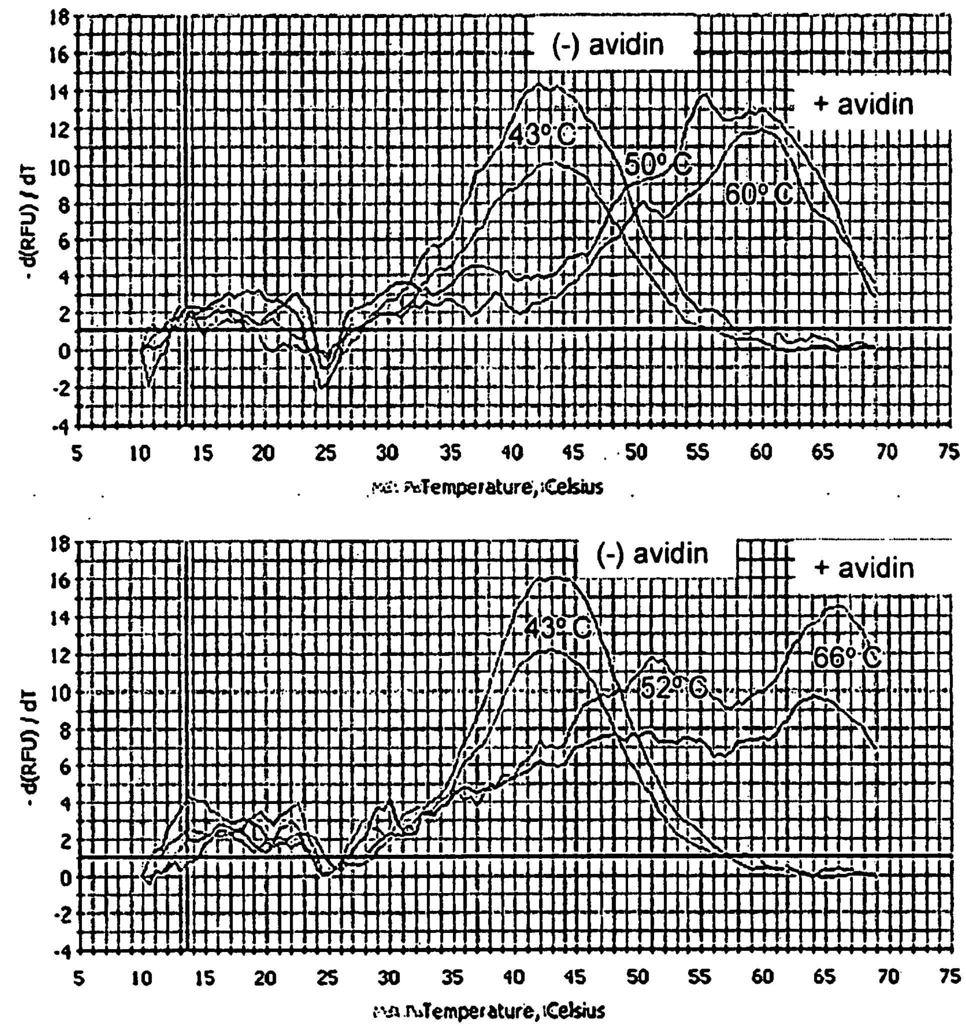


5' Biotin (-)

3' Biotin (-)

FIG. 30

**Melting Curves of AT-rich Biotinylated Oligo Dimers +/- Target**

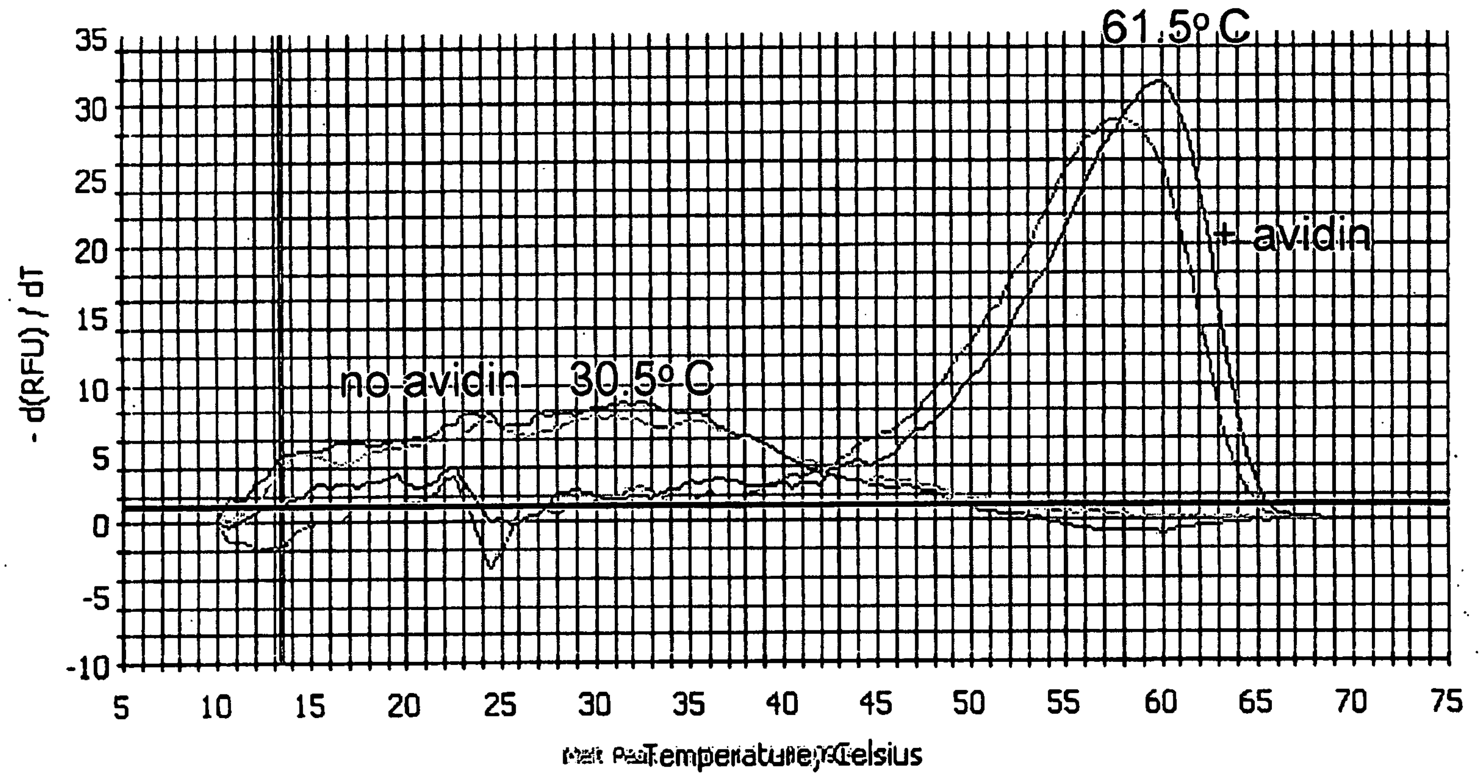


ΔTm = 31° C

(+) 5'-TTTTTTTTTTTTTAATTAAA-3' (SEQ ID NO: 27)

(-) 3'AAATTAATTTTTTTTTTTTT-5' (SEQ ID NO: 27)

FIG. 31

Detection of PDGF-BB with Aptamer-DPC Oligonucleotides

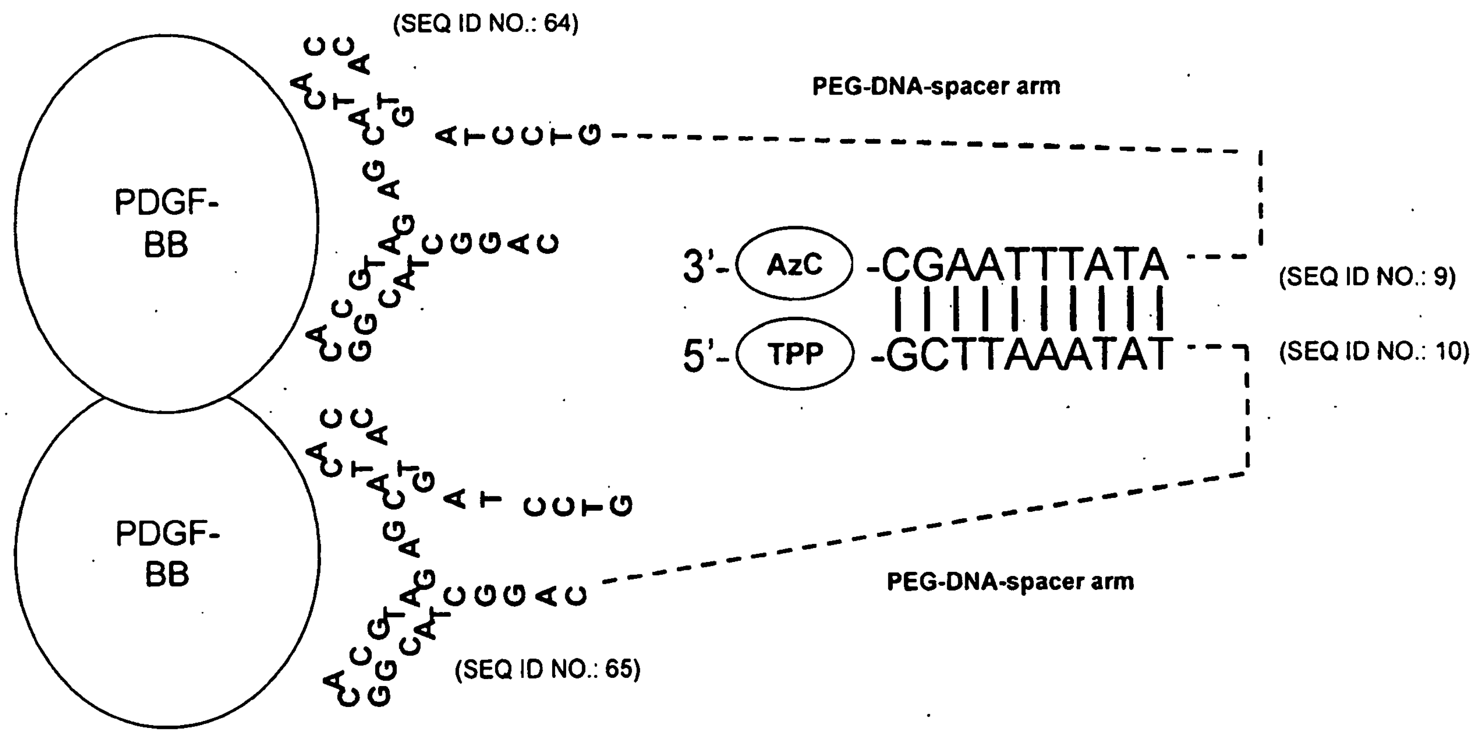


•Aptamers to B-type subunits of PDGF are conjugated to two different, complementary oligonucleotides through a single stranded DNA-PEG spacer arm.

•The azidocoumarin and triphenylphosphine precursors are in a helical structure only when the $T_m$ is raised by the aptamers binding to PDGF.

FIG. 32

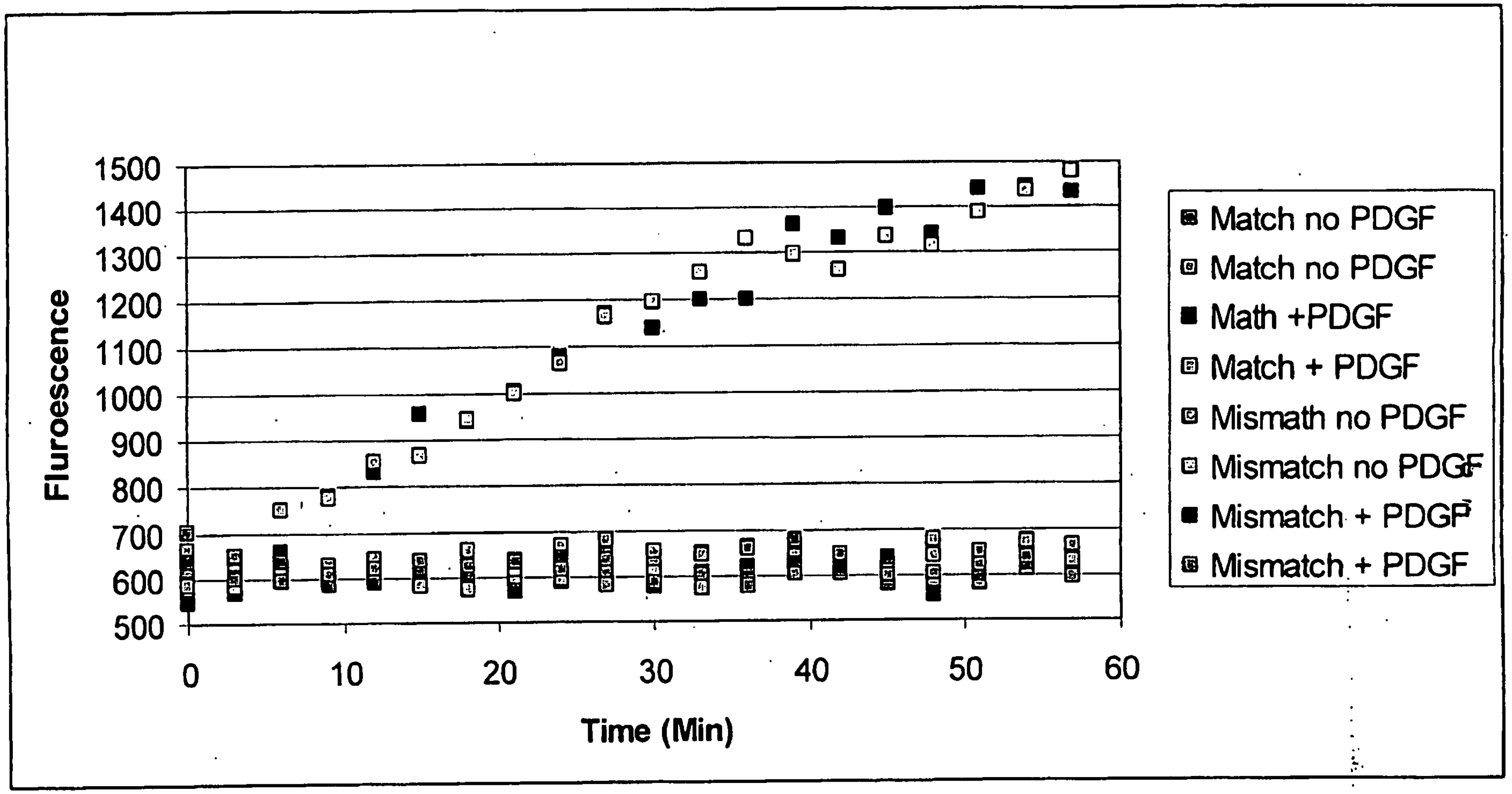


Reaction of the aptamer probes to PDGF does not occur

1) in the absence of PDGF

2) if the TPP probe is mismatched in DNA sequence to the Coumarin probe.

FIG. 33

Rates in Formamide

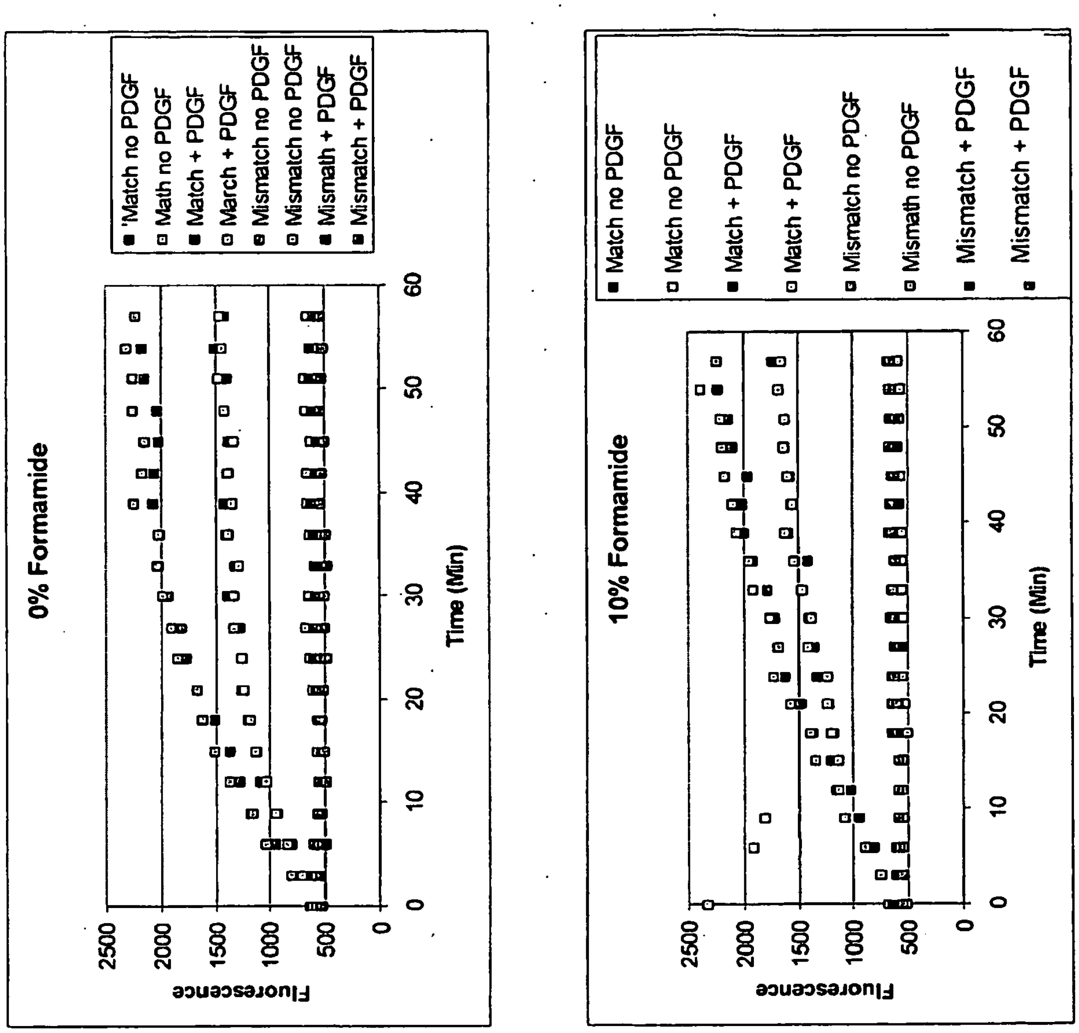


a)

b)

FIG. 34

Rates in Formamide

a)

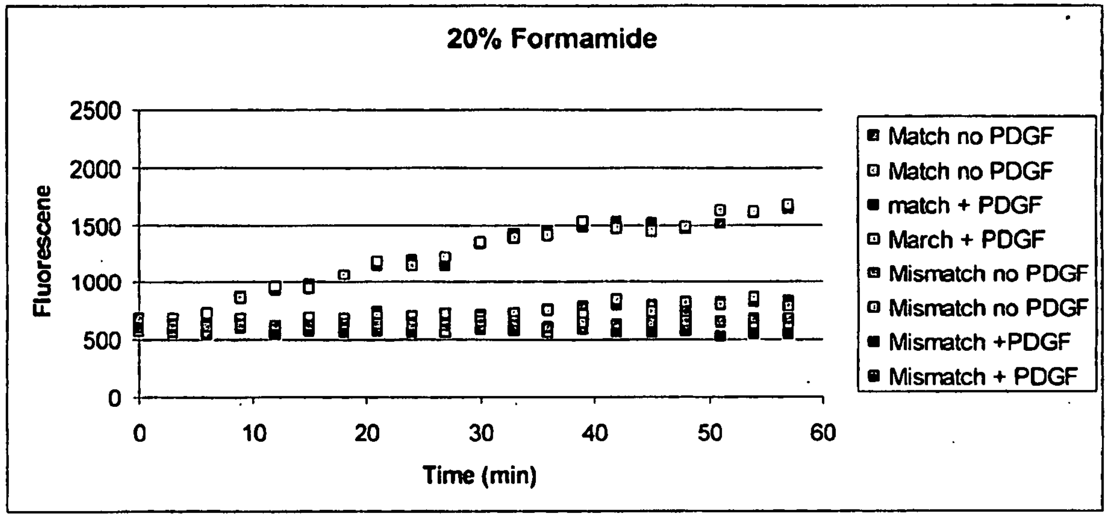


b)

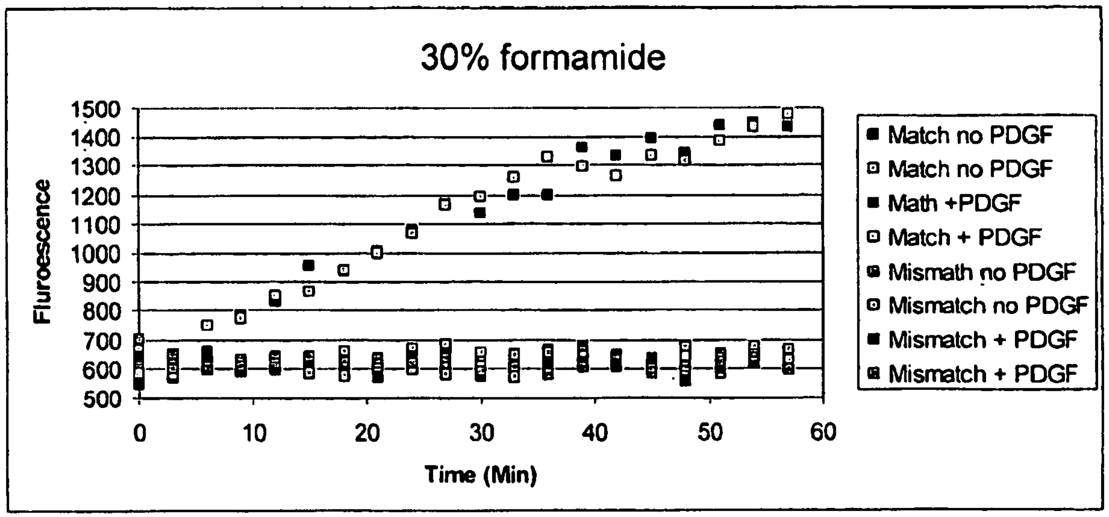

FIG. 35

Rates in Formamide

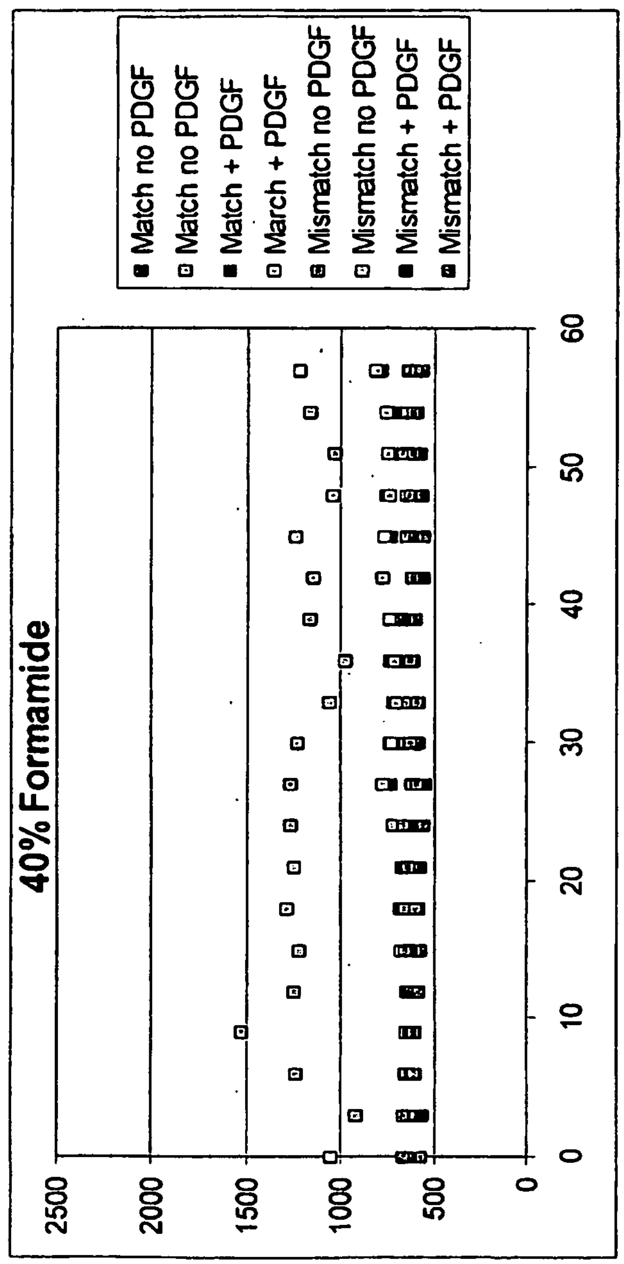

FIG. 36

Homogeneous Assay Detection of PDGF-BB with Aptamer-based Probes

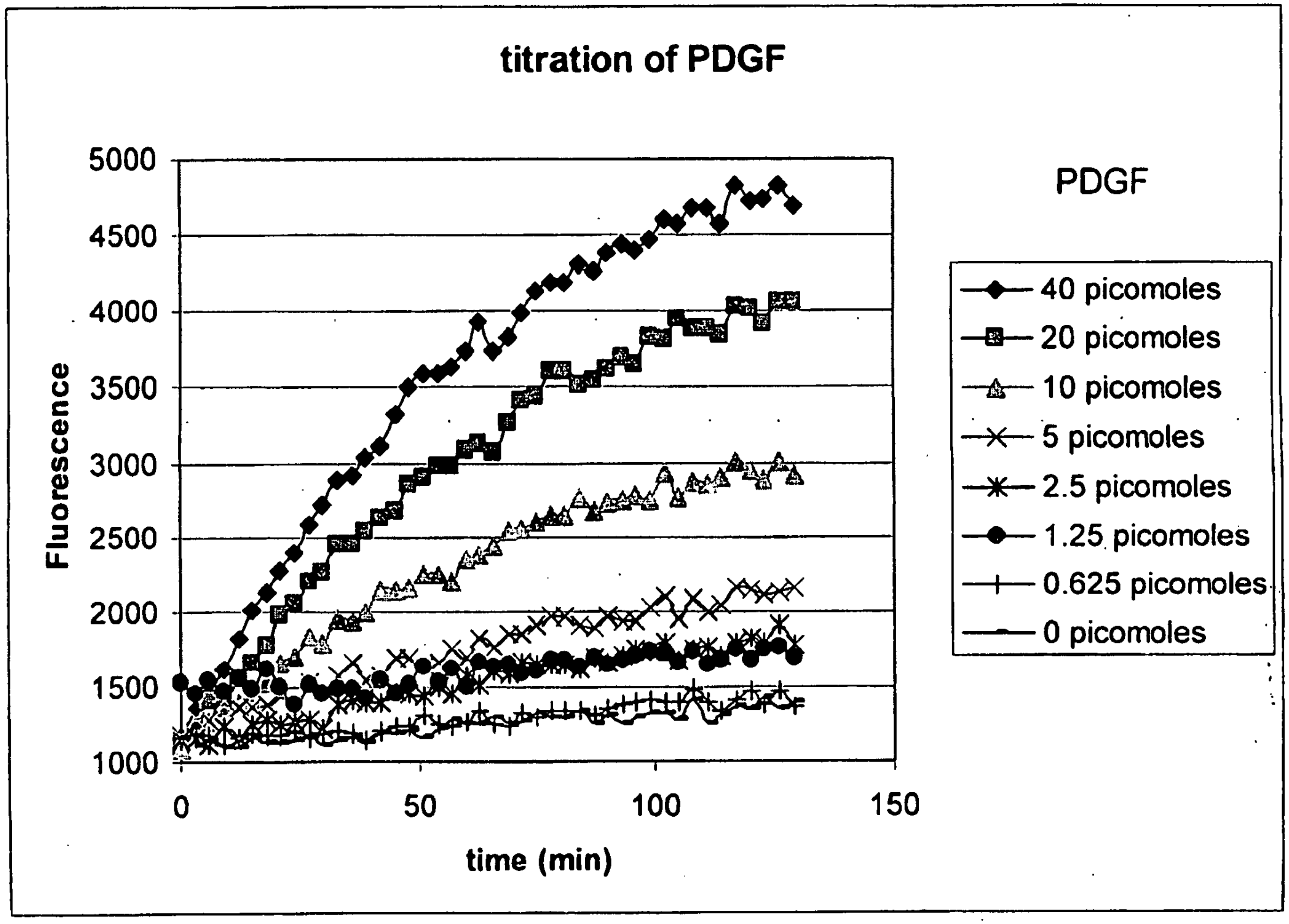

FIG. 37

Homogeneous Assay Detection of PDGF-AA with Aptamer-DPC Probes

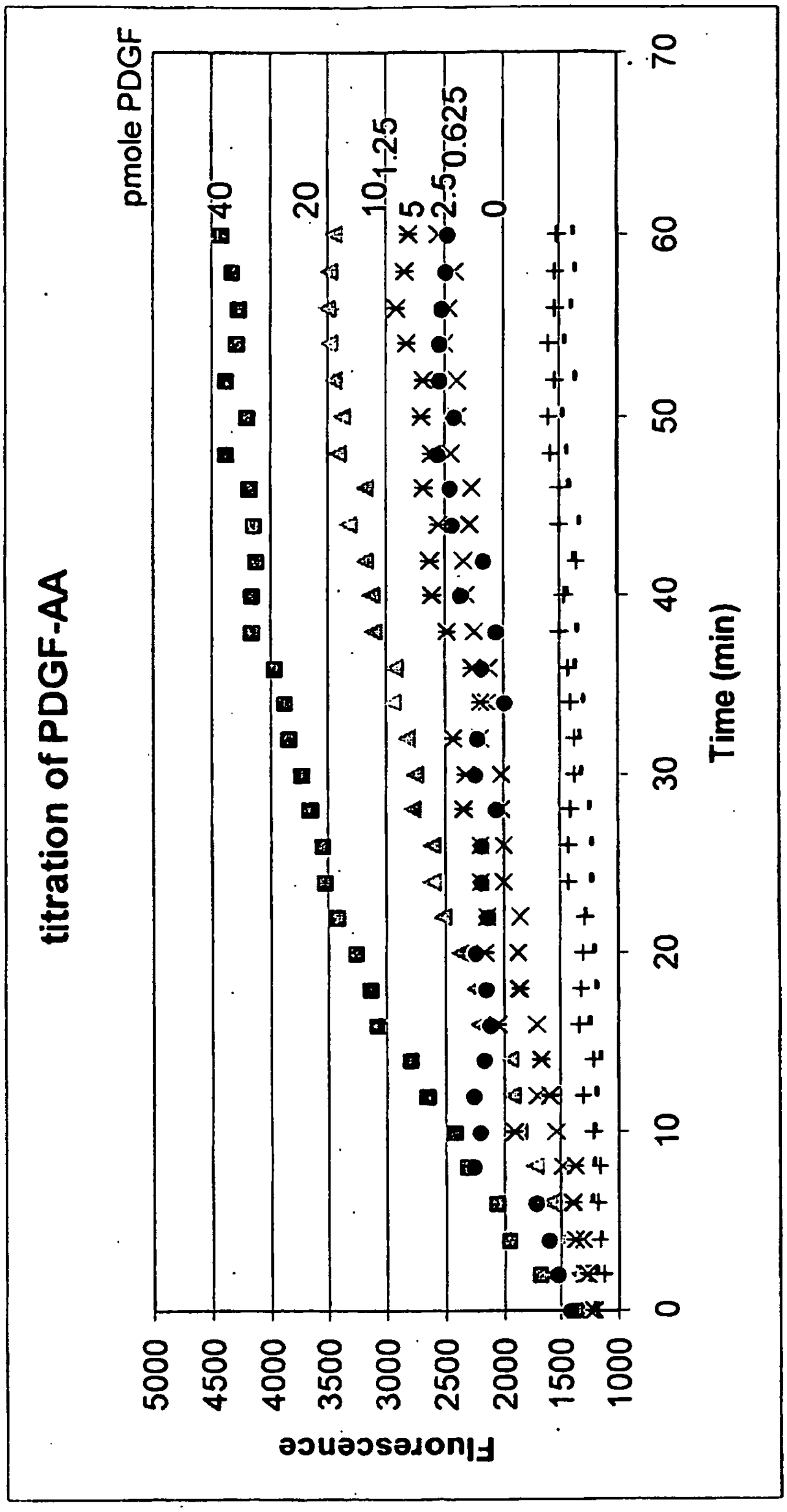

FIG. 38

Ratio of TPP:AZC Probes

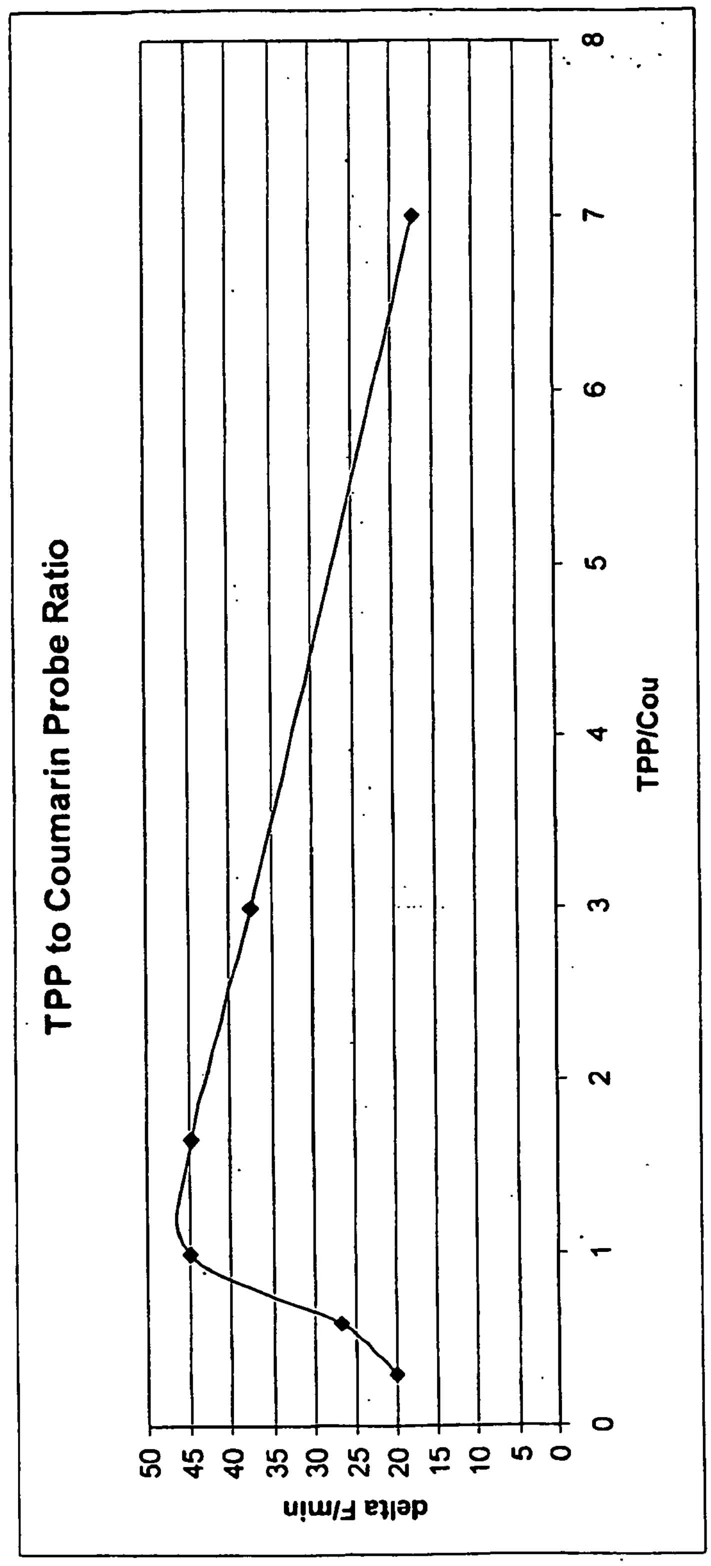

FIG. 39

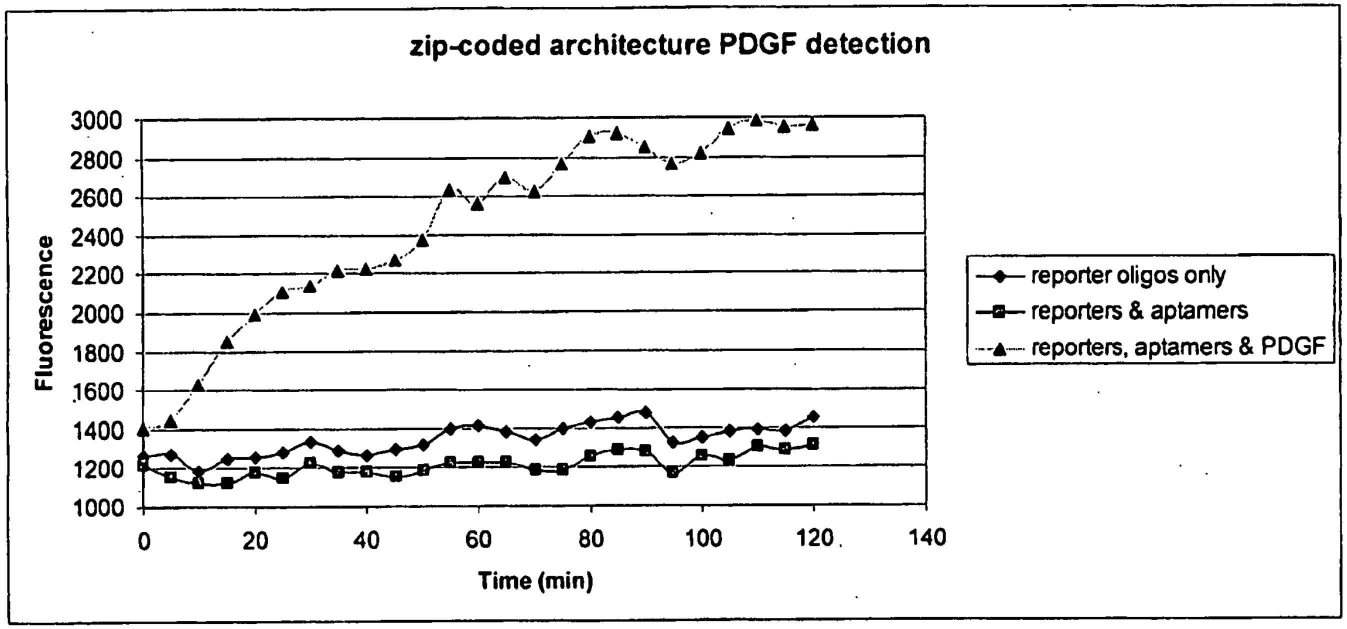


•Dependence of the splinted architecture upon the presence of both aptamer and reporter oligonucleotides.

•All reactants were tested at 0 or 0.4 µM concentration, at 22°C, in 50 mM Tris/HCl pH 8.5 – 10 mM $MgCl_2$ – 35% v/v formamide.

•Fluorescence was measured in a Wallac Victor Luminometer with excitation at 350 nm and emission at 460 nm.

FIG. 40

A)

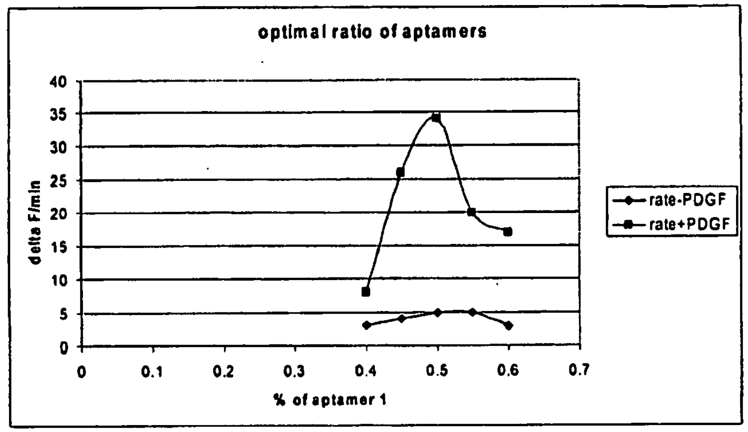


B)

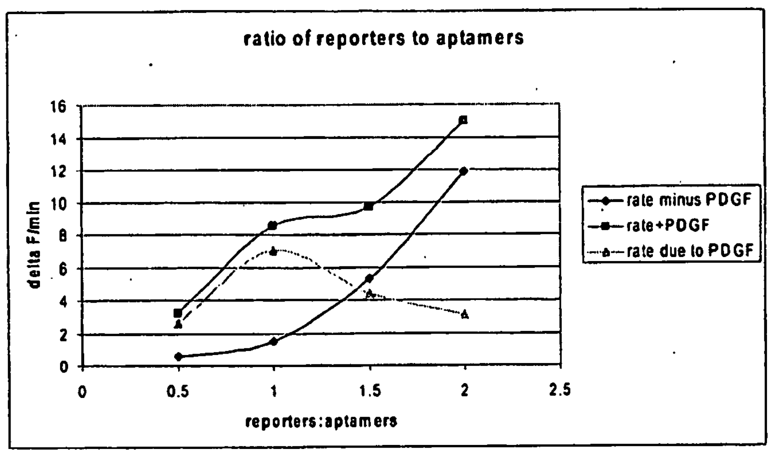


Reaction rates at various ratios of TPP to AzC aptamers oligonuceotides (A) or reporters to aptamers (B) .

FIG. 41

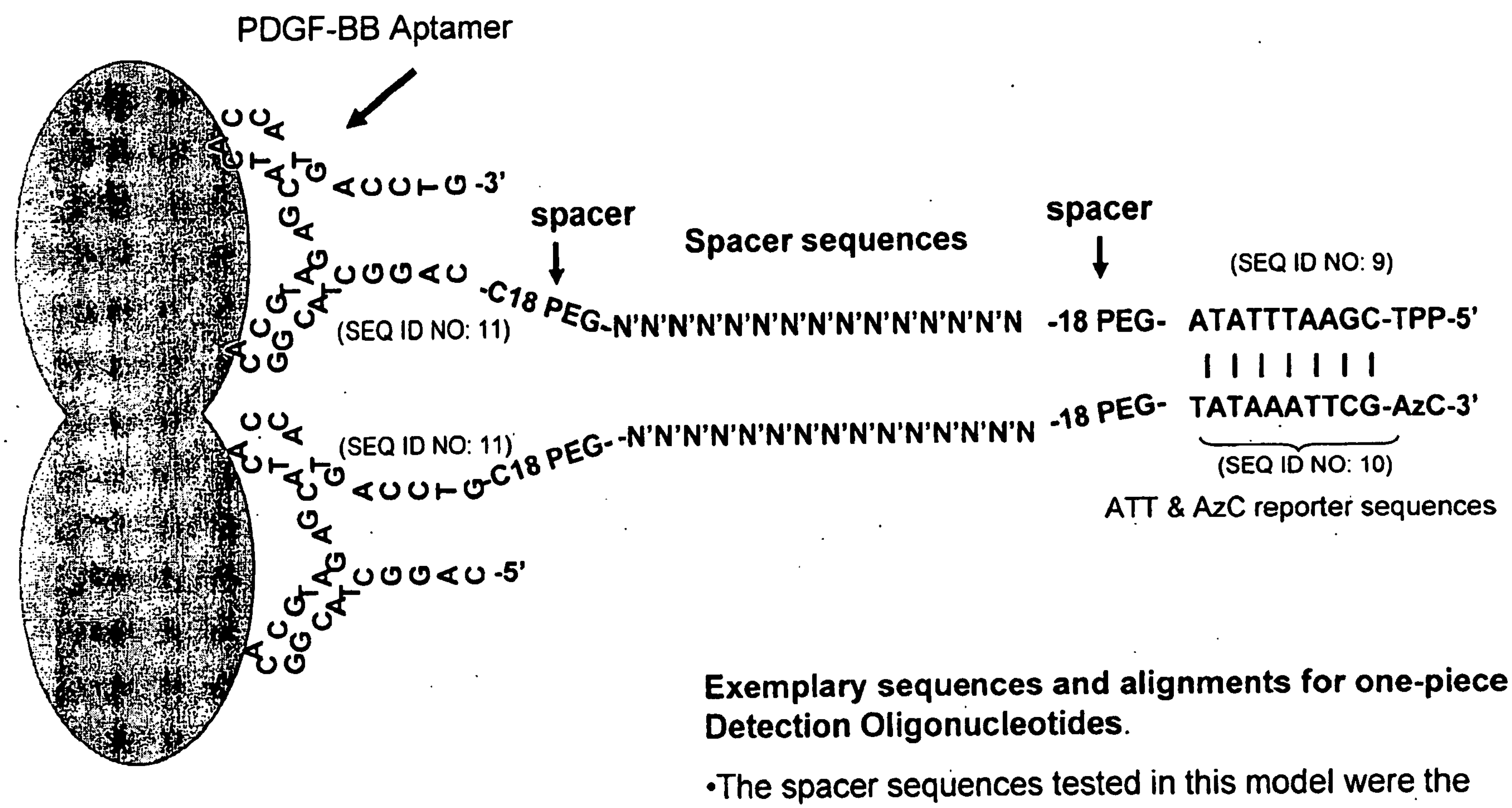


PDGF-BB Homodimer

**Exemplary sequences and alignments for one-piece Detection Oligonucleotides.**

•The spacer sequences tested in this model were the same as the zip code sequences in the aptamer oligonucleotides in FIG. 10.

•The upper sequence is oligo #104, the lower oligo #108. These two sequences are not complementary.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005074417 A
- WO 2003076943 A
- US 7070928 B
- US 7223545 B
- WO 06128138 A2, Coull
- US 7070928 B1
- US 7223545 B1
- EP 1423400 B1, Liu
- US 20040180412 A
- US SN10643752 A, Liu
- WO 07008276A2 A, Coull
- US 60845330 B
- US 60847859 B
- US 60905364 B
- US 60918023 B

### Non-patent literature cited in the description

- **Laux et al.** *British Journal of Cancer,* 2006, vol. 94, 85-92
- **Gartner et al.** *Science,* 2004, vol. 305, 1601-1605
- **Li et al.** *Angew. Chem. Int. Ed,* 2004, vol. 43, 4848-4870
- **Doyon et al.** *JACS,* 2003, vol. 125, 12372-12373
- **Citri et al.** *Nat Rev Mol Cell. Biol,* 2006, vol. 7 (7), 505-16
- **Muthuswamy et al.** *Mol Cell Biol,* 1999, vol. 19 (10), 6845-57
- **Hynes et al.** *Nat Rev Cancer,* 2005, vol. 5 (5), 341-54
- **Baselga.** *J. Science,* 2006, vol. 312 (5777), 1175-8
- **Vogel et al.** *J Clin Oncol,* 2002, vol. 20 (3), 719-26
- **Citri et al.** *Nat Rev Mol Cell Biol,* 2006, vol. 7 (7), 505-16
- **Smith et al.** *Br J Cancer,* 2004, vol. 91 (6), 1190-4
- **Wehrman et al.** *Proc Natl Acad Sci U S A,* 2006, vol. 103 (50), 19063-8
- **Anido et al.** *Embo J,* 2006, vol. 25 (13), 3234-44
- **Zollinger.** Color Chemistry: Syntheses, Properties, and Applications of Organic Dyes and Pigments. Verlag Helvetica Chimica Acta, 2003
- **Wetmur.** *Criti. Rev. in Biochem. And Mol. Biol.,* 1991, vol. 26, 227-259
- **Novikova et al.** *Russian Journal of Organic Chemistry,* 1998, vol. 34, 1762-1767
- **Brady et al.** *J. Bio. Chem.,* 2001, vol. 276, 18812-18818
- **Eitel et al.** *Synthesis,* 1989, 364-367
- **Bratenko et al.** *Chemistry of Heterocyclic Compounds,* 2004, vol. 40, 1279-1282
- **Brady et al.** *J. Biol. Chem.,* 2001, vol. 276, 18812-18818
- **Hermanson, G. T.** Bioconjugate Techniques. Academic Press, 1996
- **Citri et al.** *Nature Rev. Mol. Biol.,* 2006, vol. 7, 505-516
- **Arteaga et al.** *J. Biol. Chem.,* 1997, vol. 272, 23247-23254
- **Gannett et al.** *J. Biol. Chem.,* 1997, vol. 272, 12052-12056
- **Sakurai et al.** *J. Amer. Chem. Soc.,* 2005, vol. 127, 1660-1667
- **Thevenin et al.** *Eur. J. Biochem,* 1992, vol. 206, 471-477
- **Lipsky et al.** *Clinical Chemistry,* 2001, vol. 47 (4), 635-44
- **Fang et al.** *Chem. BioChem.,* 2003, vol. 4, 829-34
- **Green et al.** *Biochemistry,* 1996, vol. 35, 14413-24
- **Capaldi et al.** *Nucleic Acid Res.,* 2000, vol. 28 (7), e21
- **Castiglioni et al.** *Appl. and Exper. Microbio.,* 2004, 7161-72
- **Fang et al.** *Chem.BioChem.,* 2003, vol. 4, 829-34
- **Gerry et al.** *J. Mol. Biol.,* 1999, vol. 292, 251-62

SEQUENCE LISTING

<110> Landsman, Tanya
Livingston, David
Martinelli, Richard
Huang, Yumei
Seigal, Ben,
Dingxue, Yan Coull, James M.
Stern, Andrew M.

<120> RECEPTOR FAMILY PROFILING

<130> ENS-032PC

<150> US 60/845,330
<151> 2006-09-28

<150> US 60/847,859
<151> 2006-09-28

<150> US 60/905,364
<151> 2007-03-07

<150> US 60/918,023
<151> 2007-03-14

<160> 73

<170> PatentIn version 3.3

<210> 1
<211> 10
<212> DNA
<213> Artificial Sequence

<220>
<223> Probe oligonucleotide

<400> 1
tgtaggtaac 10

<210> 2
<211> 10
<212> DNA
<213> Artificial Sequence

<220>
<223> Probe oligonucleotide

<400> 2
gttacctaca 10

<210> 3
<211> 18
<212> DNA
<213> Artificial Sequence

<220>
<223> Probe oligonucleotide

<400> 3
cttcttcatg taggtaac 18

<210> 4
<211> 18
<212> DNA
<213> Artificial Sequence

<220>
<223> Probe oligonucleotide

<400> 4
cttcttcagt tacctaca 18

<210> 5
<211> 18
<212> DNA
<213> Artificial Sequence

<220>
<223> Oligonucleotide

<400> 5
caatggatgt acttcttd 18

<210> 6
<211> 18
<212> DNA
<213> Artificial Sequence

<220>
<223> Oligonucleotide

<400> 6
acatccattg acttcttc 18

<210> 7
<211> 18
<212> DNA
<213> Artificial Sequence

<220>
<223> Oligonucleotide

<400> 7
caatggatgt acttcttc 18

<210> 8
<211> 18
<212> DNA
<213> Artificial sequence

<220>
<223> Oligonucleotide

<400> 8
acatcctttg acttcttc 18

<210> 9
<211> 10
<212> DNA
<213> Artificial Sequence

<220>
<223> Oligonucleotide reporter sequence

<400> 9
cgaatttata 10

<210> 10
<211> 10
<212> DNA
<213> Artificial Sequence

<220>
<223> Oligonucleotide reporter sequence

<400> 10
tataaattcg 10

<210> 11
<211> 34
<212> DNA
<213> Artificial Sequence

<220>
<223> Oligonucleotide

<400> 11
caggctacgg cacgtagagc atcaccatga cctg 34

<210> 12
<211> 18
<212> DNA
<213> Artificial Sequence

<220>
<223> oligonucleotide zip code

<400> 12
gctgccatcg atggtcag 18

<210> 13
<211> 18
<212> DNA
<213> Artificial Sequence

<220>
<223> Oligonucleotide anti-zip code

<400> 13
ctgaccatcg atggcagc 18

<210> 14
<211> 18
<212> DNA
<213> Artificial Sequence

<220>
<223> oligonucleotide anti-zip code

<400> 14
ggactcgagc accaatac 18

<210> 15
<211> 18
<212> DNA
<213> Artificial Sequence

<220>
<223> Oligonucleotide zip code

<400> 15
gtattggt gctcgagtcc 18

<210> 16
<211> 15
<212> DNA
<213> Artificial Sequence

<220>
<223> oligonucleotide zip code

<400> 16
gctgccatcg atggt 15

<210> 17
<211> 15
<212> DNA
<213> Artificial sequence

<220>
<223> Oligonucleotide anti-zip code

<400> 17
accatcgatg gcagc 15

<210> 18
<211> 13
<212> DNA

<213> Artificial Sequence

<220>
<223> oligonucleotide zip code

<400> 18
ttggtgctcg agt 13

<210> 19
<211> 31
<212> DNA
<213> Artificial Sequence

<220>
<223> Oligol oligonucleotide

<400> 19
gtggtagttg gagctggtgg cgtaggcaag a 31

<210> 20
<211> 15
<212> DNA
<213> Artificial Sequence

<220>
<223> oligo2 oligonucleotide

<400> 20
agctccaact accac 15

<210> 21
<211> 15
<212> DNA
<213> Artificial Sequence

<220>
<223> oligo oligonucleotide

<400> 21
gtggtagttg gagct 15

<210> 22
<211> 15
<212> DNA
<213> Artificial Sequence

<220>
<223> oligo5 oligonucleotide

<400> 22
tcttgcctac gccac 15

<210> 23
<211> 15
<212> DNA
<213> Artificial Sequence

<220>

<223> Oligo6 oligonucleotide

<400> 23
agatcccact agcac 15

<216> 24
<211> 18
<212> DNA
<213> Artificial sequence

<220>
<223> Oligo 26 oligonucleotide

<400> 24
cttcggccca gatatcgt 18

<210> 25
<211> 18
<212> DNA
<213> Artificial Sequence

<220>
<223> Oligo 27 oligonucleotide

<400> 25
ctacagctac gatatctg 18

<210> 26
<211> 18
<212> DNA
<213> Artificial sequence

<220>
<223> oligo 28 oligonucleotide

<400> 26
ctacagctgt gatatcat 18

<210> 27
<211> 20
<212> DNA
<213> Artificial Sequence

<220>
<223> AT-rich oligonucleotide

<400> 27
tttttttttt tttaattaaa 20

<210> 28
<211> 55
<212> DNA
<213> Artificial sequence

<220>
<223> Oligo 201 oligonucleotide

<400> 28

caggctacgg cacgtagagc atcaccatga tcctgccccc cccccatatt taagc 55

<210> 29
<211> 55
<212> DNA
<213> Artificial Sequence

<220>
<223> Oligo 202 oligonucleotide

<400> 29
gcttaaatat cccccccccc caggctacgg cacgtagagc atcaccatga tcctg 55

<210> 30
<211> 57
<212> DNA
<213> Artificial Sequence

<220>
<223> oligo 203 oligonucleotide

<400> 30
gtgggaatgg tgcccccccc cccaggctac ggcacgtaga gcatcaccat gatcctg 57

<210> 31
<211> 30
<212> DNA
<213> Artificial sequence

<220>
<223> oligo 204 oligonucleotide

<400> 31
gtggtagttg gagtcgtggc gtaggcaaga 30

<210> 32
<211> 34
<212> DNA
<213> Artificial Sequence.

<220>
<223> oligo 205 oligonucleotide

<400> 32
gtggtagttg gagtcatacg tggcgtaggc aaga 34

<210> 33
<211> 40
<212> DNA
<213> Artificial sequence

<220>
<223> Oligo 206 oligonucleotide

<400> 33
gtggtagttg gagctcacac cacacgtggc gtaggcaaga 40

<210> 34

<211> 46
<212> DNA
<213> Artificial Sequence

<220>
<223> Oligo 207 oligonucleotide

<400> 34
gtggtagttg gagtcacaca caccacacac agtggcgtag gcaaga 46

<210> 35
<211> 68
<212> DNA
<213> Artificial Sequence

<220>
<223> oligo 208 oligonucleotide

<400> 35

**gtggtagttg gagctcacac cacaccaacc acaccacacc acacacacca cacgtggcgt 60**

**aggcaaga 68**

<210> 36
<211> 20
<212> DNA
<213> Artificial Sequence

<220>
<223> Oligo 209 oligonucleotide

<400> 36
gtgtggtgtg gtgtggtgtg 20

<210> 37
<211> 30
<212> DNA
<213> Artificial Sequence

<220>
<223> Oligo 210 oligonucleotide

<400> 37
gtggcgtagg caagagtggt agttggagct 30

<210> 38
<211> 12
<212> DNA
<213> Artificial Sequence

<220>
<223> Oligo 211 oligonucleotide

<400> 38
gtgggaatgg tg 12

<210> 39

<211> 15
<212> DNA
<213> Artificial Sequence

<220>
<223> Oligo 212 oligonucleotide

<400> 39
agatcccact agcac 15

<210> 40
<211> 15
<212> DNA
<213> Artificial Sequence

<220>
<223> oligo 213 oligonucleotide

<400> 40
agctccaact accac 15

<210> 41
<211> 15
<212> DNA
<213> Artificial Sequence

<220>
<223> Oligo 214 oligonucleotide

<400> 41
tcttgcctac gccac 15

<210> 42
<211> 35
<212> DNA
<213> Artificial Sequence

<220>
<223> Oligo 215 oligonucleotide

<400> 42
caggctacgg cacgtagagc atcaccatga tcctg 35

<210> 43
<211> 28
<212> DNA
<213> Artificial Sequence

<220>
<223> Oligo 106 oligonucleotide

<220>
<221> misc_feature
<222> (18)..(19)
<223> C18 PEG

<400> 43
ggactcgagc accaatacta taaattcg 28

<210> 44
<211> 28
<212> DNA
<213> Artificial Sequence

<220>
<223> Oligo 109 oligonucleotide

<220>
<221> misc_feature
<222> (10)..(11)
<223> C18 PEG

<400> 44
cgaatttata ctgaccatcg atggcagc 28

<210> 45
<211> 53
<212> DNA
<213> Artificial Sequence

<220>
<223> oligo 112 oligonucleotide

<220>
<221> misc_feature
<222> (35)..(36)
<223> C18 PEG

<400> 45
caggctacgg cacgtagagc atcaccatga tcctggctgc catcgatggt cag 53

<210> 46
<211> 53
<212> DNA
<213> Artificial Sequence

<220>
<223> oligo 113 oligonucleotide

<220>
<221> misc_feature
<222> (18)..(19)
<223> C18 PEG

<400> 46
gtattggtgc tcgagtccca ggctacggca cgtagagcat caccatgatc ctg 53

<210> 47
<211> 53
<212> DNA
<213> Artificial Sequence

<220>
<223> Oligo 119 oligonucleotide

<400> 47
gtattggtgc tcgagtccca ggctacggca cgtagagcat caccatgatc ctg 53

<210> 48
<211> 63
<212> DNA
<213> Artificial Sequence

<220>
<223> Oligo 120 bligonucleotide

<400> 48

gtattggtgc tcgagtcccc cccccccca ggctacggca cgtagagcat caccatgatc 60
ctg 63

<210> 49
<211> 73
<212> DNA
<213> Artificial Sequence

<220>
<223> oligo 121 oligonucleotide

<400> 49

gtattggtgc tcgagtcccc cccccccccc cccccccca ggctacggca cgtagagcat 60
caccatgatc ctg 73

<210> 50
<211> 53
<212> DNA
<213> Artificial Sequence

<220>
<223> Oligo 122 oligonucleotide

<400> 50
caggctacgg cacgtagagc atcaccatga tcctggctgc catcgatggt cag 53

<210> 51
<211> 63
<212> DNA
<213> Artificial Sequence

<220>
<223> oligo 123 oligonucleotide

<400> 51

caggctacgg cacgtagagc atcaccatga tcctgccccc cccccgctgc catcgatggt 60
cag 63

<210> 52
<211> 73

<212> DNA
<213> Artificial Sequence

<220>
<223> oligo 124 oligonucleotide

<400> 52

caggctacgg cacgtagagc atcaccatga tcctgccccc cccccccccc cccccgctgc 60
catcgatggt cag 73

<210> 53
<211> 50
<212> DNA
<213> Artificial sequence

<220>
<223> oligo 127 oligonucleotide

<400> 53
caggctacgg cacgtagagc atcaccatga tcctggctgc catcgatggt 50

<210> 54
<211> 47
<212> DNA
<213> Artificial Sequence

<220>
<223> oligo 128 oligonucleotide

<400> 54
caggctacgg cacgtagagc atcaccatga tcctggctgc catcgat 47

<210> 55
<211> 50
<212> DNA
<213> Artificial Sequence

<220>
<223> Oligo 129 oligonucleotide

<400> 55
ttggtgctcg agtcccaggc tacggcacgt agagcatcac catgatcctg 50

<210> 56
<211> 47
<212> DNA
<213> Artificial Sequence

<220>
<223> Oligo 130 oligonucleotide

<400> 56
gtgctcgagt cccaggctac ggcacgtaga gcatcaccat gatcctg 47

<210> 57

<211> 18
<212> DNA
<213> Artificial Sequence

<220>
<223> Oligonucleotide zip code

<400> 57
gctgaggtac gatgctga 18

<210> 58
<211> 18
<212> DNA
<213> Artificial Sequence

<220>
<223> oligonucleotide anti-zip code

<400> 58
tcagcatcgt acctcagc 18

<210> 59
<211> 35
<212> DNA
<213> Artificial Sequence

<220>
<223> Oligonucleotide

<400> 59
ttggtgctcg agtccccccc cccccccccc ccccc 35

<210> 60
<211> 38
<212> DNA
<213> Artificial Sequence

<220>
<223> Oligonucleotide

<400> 60
cccccccccc cccccccccc gctgaggtac gatgctga 38

<210> 61
<211> 15
<212> DNA
<213> Artificial Sequence

<220>
<223> oligo EDC1 oligonucleotide

<400> 61
gtggtagttg gagct 15

<210> 62
<211> 15
<212> DNA
<213> Artificial Sequence

<220>
<223> Oligo EDC2 oligonucleotide

<400> 62
agctccaact accac 15

<210> 63
<211> 15
<212> DNA
<213> Artificial sequence

<220>
<223> Oligo EDC3 oligonucleotide

<400> 63
agatcccact agcac 15

<210> 64
<211> 35
<212> DNA
<213> Artificial Sequence

<220>
<223> Aptamer oligonucleotide

<400> 64
caggctacgg cacgtagagc atcaccatga tcctg 35

<210> 65
<211> 35
<212> DNA
<213> Artificial sequence

<220>
<223> Aptamer oligonucleotide

<400> 65
caggctacgg cacgtagagc atcaccatga tcctg 35

<210> 66
<211> 35
<212> DNA
<213> Artificial

<220>
<223> Zip2

<400> 66
ttggtgctcg agtccccccc cccccccccc ccccc 35

<210> 67
<211> 35
<212> DNA
<213> Artificial

<220>
<223> zip3

<400> 67
cccccccccc cccccccccc gctgccatcg atggt 35

<210> 68
<211> 35
<212> DNA
<213> Artificial

<220>
<223> Zip5

<400> 68
cccccccccc ccccccccgt gccatccata gtcag 35

<210> 69
<211> 28
<212> DNA
<213> Artificial

<220>
<223> Antizip2 reporter 1

<400> 69
ggactcgagc accaatacta taaattcg 28

<210> 70
<211> 28
<212> DNA
<213> Artificial

<220>
<223> Antizip3 reporter

<400> 70
cgaatttata ctgaccatcg atggcagc 28

<210> 71
<211> 28
<212> DNA
<213> Artificial

<220>
<223> Antizip5 reporter mismatch

<400> 71
ccaattaata ctgactatgg atggcacg 28

<210> 72
<211> 31
<212> DNA
<213> Artificial

<220>
<223> Antizip5 reporter

<400> 72
ggactcgagc accaatacta taaattcgcc c 31

<210> 73
<211> 31
<212> DNA
<213> Artificial

<220>
<223> Antizip2 report 2

<400> 73
cccgcttaaa tatcataacc acgagctcag g 31